# EUROPEAN PATENT SPECIFICATION

(11) **EP 4 281 484 B1**
(45) Date of publication and mention of the grant of the patent: **18.03.2026**
(21) Application number: 22707001.8
(22) Date of filing: 24.01.2022
(51) Int. Cl.: C07K 16/30, C07K 16/32, A61P 35/00

(54) **ANTI-HER-2/TROP-2 CONSTRUCTS AND USES THEREOF**
ANTI-HER-2/TROP-2-KONSTRUKTE UND VERWENDUNGEN DAVON
CONSTRUCTIONS ANTI-HER-2/TROP-2 ET LEURS UTILISATIONS

(30) Priority: 22.01.2021 US 202163140638 P
(43) Date of publication of application: 29.11.2023
(73) Proprietor: Hangzhou Baikai Biopharmaceutical Co., Ltd, Hangzhou, Zhejiang 310018 (CN)
(72) Inventor: BAO, Haifeng, Germantown, Maryland 20817 (US); ZHONG, Haihong, Germantown, Maryland 20817 (US); YUAN, Wei, Germantown, Maryland 20817 (US); LIU, Dingguo, Germantown, Maryland 20817 (US)
(74) Representative: CMS Cameron McKenna Nabarro Olswang LLP
(86) International application number: PCT/US2022/070326
(87) International publication number: WO 2022/159984

(56) References cited:
- WO-A1-2018/057955
- WO-A1-2018/059502
- WO-A1-2019/222428
- WO-A2-2022/051647
- ANNA SHVARTSUR ET AL: "Trop2 and its overexpression in cancers: regulation and clinical/ therapeutic implications", GENES & CANCER, 1 March 2015 (2015-03-01), pages 3 - 4, XP055622384, Retrieved from the Internet <URL:https://www.ncbi.nlm.nih.gov/pmc/articles/PMC4426947/pdf/ganc-06-084.pdf> [retrieved on 20190916], DOI: 10.18632/genesandcancer.40
- RIJ CATHARINA M VAN ET AL: "Pretargeted ImmunoPET of Prostate Cancer with an Anti-TROP-2 x Anti-HSG Bispecific Antibody in Mice with PC3 Xenografts", MOLECULAR IMAGING & BIOLOGY, ELSEVIER, BOSTON, vol. 17, no. 1, 25 July 2014 (2014-07-25), pages 94 - 101, XP035428181, ISSN: 1536-1632, [retrieved on 20140725], DOI: 10.1007/S11307-014-0772-X
- HYUNBO SHIM: "Bispecific Antibodies and Antibody?Drug Conjugates for Cancer Therapy: Technological Considerations", BIOMOLECULES, vol. 10, no. 3, 26 February 2020 (2020-02-26), pages 360, XP055704728, DOI: 10.3390/biom10030360

## Description

### TECHNICAL FIELD

The present disclosure relates to anti-HER2/Trop2 constructs and the uses thereof.

### BACKGROUND OF THE APPLICATION

Today, cancer remains a major cause of death worldwide despite the numerous advanced diagnostic and therapeutic methods that have been developed. The major barrier to successful treatment and prevention of cancer lies in the fact that many cancers still fail to respond to the current chemotherapeutic and immunotherapy intervention, and many individuals suffer a recurrence or death, even after aggressive therapy.

Cancer immunotherapy is enjoying a renaissance, and in the past few years the rapidly advancing field has produced several new methods of treating cancer. Numerous cancer immunotherapy strategies have been the focus of extensive research and clinical evaluation including, but not limited to, treatment using depleting antibodies to specific tumor antigens; treatment using antibody-drug conjugates; treatment using agonistic, antagonistic, or blocking antibodies to co-stimulatory or co-inhibitory molecules (immune checkpoints); treatment using bispecific T cell engaging antibodies (BiTE^{®}) such as blinatumomab; treatment involving administration of biological response modifiers such as IL-2, IL-12, IL-15, IL-21, GM-CSF, IFN-α, IFN-β and IFN-y; treatment using therapeutic vaccines such as sipuleucel-T; treatment using dendritic cell vaccines, or tumor antigen peptide vaccines; treatment using chimeric antigen receptor (CAR)-T cells; treatment using CAR-NK cells; treatment using tumor infiltrating lymphocytes (TILs); and treatment using adoptively transferred anti-tumor T cells (ex vivo expanded and/or TCR transgenic).

HER2 (also known as CD340 or ERBB2), a member of the human epidermal growth factor receptor family, is aberrantly expressed in certain malignancies, including breast gastric, and lung cancer, primarily due to HER2 genomic amplification. HER2 over-expression is associated with increased risk of recurrence and poorer prognosis in these cancers. In HER2-positive malignancies, this protein can stimulate downstream RAS-RAF-ERK and PI3K-PTEN-AKT signaling and play a key role in cell proliferation.

Trophoblast cell-surface antigen-2 (Trop-2) (also known as tumor-associated calcium signal transducer 2 (TACSTD1), membrane component chromosome 1 surface marker 1 (M1S1), gastrointestinal antigen 733-1 (GA733-1), and epithelial glycoprotein-1 (EGP-1)), belongs to the TACSTD family including at least two type I membrane proteins. Although it was first discovered as a cell surface marker of trophoblast cells, subsequent studies have suggested that Trop-2 is overexpressed in different cancer types.

WO2019222428 relates to the composition of bispecific antibodies against cadherin-17 and CD3 and method of using the antibodies for cancer treatment. WO2022051647 relates to conditional Bispecific Redirected Activation constructs, or COBRAs, that are administered in an active pro-drug format, which includes a human serum albumin (HSA) domain that increases its serum half-life. Upon exposure to tumor proteases, the constructs are cleaved and activated, such that they can bind both tumor target antigens (TTAs) as well as CD3, and thus recruiting T cells expressing CD3 to the tumor, resulting in treatment.

### DISCLOSURE OF THE APPLICATION

The invention is as set out in the appended set of claims. Any part of the description not within the scope of the claims does not form a part of the invention but serves in the understanding of the claimed invention. Any embodiment of the description not within the scope of the claims does not form a part of the invention but forms part of the disclosure relating to the claimed invention. Any references in the description to methods of treatment, including administration to a patient in a method of treatment are to be interpreted as references to the constructs and pharmaceutical compositions of the present invention for use in a method for treatment of the human (or animal) body by therapy, or for use in administering to a patient in a method of treatment. Any references in the description to a method which falls within the exceptions from patentability according to Article 53(c) EPC is not within the scope of the claims.

The present application in one aspect provides novel constructs that specifically bind to both HER2 and Trop-2 (e.g., bispecific antibodies or ADCs described herein). In some embodiments, these construct comprise a full-length antibody that target one of HER2 and Trop-2 fused with a scFv that target to the other of HER2 and Trop2. See, e.g., FIGs. 1-5. In some embodiments, the construct is particularly advantageous in its ability to robustly bind to cancer cells expressing HER2 and/or Trop2 at a higher level that the parental antibodies (see, e.g., exemplified bispecific antibody HT002 and TH002 as shown in FIG. 6A). In some embodiments, the construct is particularly advantageous in its ability to robustly bind to cancer cells that express a high level ofHER2 and/or Trop-2 (see, e.g., exemplified bispecific antibody HT002 and TH001b as shown in FIG. 7). In some embodiments, the construct is particularly advantageous in its ability to achieve a durable and/or complete anti-tumor response (see, e.g., FIG. 12).

The present application in another aspect provides methods of using the constructs described herein, e.g., disclosed herein, for treating a cancer. In some embodiments, the cancer is HER2 positive and/or Trop-2 positive. In some embodiments, the cancer is HER2 low and/or Trop-2 low. In some embodiments, the cancer is HER2 high/medium and/or Trop-2 high/medium.

A bispecific antibody is an antibody that includes two different antigen binding sites of monoclonal antibodies that can bind to two different antigens or two different sites of one antigen. In addition to simultaneously blocking two different signaling pathways and thereby enhancing tumor cell killing, bispecific antibody can also potentially increase binding specificity by interacting with two different cell-surface antigens instead of one. It is nowadays considered as a next generationof effective molecules for cancer therapy. It can minimize the regulatory and commercial issues that stem from administration of multiple therapeutic molecules. It also has the potential for novel activities that do not exist in mixtures of the parental antibodies. Several bispecific antibodies are marketed, and many are in clinical development.

The present application provides novel bispecific antibodies and antigen-binding fragments that specifically bind and neutralize both HER2 and Trop-2 proteins. The present application further provides ADCs comprising these novel bispecific antibodies and antigen-binding fragments linked to an effector molecule. Accordingly, the application relates to novel compositions and methods for diagnosing, prognosing, and treating conditions that would benefit from modulating HER2 and Trop-2 (e.g., cancer, persistent infectious diseases, autoimmune diseases, asthma, transplant rejection, and inflammatory disorders) using the novel bispecific anti-HER2/Trop-2 antibodies, antigen-binding fragments, and ADCs described herein.

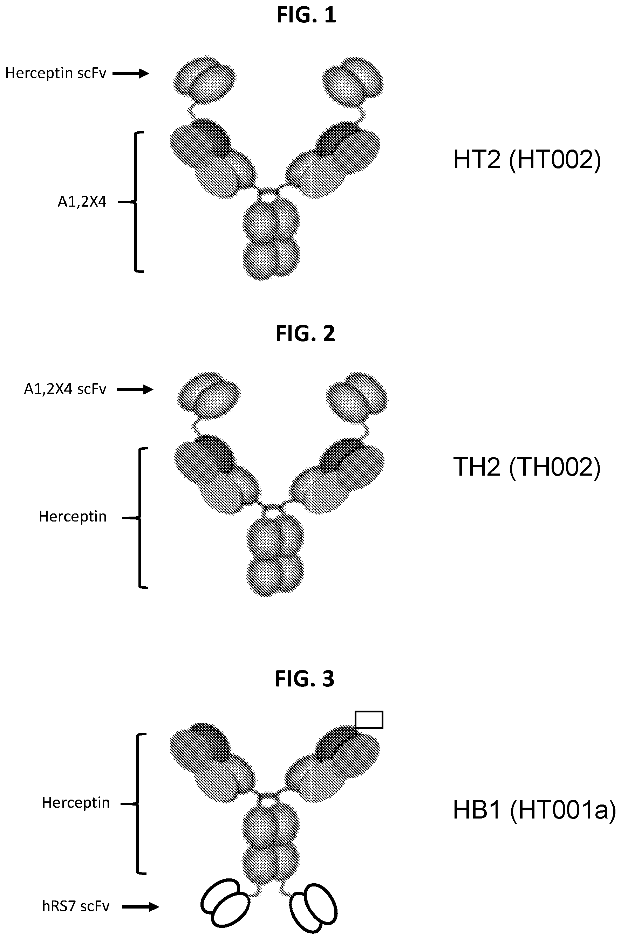

In one aspect, the present application provides constructs including isolated bispecific antibodies, and antigen-binding fragments thereof, specifically designed to bind to and neutralize both HER2 and Trop-2. In various embodiments, the bispecific antibodies and antigen-binding fragments thereof are designed as depicted in FIGS. 1-5.

In various embodiments, the bispecific anti-HER2/Trop-2 antibody comprises a first immunoglobulin antigen-binding domain and a second immunoglobulin antigen-binding domain, wherein the first immunoglobulin antigen-binding domain specifically binds to HER2 antibody binding sites, and the second immunoglobulin antigen-binding domain specifically binds to Trop-2 antibody binding site. In various embodiments, the bispecific anti-HER2/Trop-2 antibody comprises a first immunoglobulin antigen-binding domain and a second immunoglobulin antigen-binding domain, wherein the first immunoglobulin antigen-binding domain specifically binds to Trop-2 antibody binding sites, and the second immunoglobulin antigen-binding domain binds to HER2 antibody binding sites.

In various embodiments, the first immunoglobulin antigen-binding domain of the bispecific anti-HER2/Trop-2 antibody comprises an scFv antibody fragment, and the second antigen-binding domain comprises a V_{H} and a V_{L}. In various embodiments, the first antigen-binding domain of the bispecific anti-HER2/Trop-2 antibody comprises a V_{H} and a V_{L}, and the second immunoglobulin antigen-binding domain comprises an scFv antibody fragment.

In various embodiments, the first or second immunoglobulin antigen-binding domain of the bispecific anti-HER2/Trop-2 antibody comprises a V_{H} further comprising a heavy chain constant region or fragment thereof and a V_{L} further comprising a light chain constant region or fragment thereof. In various embodiments, the heavy chain constant region or fragment thereof is an IgG constant region. In various embodiments, the IgG constant region or fragment thereof is an IgG1 constant region. In various embodiments, the LC constant region is a kappa constant region. In various embodiments, the light chain constant region is a lambda constant region. In various embodiments, the first or second immunoglobulin antigen-binding domain of the bispecific anti-HER2/Trop-2 antibody comprises a heavy chain which can include an Fc domain comprising a CH2 and a CH3 region. In various embodiments, the Fc domain is an IgG1 Fc domain. In various embodiments, the IgG1 Fc domain is a native IgG1 Fc domain.

In various embodiments, the first or second immunoglobulin antigen-binding domain is a monoclonal antibody. In various embodiments, the first or second immunoglobulin antigen-binding domain is a humanized antibody. In various embodiments, the first or second immunoglobulin antigen-binding domain is a human antibody. In various embodiments, the first or second immunoglobulin antigen-binding domain is a chimeric antibody. In various embodiments, the first or second immunoglobulin antigen-binding domain is an affinity optimized antibody.

In various embodiments, the second immunoglobulin antigen-binding domain of the bispecific anti-HER2/Trop-2 antibody is covalently linked to the carboxy-terminus of the heavy chain of the first immunoglobulin antigen-binding domain. In various embodiments, the bispecific anti- HER2/Trop-2 antibody comprises a linker interposed between the second immunoglobulin antigen binding domain and the carboxy-terminus of the heavy chain of the first immunoglobulin antigen-binding domain. In various embodiments, the peptide linker comprises the amino acid sequence of SEQ ID NO: 25. In various embodiments, the second immunoglobulin antigen-binding domain of the bispecific anti-HER2/Trop-2 antibody is covalently linked to the amino-terminus of the heavy chain of the first immunoglobulin antigen-binding domain. In various embodiments, the bispecific anti-HER2/Trop-2 antibody comprises a linker interposed between the second immunoglobulin antigen-binding domain and the amino-terminus of the heavy chain of the first immunoglobulin antigen-binding domain. In various embodiments, the peptide linker comprises the amino acid sequence of SEQ ID NO: 25.

**In** various embodiments, the V_{H} and V_{L} of the scFv are covalently linked via a peptide linker. In various embodiments, the peptide linker comprises the amino acid sequence set forth in SEQ ID NO: 24.

**In** various embodiments, the bispecific anti-HER2/Trop-2 antibody comprises a first immunoglobulin antigen binding-domain that specifically binds to HER2, and a second immunoglobulin antigen-binding domain that specifically binds to Trop-2, wherein the first immunoglobulin antigen-binding domain comprises a scFv comprising the amino acid sequence set forth in SEQ ID NO: 3; wherein the second immunoglobulin antigen-binding domain comprises a V_{H} comprising the amino acid sequence set forth in SEQ ID NO: 4 and a V_{L} comprising the amino acid sequence set forth in SEQ ID NO: 5.

In various embodiments, the bispecific anti-HER2/Trop-2 antibody comprises a first immunoglobulin antigen binding-domain that specifically binds to Trop-2, and a second immunoglobulin antigen-binding domain that specifically binds to HER2, wherein the first immunoglobulin antigen-binding domain comprises a scFv comprising the amino acid sequence set forth in SEQ ID NO: 6; wherein the second immunoglobulin antigen-binding domain comprises a V_{H} comprising the amino acid sequence set forth in SEQ ID NO: 7 and a V_{L} comprising the amino acid sequence set forth in SEQ ID NO: 8.

In various embodiments, the bispecific anti-HER2/Trop-2 antibody comprises a first immunoglobulin antigen binding-domain that specifically binds to HER2, and a second immunoglobulin antigen-binding domain that specifically binds to Trop-2, wherein the first immunoglobulin antigen-binding domain comprises a V_{H} comprising the amino acid sequence set forth in SEQ ID NO: 7 and a V_{L} comprising the amino acid sequence set forth in SEQ ID NO: 8; and wherein the second immunoglobulin antigen-binding domain comprises a scFv comprising the amino acid sequence set forth in SEQ ID NO: 9.

In various embodiments, the bispecific anti-HER2/Trop-2 antibody comprises a first immunoglobulin antigen binding-domain that specifically binds to Trop-2, and a second immunoglobulin antigen-binding domain that specifically binds to HER2, wherein the first immunoglobulin antigen-binding domain comprises a V_{H} comprising the amino acid sequence set forth in SEQ ID NO: 10 and a V_{L} comprising the amino acid sequence set forth in SEQ ID NO: 11; and wherein the second immunoglobulin antigen-binding domain comprises a scFv comprising the amino acid sequence set forth in SEQ ID NO: 3.

In various embodiments, the bispecific anti-HER2/Trop-2 antibody comprises a first immunoglobulin antigen binding-domain that specifically binds to HER2, and a second immunoglobulin antigen-binding domain that specifically binds to Trop-2, wherein the first immunoglobulin antigen-binding domain comprises a scFv comprising the amino acid sequence set forth in SEQ ID NO: 3; and wherein the second immunoglobulin antigen-binding domain comprises a V_{H} comprising the amino acid sequence set forth in SEQ ID NO: 10 and a V_{L} comprising the amino acid sequence set forth in SEQ ID NO: 11.

In various embodiments, the bispecific anti-HER2/Trop-2 antibody comprises an immunoglobulin heavy chain (HC) and an immunoglobulin light chain (LC), wherein the HC comprises the amino acid sequence of SEQ ID NO: 12, and the LC comprises the amino acid sequence of SEQ ID NO: 13.

In various embodiments, the bispecific antibody binds to HER-2 and Trop-2 protein with a dissociation constant (K_{D}) of at least about 1x10⁻⁶ M, at least about 1x10⁻⁷ M, at least about 1x10⁻⁸ M, at least about 1x10⁻⁹ M, at least about 1x10⁻¹⁰ M, at least about 1x10⁻¹¹ M, or at least about 1x10⁻¹² M.

In another aspect, the present application provides isolated immunoconjugates or fusion proteins comprising a a bispecific HER2/Trop-2 binding antibody conjugated to, linked to (or otherwise stably associated with) an effector molecule. In various embodiments, the effector molecule is an immunotoxin, cytokine, chemokine, drug moiety, therapeutic agent, or chemotherapeutic agent. In various embodiments, the present application provides antibody drug conjugates (ADCs) having the formula: Ab-(L-D)n, wherein Ab is a bispecific HER2/Trop-2 binding antibody, L is a linker, D is a drug moiety, and n is an integer from 2, 3, 4, 5, 6, 7, 8, 9, and 10.

In another aspect, the bispecific antibodies disclosed herein may be covalently linked to (or otherwise stably associated with) an additional functional moiety, such as a label or a moiety that confers desirable pharmacokinetic properties. In various embodiments, the label is selected from the group consisting of: a fluorescent label, a radioactive label, and a label having a distinctive nuclear magnetic resonance signature.

In another aspect, the present application relates to a pharmaceutical composition comprising an isolated bispecific antibody or antigen-binding fragment, or ADC of the present application in admixture with a pharmaceutically acceptable carrier. In various embodiments, the pharmaceutical composition comprises an isolated human antibody in admixture with a pharmaceutically acceptable carrier. In various embodiments, the pharmaceutical composition is formulated for administration via a route selected from the group consisting of subcutaneous injection, intraperitoneal injection, intramuscular injection, intrasternal injection, intravenous injection, intraarterial injection, intrathecal injection, intraventricular injection, intraurethral injection, intracranial injection, intrasynovial injection or via infusions.

In another aspect, the present application relates to the use of the bispecific antibodies, or antigen-binding fragments thereof, or ADCs of the present application to diagnose, prognose, and treat human diseases (e.g., cancer, infectious diseases, autoimmune diseases, asthma, transplant rejection, and inflammatory disorders).

In various embodiments disclosed herein, the present application relates to methods of treating a subject suffering from a HER-2- or Trop-2-associated disorder, comprising administering to said subject a therapeutically effective amount of an antibody or antigen-binding fragment thereof, or ADC of the present application. In various embodiments, the cancer is a cancer associated with elevated expression of HER-2 or Trop-2. In various embodiments, the cancer is selected from the group consisting of colorectal cancer (CRC), renal cancer, non-small-cell lung cancer (NSCLC), prostate cancer, breast cancer, ovarian cancer, pancreatic cancer, gastric cancer, liver cancer, head and neck cancer, and glioma. In various embodiments, the subject previously responded to treatment with an anti-cancer therapy, but, upon cessation of therapy, suffered relapse (hereinafter "a recurrent cancer"). In various embodiments, the subject has resistant or refractory cancer.

In another aspect, the present application relates to combination therapies designed to treat a cancer, or an infectious disease in an subject, comprising administering to the subject a therapeutically effective amount of an isolated antibody or antigen-binding fragment of the present application, and b) one or more additional therapies selected from the group consisting of immunotherapy, chemotherapy, small molecule kinase inhibitor targeted therapy, surgery, radiation therapy, vaccination protocols, stem cell transplantation, and immune cell therapy (CAR-T, CAR-NK), wherein the combination therapy provides increased cell killing of tumor cells, i.e., a synergy exists between the isolated antibody or antigen-binding fragment and the additional therapies when co-administered.

In another aspect, the present application provides a method for detecting in vitro or in vivo the presence of human HER2- and Trop-2 antigen in a sample, e.g., for diagnosing a human HER2- and Trop-2-related disease.

In another aspect, isolated nucleic acids comprising the polynucleotide sequence that encodes the antibodies or antigen-binding fragments disclosed herein are provided. Also provided are isolated cells comprising the expression vectors of the application. In various embodiments, the cell is a host cell comprising an expression vector of the present application. In various embodiments, the cell is a hybridoma, wherein the chromosome of the cell comprises a nucleic acid of the present application. Further provided is a method of making the antibody or antigen-binding fragment of the present application comprising culturing or incubating the cell under conditions that allow the cell to express the antibody or antigen-binding fragment of the present application.

### BRIEF DESCRIPTION OF THE DRAWINGS

FIG. 1 depicts a representative bispecific antibody (designated HT2 (HT002)) comprising the anti-Trop-2 antibody A1,2X4 with a N-terminal Herceptin scFv attached via a linker.
FIG. 2 depicts a representative bispecific antibody (designated TH2 (TH002)) comprising the anti-HER2 antibody Herceptin with a N-terminal A1,2X4 scFv attached via a linker.
FIG. 3 depicts a representative bispecific antibody (designated HB1 (HT001a)) comprising the anti-HER2 antibody Herceptin with a C-terminal hRS7 scFv attached via a linker.

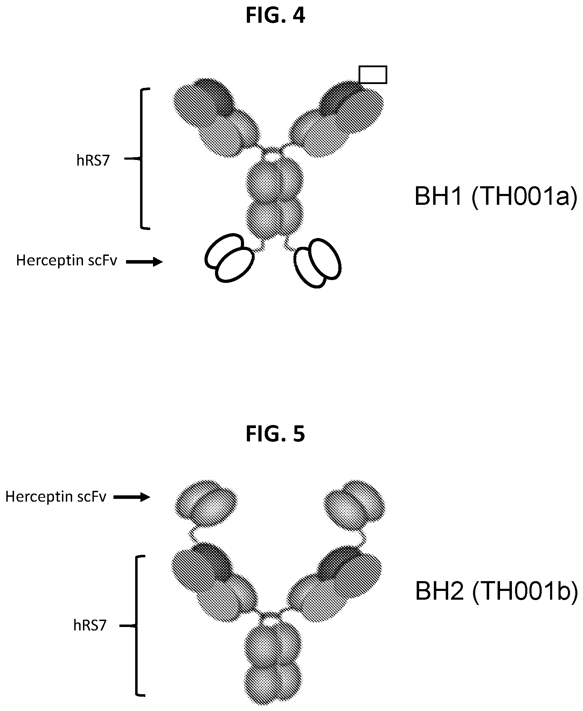

FIG. 4 depicts a representative bispecific antibody (designated BH1 (TH001a)) comprising the anti-Trop-2 antibody hRS7 with a C-terminal Herceptin scFv attached via a linker.
FIG. 5 depicts a representative bispecific antibody (designated BH2 (TH001b)) comprising the anti-Trop-2 antibody hRS7 with a N-terminal Herceptin scFv attached via a linker.

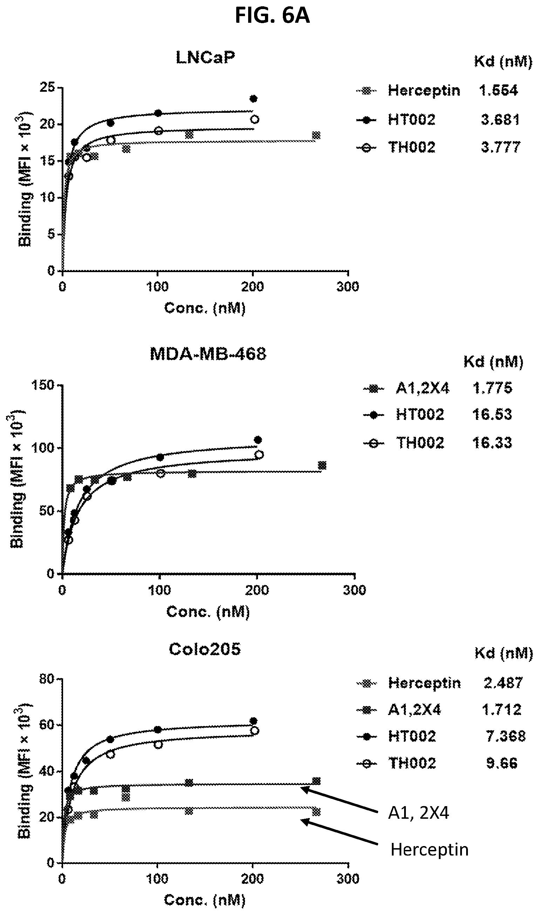

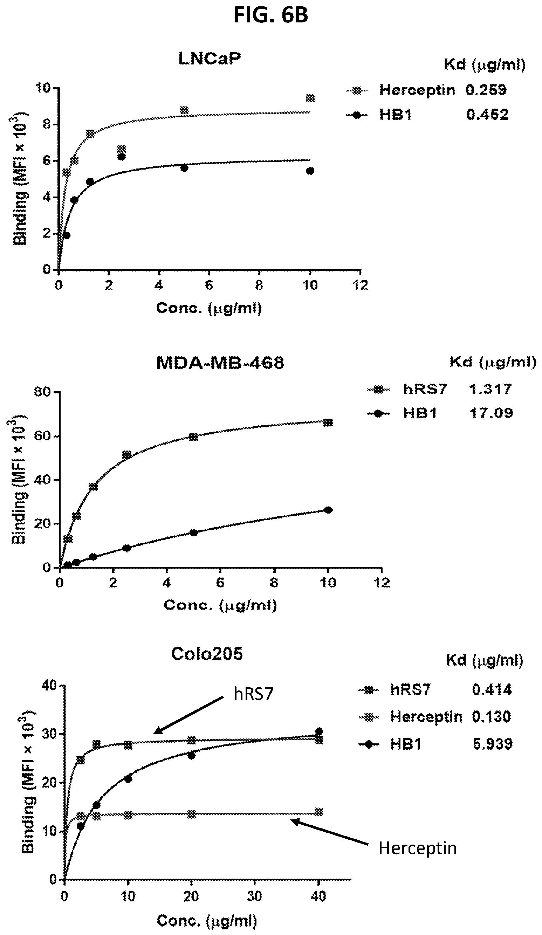

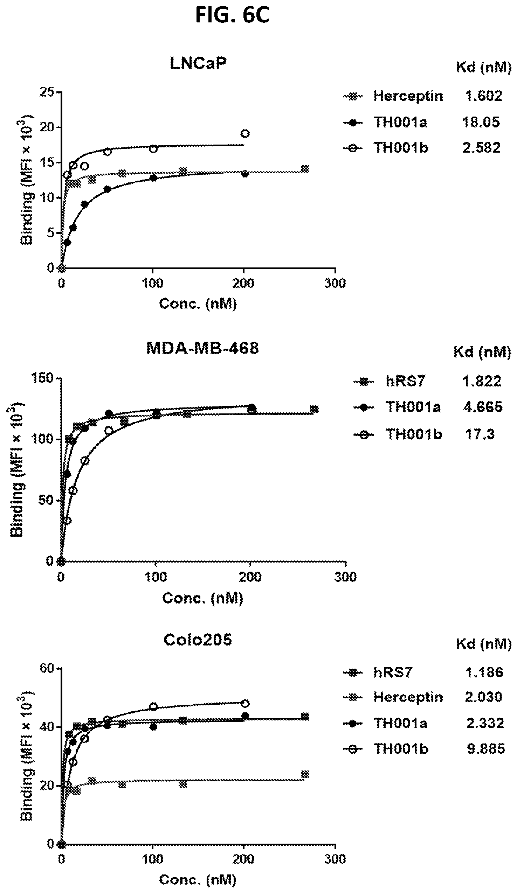

FIGs. 6A-6C depict line graphs depicting the results of the evaluation of the binding affinity of bispecific antibodies HT2 (HT002) and TH2 (TH002) (FIG. 6A), HB1 (HT001a) (FIG. 6B), BH1 (TH001a) and BH2 (TH001b) (FIG. 6C) by flow cytometry analysis (FACS).

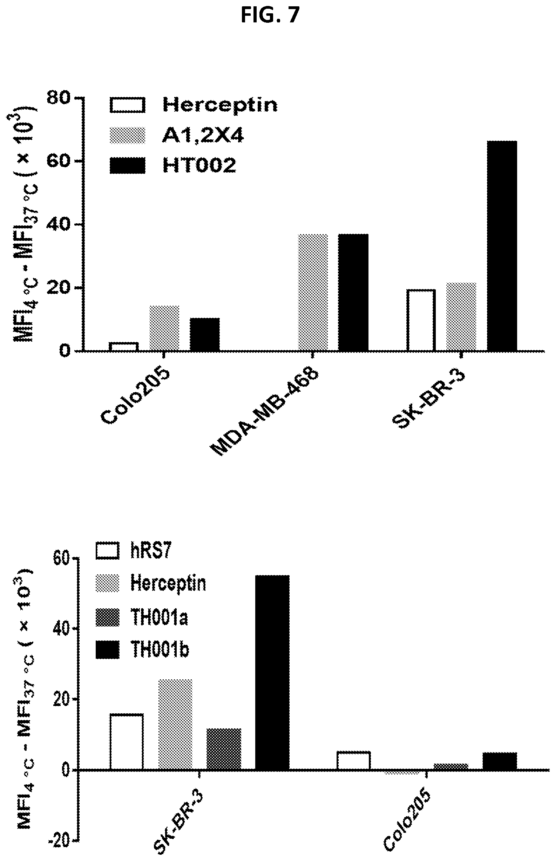

FIG. 7 depicts the results from flow cytometry analysis of bispecific antibody TH2 (TH002), BH1 (TH001a), and BH2 (TH001b) internalization.

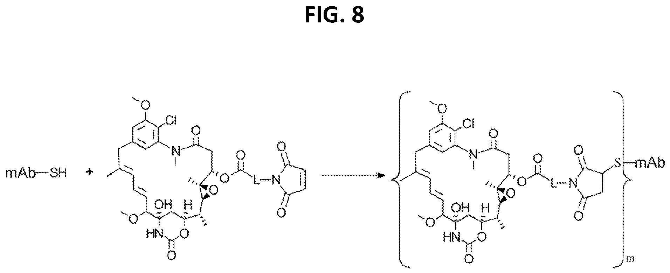

FIG. 8 depicts conjugation procedure for DM payload with reduced mAb.

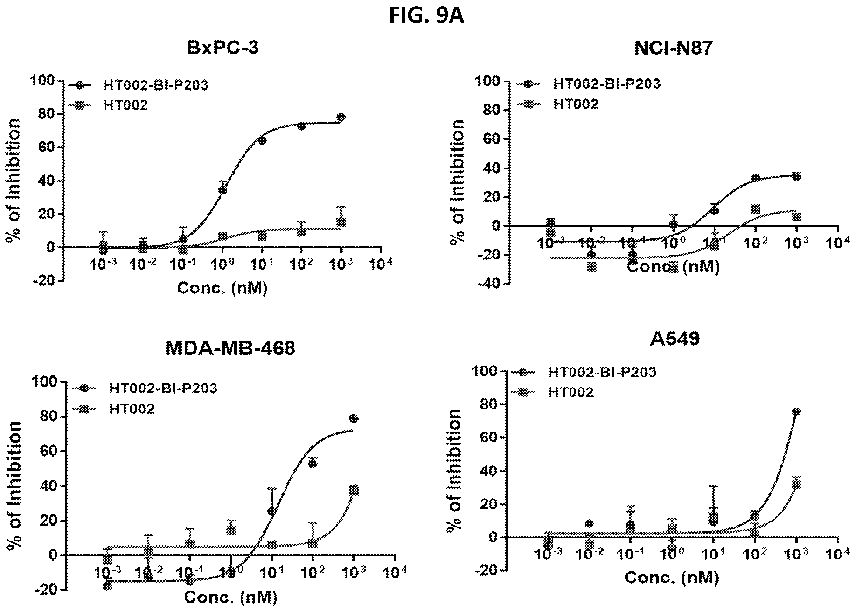

FIGs. 9A-9C depict the in *vitro* cytotoxicity activity of bispecific ADCs in tumor cells with different levels of HER2 and Trop-2 expression.

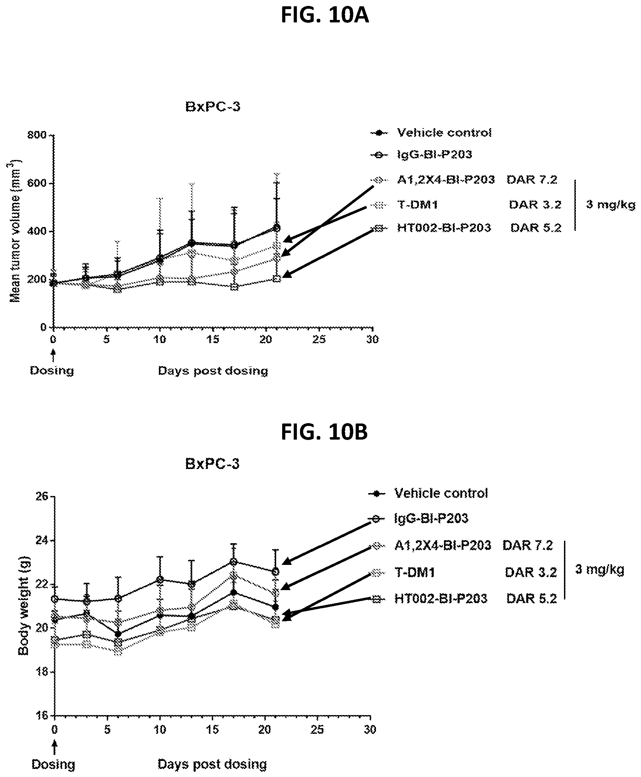

FIGs. 10A-10B show *in vivo* efficacy of HT002-BI-P203 in BxPC3 xenograft model.

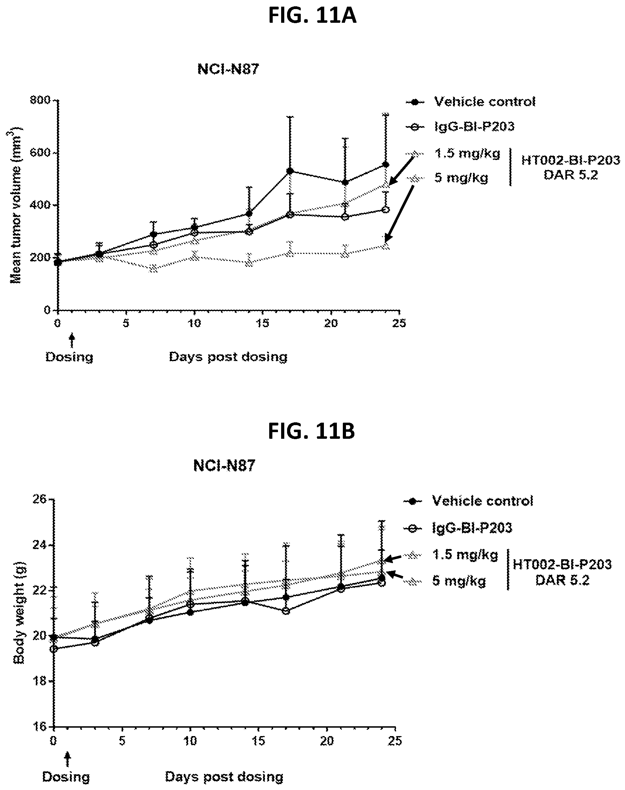

FIGs. 11A-11B show *in vivo* efficacy of HT002-BI-P203 in N87 xenograft model.

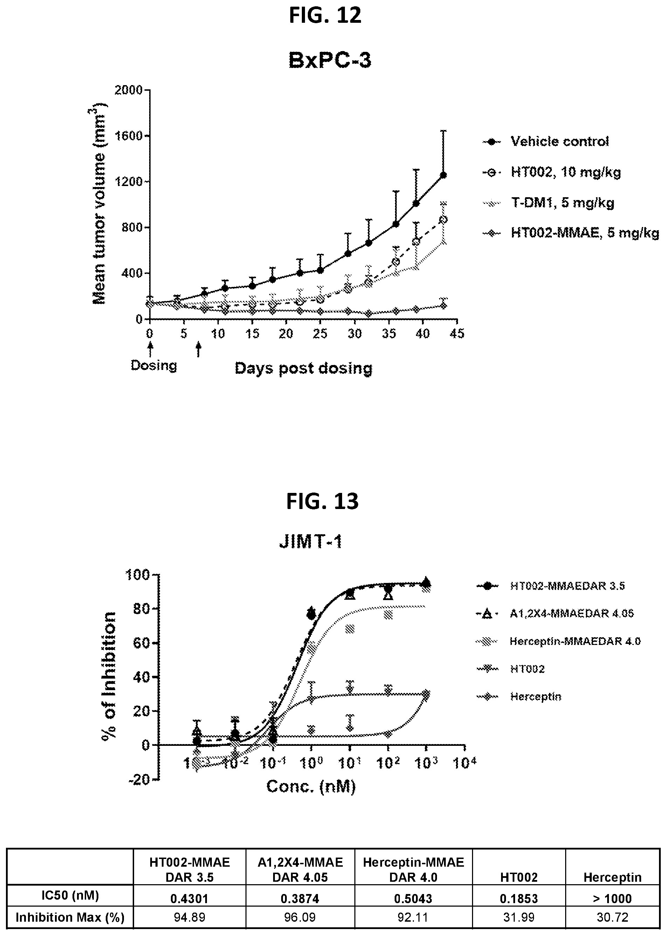

FIG. 12 shows *in vivo* efficacy of HT002-MMAE in BxPC3 xenograft model.
FIG. 13 shows the inhibition effect of HT002-MMAE in JIMT-1 breast cancer cells in an *in vitro* cytotoxicity assay.

### MODE(S) OF CARRYING OUT THE APPLICATION

In one aspect, the present application relates to isolated novel bispecific antibodies and antigen-binding fragments thereof that specifically bind to and neutralize both human HER2 and Trop-2. In another aspect, the application relates to the novel bispecific anti-HER2/Trop-2 antibodies and antigen-binding fragments thereof for use in treating conditions that would benefit from modulating HER2 and Trop-2 (e.g., cancer, persistent infectious diseases, autoimmune diseases, asthma, transplant rejection, and inflammatory disorders) . Also provided are nucleic acid molecules, and derivatives and fragments thereof, comprising a sequence of polynucleotides that encode all or a portion of a polypeptide that binds to HER2 and Trop-2, such as a nucleic acid encoding all or part of an anti-HER2/Trop-2 antibody, antibody fragment, or antibody derivative. Also provided are vectors and plasmids comprising such nucleic acids, and cells or cell lines comprising such nucleic acids and/or vectors and plasmids. Also provided are methods of making, identifying, or isolating antigen binding proteins that bind to human HER2 and Trop-2, such as anti-HER2/Trop-2 antibodies, methods of determining whether an antigen binding protein binds to Trop-2, methods of making compositions, such as pharmaceutical compositions, comprising an antigen binding protein that binds to human HER2 andTrop-2, and methods for administering an antibody, or antigen-binding fragment thereof that binds HER2 and Trop-2 to a subject, for example disclosed herein, methods for treating a condition mediated by HER2 and Trop-2.

In another aspect, the present application also relates to antibody-drug conjugates (ADC) derived from bispecific anti-HER2/Trop-2 antibodies. Also provided are methods of making ADC derived from such bispecific antibodies. Also provided are methods of using the ADC derived from bispecific antibodies, for example disclosed herein, methods of treating cancer in a subject in need thereof.

### Definitions

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those commonly used and well known in the art. The methods and techniques of the present application are generally performed according to conventional methods well known in the art and as described in various general and more specific references that are cited and discussed throughout the present specification unless otherwise indicated. See, e.g., Green and Sambrook, Molecular Cloning: A Laboratory Manual, 4th ed., Cold Spring Harbor Laboratory Press, Cold Spring Harbor, N.Y. (2012). Enzymatic reactions and purification techniques are performed according to manufacturer's specifications, as commonly accomplished in the art or as described herein. The nomenclature used in connection with, and the laboratory procedures and techniques of, analytical chemistry, synthetic organic chemistry, and medicinal and pharmaceutical chemistry described herein are those commonly used and well known in the art. Standard techniques are used for chemical syntheses, chemical analyses, pharmaceutical preparation, formulation, and delivery, and treatment of subjects.

Polynucleotide and polypeptide sequences are indicated using standard one- or three-letter abbreviations. Unless otherwise indicated, polypeptide sequences have their amino termini at the left and their carboxy termini at the right, and single-stranded nucleic acid sequences, and the top strand of double-stranded nucleic acid sequences, have their 5' termini at the left and their 3' termini at the right. A particular section of a polypeptide can be designated by amino acid residue number such as amino acids 80 to 119, or by the actual residue at that site such as Ser80 to Ser119. A particular polypeptide or polynucleotide sequence also can be described based upon how it differs from a reference sequence. Polynucleotide and polypeptide sequences of particular light and heavy chain variable domains are designated L1 ("light chain variable domain 1") and H1 ("heavy chain variable domain 1"). Antibodies comprising a light chain and heavy chain are indicated by combining the name of the light chain and the name of the heavy chain variable domains. For example, "L4H7," indicates, for example, an antibody comprising the light chain variable domain of L4 and the heavy chain variable domain of H7.

The term "antigen binding molecule" as used herein refers in its broadest sense to a molecule that specifically binds an antigenic determinant. Examples of antigen binding molecules are antibodies, antibody fragments and scaffold antigen binding proteins. An "antigen binding molecule that binds to the same epitope" as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

As used herein, the term "antigen-binding site" refers to the part of the antigen binding molecule that specifically binds to an antigenic determinant. More particularly, the term "antigen-binding site" refers the part of an antibody that comprises the area which specifically binds to and is complementary to part or all of an antigen. Where an antigen is large, an antigen binding molecule may only bind to a particular part of the antigen, which part is termed an epitope. An antigen-binding site may be provided by, for example, one or more variable domains (also called variable regions). Preferably, an antigen-binding site comprises an antibody light chain variable region (V_{L}) and an antibody heavy chain variable region (V_{H}).

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In various embodiments, the antigen is a human protein.

The term "antibody" herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term "chimeric" antibody refers to an antibody in which a portion of the heavy and/or light chain is derived from a particular source or species, while the remainder of the heavy and/or light chain is derived from a different source or species.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization. Other forms of "humanized antibodies" encompassed by the present application are those in which the constant region has been additionally modified or changed from that of the original antibody to generate the properties according to the application, especially in regard to C1q binding and/or Fc receptor (FcR) binding.

A "human" antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "monoclonal antibody" as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term "monospecific" antibody as used herein denotes an antibody that has one or more binding sites each of which bind to the same epitope of the same antigen.

The term "bispecific" means that the antibody is able to specifically bind to at least two distinct antigenic determinants, for example two binding sites each formed by a pair of an antibody heavy chain variable domain (V_{H}) and an antibody light chain variable domain (V_{L}) binding to different antigens or to different epitopes on the same antigen. Such a bispecific antibody is an 1+1 format. Other bispecific antibody formats are 2+1 formats (comprising two binding sites for a first antigen or epitope and one binding site for a second antigen or epitope) or 2+2 formats (comprising two binding sites for a first antigen or epitope and two binding sites for a second antigen or epitope). Typically, a bispecific antibody comprises two antigen binding sites, each of which is specific for a different antigenic determinant.

The term "valent" as used within the current application denotes the presence of a specified number of binding sites in an antigen binding molecule. As such, the terms "bivalent", "tetravalent", and "hexavalent" denote the presence of two binding sites, four binding sites, and six binding sites, respectively, in an antigen binding molecule. The bispecific antibodies according to the application are at least "bivalent" and may be "trivalent" or "multivalent" (e.g. "tetravalent" or "hexavalent"). In various embodiments, the antibodies of the present application have two or more binding sites and are bispecific. That is, the antibodies may be bispecific even in cases where there are more than two binding sites (i.e. that the antibody is trivalent or multivalent). In particular, the application relates to bispecific bivalent antibodies, having one binding site for each antigen they specifically bind to.

The terms "full length antibody", "full-length antibody", "intact antibody", and "whole antibody" are used herein interchangeably to refer to an antibody having a structure substantially similar to a native antibody structure. "Native antibodies" refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (V_{H}), also called a variable heavy domain or a heavy chain variable domain, followed by three constant domains (CH1, CH2, and CH3), also called a heavy chain constant region. Similarly, from N- to C-terminus, each light chain has a variable region (V_{L}), also called a variable light domain or a light chain variable domain, followed by a light chain constant domain (CL), also called a light chain constant region. The heavy chain of an antibody may be assigned to one of five types, called alpha (IgA), delta (IgD), epsilon (IgE), gamma (IgG), or mu (IgM), some of which may be further divided into subtypes, e.g., gamma 1 (IgG1), gamma 2 (IgG2), gamma 3 (IgG3), gamma 4 (IgG4), alpha 1 (IgA1) and alpha 2 (IgA2). The light chain of an antibody may be assigned to one of two types, called kappa and lambda, based on the amino acid sequence of its constant domain.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies, triabodies, tetrabodies, cross-Fab fragments; linear antibodies; single-chain antibody molecules (e.g. scFv); multispecific antibodies formed from antibody fragments and single domain antibodies. For a review of certain antibody fragments, see Hudson et al., Nat Med 9, 129-134 (2003). For a review of scFv fragments, see e.g. Pluckthun, in The Pharmacology of Monoclonal Antibodies, vol. 113, Rosenburg and Moore eds., Springer-Verlag, New York, pp. 269-315 (1994); see also WO 93/16185; and U.S. Pat. Nos. 5,571,894 and 5,587,458. For discussion of Fab and F(ab')2 fragments comprising salvage receptor binding epitope residues and having increased in vivo half-life, see U.S. Pat. No. 5,869,046. Diabodies are antibody fragments with two antigen-binding sites that may be bivalent or bispecific, see, for example, EP 404,097; WO 1993/01161; Hudson et al., Nat Med 9, 129-134

(2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, Mass.; see e.g. U.S. Pat. No. 6,248,516 B1). In addition, antibody fragments comprise single chain polypeptides having the characteristics of a V_{H} domain, namely being able to assemble together with a V_{L} domain, or of a V_{L} domain, namely being able to assemble together with a V_{H} domain to a functional antigen binding site and thereby providing the antigen binding property of full-length antibodies. Antibody fragments can be made by various techniques, including but not limited to proteolytic digestion of an intact antibody as well as production by recombinant host cells (e.g. E. coli or phage), as described herein.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term "Fab fragment" refers to an antibody fragment comprising a light chain fragment comprising a V_{L} domain and a constant domain of a light chain (CL), and a V_{H} domain and a first constant domain (CH1) of a heavy chain. Fab' fragments differ from Fab fragments by the addition of a few residues at the carboxy terminus of the heavy chain CH1 domain including one or more cysteines from the antibody hinge region. Fab'-SH are Fab' fragments wherein the cysteine residue(s) of the constant domains bear a free thiol group. Pepsin treatment yields an F(ab')₂ fragment that has two antigen-combining sites (two Fab fragments) and a part of the Fc region.

A "single chain Fab fragment" or "scFab" is a polypeptide consisting of an antibody heavy chain variable domain (V_{H}), an antibody constant domain 1 (CH1), an antibody light chain variable domain (V_{L}), an antibody light chain constant domain (CL) and a linker, wherein said antibody domains and said linker have one of the following orders in N-terminal to C-terminal direction: a) V_{L}-CH1-linker-V_{L}-CL, b) V_{L}-CL-linker-V_{H}-CH1, c) V_{H}-CL-linker-V_{L}-CH1 or d) V_{L}-CH1-linker-V_{H}-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "single-chain variable fragment (scFv)" is a fusion protein of the variable regions of the heavy (V_{H}) and light chains (V_{L}) of an antibody, connected with a short linker peptide of ten to about 25 amino acids. The linker is usually rich in glycine for flexibility, as well as serine or threonine for solubility, and can either connect the N-terminus of the V_{H} with the C-terminus of the V_{L}, or vice versa. This protein retains the specificity of the original antibody, despite removal of the constant regions and the introduction of the linker. scFv antibodies are, e.g. described in Houston, J. S., Methods in Enzymol. 203 (1991) 46-96). In addition, antibody fragments comprise single chain polypeptides having the characteristics of a V_{H} domain, namely being able to assemble together with a V_{L} domain, or of a V_{L} domain, namely being able to assemble together with a V_{H} domain to a functional antigen binding molecule and thereby providing the antigen binding property of full-length antibodies.

The term "Fc domain" or "Fc region" herein is used to define a C-terminal region of an antibody heavy chain that contains at least a portion of the constant region. The term includes native sequence Fc regions and variant Fc regions. Particularly, a human IgG heavy chain Fc region extends from Cys226, or from Pro230, to the carboxyl-terminus of the heavy chain. However, the C-terminal lysine (Lys447) of the Fc region may or may not be present. The amino acid sequences of the heavy chains are always presented with the C-terminal lysine, however variants without the C-terminal lysine are included in the application.

An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see U.S. Pat. No. 5,821,333). Such variant CH3 domains may be used to promote heterodimerization of two non-identical antibody heavy chains as herein described. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, Md., 1991.

A "region equivalent to the Fc region of an immunoglobulin" is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody-dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity (see, e.g., Bowie, J. U. et al., Science 247:1306-10 (1990)).

The term "effector functions" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

An "activating Fc receptor" is an Fc receptor that following engagement by an Fc region of an antibody elicits signaling events that stimulate the receptor-bearing cell to perform effector functions. Activating Fc receptors include FcyRIIIa (CD16a), FcyRI (CD64), FcγRIIa (CD32), and FcαRI (CD89). A particular activating Fc receptor is human FcyRIIIa (see UniProt accession no. P08637, version 141).

A "blocking" antibody or an "antagonist" antibody is one that inhibits or reduces a biological activity of the antigen it binds. In some embodiments, blocking antibodies or antagonist antibodies substantially or completely inhibit the biological activity of the antigen. For example, the bispecific antibodies of the application block the signaling through HER2 and Trop-2.

As used herein, "specific binding" is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)).

The terms "affinity" or "binding affinity" as used herein refers to the strength of the sum total of non-covalent interactions between a single binding site of a molecule (e.g. an antibody) and its binding partner (e.g. an antigen). The affinity of a molecule X for its partner Y can generally be represented by the dissociation constant (KD), which is the ratio of dissociation and association rate constants (koff and kon, respectively). A particular method for measuring affinity is Surface Plasmon Resonance (SPR). As used herein, the term "high affinity" of an antibody refers to an antibody having a Kd of 10⁻⁹ M or less and even more particularly 10⁻¹⁰ M or less for a target antigen. The term "low affinity" of an antibody refers to an antibody having a Kd of 10⁻⁸ M or higher. The term "reduced binding", as used herein refers to a decrease in affinity for the respective interaction, as measured for example by SPR. Conversely, "increased binding" refers to an increase in binding affinity for the respective interaction.

The term "a bispecific antibody comprising a first immunoglobulin antigen binding-domain that specifically binds to HER2 and a second immunoglobulin antigen binding-domain that specifically binds to Trop-2", "a bispecific antibody that specifically binds HER2 and Trop-2", "bispecific antigen binding molecule specific for HER2 and Trop-2" are used interchangeably herein and refer to a bispecific antibody that is capable of binding HER2 and Trop-2 with sufficient affinity such that the antibody is useful as a diagnostic and/or therapeutic agent in targeting HER2 and Trop-2.

The terms "anti-HER2 antibody" and "an antibody comprising an antigen-binding site that binds to HER2" refer to an antibody that is capable of binding HER2, especially a HER2 polypeptide expressed on a cell surface, with sufficient affinity such that the antibody is useful as a diagnostic and/or therapeutic agent in targeting HER2. In one embodiment, the extent of binding of an anti-HER2 antibody to an unrelated, non-HER2 protein is less than about 10% of the binding of the antibody to HER2 as measured, e.g., by radioimmunoassay (RIA) or flow cytometry (FACS) or by a Surface Plasmon Resonance assay using a biosensor system such as a Biacore^{®} system. In certain embodiments, an antigen binding molecule that binds to human HER2 has a KD value of the binding affinity for binding to human HER2 of, e.g., from 10⁻⁸ M to 10⁻¹³ M. The term "anti-HER2 antibody" also encompasses bispecific antibodies that are capable of binding HER2 and a second antigen.

The terms "anti-Trop-2 antibody" and "an antibody comprising an antigen-binding site that binds to Trop-2" refer to an antibody that is capable of binding Trop-2, especially a Trop-2 polypeptide expressed on a cell surface, with sufficient affinity such that the antibody is useful as a diagnostic and/or therapeutic agent in targeting Trop-2. In one embodiment, the extent of binding of an anti- Trop-2 antibody to an unrelated, non-Trop-2 protein is less than about 10% of the binding of the antibody to Trop-2 as measured, e.g., by radioimmunoassay (RIA) or flow cytometry (FACS) or by a Surface Plasmon Resonance assay using a biosensor system such as a Biacore^{®} system. In certain embodiments, an antigen binding molecule that binds to human Trop-2 has a KD value of the binding affinity for binding to human Trop-2 of, e.g., from 10⁻⁸ M to 10⁻¹³ M. The term "anti- Trop-2 antibody" also encompasses bispecific antibodies that are capable of binding Trop-2 and a second antigen.

The term "recombinant human antibody", as used herein, is intended to include all human antibodies that are prepared, expressed, created or isolated by recombinant means, such as antibodies expressed using a recombinant expression vector transfected into a host cell; antibodies isolated from a recombinant, combinatorial human antibody library; antibodies isolated from an animal (e.g., a mouse) that is transgenic for human immunoglobulin genes; or antibodies prepared, expressed, created or isolated by any other means that involves splicing of human immunoglobulin gene sequences to other DNA sequences. Such recombinant human antibodies have variable and constant regions derived from human germline immunoglobulin sequences. In various embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.* All such recombinant means are well known to those of ordinary skill in the art.

The term "epitope" as used herein includes any protein determinant capable of specific binding to an immunoglobulin or T-cell receptor or otherwise interacting with a molecule. Epitopic determinants generally consist of chemically active surface groupings of molecules such as amino acids or carbohydrate or sugar side chains and generally have specific three-dimensional structural characteristics, as well as specific charge characteristics. An epitope may be "linear" or "conformational." In a linear epitope, all of the points of interaction between the protein and the interacting molecule (such as an antibody) occur linearly along the primary amino acid sequence of the protein. In a conformational epitope, the points of interaction occur across amino acid residues on the protein that are separated from one another. Once a desired epitope on an antigen is determined, it is possible to generate antibodies to that epitope, e.g., using the techniques described in the present disclosure. Alternatively, during the discovery process, the generation and characterization of antibodies may elucidate information about desirable epitopes. From this information, it is then possible to competitively screen antibodies for binding to the same epitope. An approach to achieve this is to conduct cross-competition studies to find antibodies that competitively bind with one another, e.g., the antibodies compete for binding to the antigen.

An antigen binding protein, including an antibody, "specifically binds" to an antigen if it binds to the antigen with a high binding affinity as determined by a dissociation constant (K_{D}, or corresponding Kb, as defined below) value of at least 1 x 10⁻⁶ M, or at least 1 x 10⁻⁷ M, or at least 1 x 10⁻⁸ M, or at least 1 x 10⁻⁹ M, or at least 1 x 10⁻¹⁰ M, or at least 1 x 10⁻¹¹ M. An antigen binding protein that specifically binds to the human antigen of interest may be able to bind to the same antigen of interest from other species as well, with the same or different affinities. The term "K_{D}" as used herein refers to the equilibrium dissociation constant of a particular antibody-antigen interaction.

The term "immunoconjugate" or "fusion protein" as used herein refers to a molecule comprising an antibody or antigen-binding fragment thereof conjugated (or linked) directly or indirectly to an effector molecule. The term "fused" as used herein refers to components that are linked by peptide bonds, either directly or via one or more peptide linkers. The effector molecule can be a detectable label, an immunotoxin, cytokine, chemokine, therapeutic agent, or chemotherapeutic agent. The antibody or antigen-binding fragment thereof may be conjugated to an effector molecule via a peptide linker. An immunoconjugate and/or fusion protein retains the immunoreactivity of the antibody or antigen-binding fragment, e.g., the antibody or antigen-binding fragment has approximately the same, or only slightly reduced, ability to bind the antigen after conjugation as before conjugation. As used herein, an immunoconjugate may also be referred to as an antibody drug conjugate (ADC). Because immunoconjugates and/or fusion proteins are originally prepared from two molecules with separate functionalities, such as an antibody and an effector molecule, they are also sometimes referred to as "chimeric molecules."

"Linker" refers to a molecule that joins two other molecules, either covalently, or through ionic, van der Waals or hydrogen bonds, e.g., a nucleic acid molecule that hybridizes to one complementary sequence at the 5' end and to another complementary sequence at the 3' end, thus joining two non-complementary sequences. A "cleavable linker" refers to a linker that can be degraded or otherwise severed to separate the two components connected by the cleavable linker. Cleavable linkers are generally cleaved by enzymes, typically peptidases, proteases, nucleases, lipases, and the like. Cleavable linkers may also be cleaved by environmental cues, such as, for example, changes in temperature, pH, salt concentration, etc.

The term "peptide linker" as used herein refers to a peptide comprising one or more amino acids, typically about 2-20 amino acids. Peptide linkers are known in the art or are described herein. Suitable, non-immunogenic linker peptides include, for example, (G₄S)ₙ, (SG₄)ₙ or G₄(SG₄)n peptide linkers. "n" is generally a number between 1 and 10, typically between 2 and 4.

The term "immunogenicity" as used herein refers to the ability of an antibody or antigen binding fragment to elicit an immune response (humoral or cellular) when administered to a recipient and includes, for example, the human anti-mouse antibody (HAMA) response. A HAMA response is initiated when T-cells from a subject make an immune response to the administered antibody. The T-cells then recruit B-cells to generate specific "anti-antibody" antibodies.

The term "immune cell" as used herein means any cell of hematopoietic lineage involved in regulating an immune response against an antigen (e.g., an autoantigen). In various embodiments, an immune cell is, e.g., a T cell, a B cell, a dendritic cell, a monocyte, a natural killer cell, a macrophage, Langerhan's cells, or Kuffer cells.

The terms "polypeptide", "peptide" and "protein" are used interchangeably herein to refer to a polymer of amino acid residues. In various embodiments, "peptides", "polypeptides", and "proteins" are chains of amino acids whose alpha carbons are linked through peptide bonds. The terminal amino acid at one end of the chain (amino terminal) therefore has a free amino group, while the terminal amino acid at the other end of the chain (carboxy terminal) has a free carboxyl group. As used herein, the term "amino terminus" (abbreviated N-terminus) refers to the free α-amino group on an amino acid at the amino terminal of a peptide or to the α-amino group (imino group when participating in a peptide bond) of an amino acid at any other location within the peptide. Similarly, the term "carboxy terminus" refers to the free carboxyl group on the carboxy terminus of a peptide or the carboxyl group of an amino acid at any other location within the peptide. Peptides also include essentially any polyamino acid including, but not limited to, peptide mimetics such as amino acids joined by an ether as opposed to an amide bond.

The term "recombinant polypeptide", as used herein, is intended to include all polypeptides, including fusion molecules that are prepared, expressed, created, derived from, or isolated by recombinant means, such as polypeptides expressed using a recombinant expression vector transfected into a host cell.

Polypeptides of the disclosure include polypeptides that have been modified in any way and for any reason, for example, to: (1) reduce susceptibility to proteolysis, (2) reduce susceptibility to oxidation, (3) alter binding affinity for forming protein complexes, (4) alter binding affinities, and (5) confer or modify other physicochemical or functional properties. For example, single or multiple amino acid substitutions (e.g., conservative amino acid substitutions) may be made in the naturally occurring sequence (e.g., in the portion of the polypeptide outside the domain(s) forming intermolecular contacts). A "conservative amino acid substitution" refers to the substitution in a polypeptide of an amino acid with a functionally similar amino acid. The following six groups each contain amino acids that are conservative substitutions for one another:
Alanine (A), Serine (S), and Threonine (T)
Aspartic acid (D) and Glutamic acid (E)
Asparagine (N) and Glutamine (Q)
Arginine (R) and Lysine (K)
Isoleucine (I), Leucine (L), Methionine (M), and Valine (V)
Phenylalanine (F), Tyrosine (Y), and Tryptophan (W)

A "non-conservative amino acid substitution" refers to the substitution of a member of one of these classes for a member from another class. In making such changes, according to various embodiments, the hydropathic index of amino acids may be considered. Each amino acid has been assigned a hydropathic index on the basis of its hydrophobicity and charge characteristics. They are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art (see, for example, Kyte et al., 1982, J. Mol. Biol. 157:105-131). It is known that certain amino acids may be substituted for other amino acids having a similar hydropathic index or score and still retain a similar biological activity. In making changes based upon the hydropathic index, in various embodiments, the substitution of amino acids whose hydropathic indices are within ± 2 is included. In various embodiments, those that are within ± 1 are included, and in various embodiments, those within ± 0.5 are included.

It is also understood in the art that the substitution of like amino acids can be made effectively on the basis of hydrophilicity, particularly where the biologically functional protein or peptide thereby created is intended for use in immunological embodiments, as disclosed herein. In various embodiments, the greatest local average hydrophilicity of a protein, as governed by the hydrophilicity of its adjacent amino acids, correlates with its immunogenicity and antigenicity, i.e., with a biological property of the protein.

The following hydrophilicity values have been assigned to these amino acid residues: arginine (+3.0); lysine (+3.0); aspartate (+3.0.+-.1); glutamate (+3.0.+-.1); serine (+0.3); asparagine (+0.2); glutamine (+0.2); glycine (0); threonine (-0.4); proline (-0.5.+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In making changes based upon similar hydrophilicity values, in various embodiments, the substitution of amino acids whose hydrophilicity values are within ± 2 is included, in various embodiments, those that are within ± 1 are included, and in various embodiments, those within ± 0.5 are included. Exemplary amino acid substitutions are set forth in Table 1.

**Table 1**

| Original Residues | Exemplary Substitutions | Preferred Substitutions |
|---|---|---|
| Ala | Val, Leu, Ile | Val |
| Arg | Lys, Gln, Asn | Lys |
| Asn | Gln | |
| Asp | Glu | |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | |
| Trp | Tyr, Phe | Tyr |
| Tyr | Trp, Phe, Thr, Ser | Phe |
| Val | Ile, Met, Leu, Phe, | Leu |
| Ala, Norleucine | | |

The term "polypeptide fragment" and "truncated polypeptide" as used herein refers to a polypeptide that has an amino-terminal and/or carboxy-terminal deletion as compared to a corresponding full-length protein. In various embodiments, fragments can be, *e.g.,* at least 5, at least 10, at least 25, at least 50, at least 100, at least 150, at least 200, at least 250, at least 300, at least 350, at least 400, at least 450, at least 500, at least 600, at least 700, at least 800, at least 900 or at least 1000 amino acids in length. In various embodiments, fragments can also be, *e.g.,* at most 1000, at most 900, at most 800, at most 700, at most 600, at most 500, at most 450, at most 400, at most 350, at most 300, at most 250, at most 200, at most 150, at most 100, at most 50, at most 25, at most 10, or at most 5 amino acids in length. A fragment can further comprise, at either or both of its ends, one or more additional amino acids, for example, a sequence of amino acids from a different naturally-occurring protein (*e.g.,* an Fc or leucine zipper domain) or an artificial amino acid sequence (*e.g.,* an artificial linker sequence).

The terms "polypeptide variant" and "polypeptide mutant" as used herein refers to a polypeptide that comprises an amino acid sequence wherein one or more amino acid residues are inserted into, deleted from and/or substituted into the amino acid sequence relative to another polypeptide sequence. In various embodiments, the number of amino acid residues to be inserted, deleted, or substituted can be, *e.g.,* at least 1, at least 2, at least 3, at least 4, at least 5, at least 10, at least 25, at least 50, at least 75, at least 100, at least 125, at least 150, at least 175, at least 200, at least 225, at least 250, at least 275, at least 300, at least 350, at least 400, at least 450 or at least 500 amino acids in length. Variants of the present disclosure include fusion proteins.

A "derivative" of a polypeptide is a polypeptide that has been chemically modified, e.g., conjugation to another chemical moiety such as, for example, polyethylene glycol, albumin (e.g., human serum albumin), phosphorylation, and glycosylation.

The term "% sequence identity" is used interchangeably herein with the term "% identity" and refers to the level of amino acid sequence identity between two or more peptide sequences or the level of nucleotide sequence identity between two or more nucleotide sequences, when aligned using a sequence alignment program. For example, as used herein, 80% identity means the same thing as 80% sequence identity determined by a defined algorithm and means that a given sequence is at least 80% identical to another length of another sequence. In various embodiments, the % identity is selected from, e.g., at least 60%, at least 65%, at least 70%, at least 75%, at least 80%, at least 85%, at least 90%, at least 95%, or at least 99% or more sequence identity to a given sequence. In various embodiments, the % identity is in the range of, e.g., about 60% to about 70%, about 70% to about 80%, about 80% to about 85%, about 85% to about 90%, about 90% to about 95%, or about 95% to about 99%.

The term "% sequence homology" is used interchangeably herein with the term "% homology" and refers to the level of amino acid sequence homology between two or more peptide sequences or the level of nucleotide sequence homology between two or more nucleotide sequences, when aligned using a sequence alignment program. For example, as used herein, 80% homology means the same thing as 80% sequence homology determined by a defined algorithm, and accordingly a homologue of a given sequence has greater than 80% sequence homology over a length of the given sequence. In various embodiments, the % homology is selected from, e.g., at least 60%, at least 65%, at least 70%, at least 75%, at least 80%, at least 85%, at least 90%, at least 95%, or at least 99% or more sequence homology to a given sequence. In various embodiments, the % homology is in the range of, e.g., about 60% to about 70%, about 70% to about 80%, about 80% to about 85%, about 85% to about 90%, about 90% to about 95%, or about 95% to about 99%.

Exemplary computer programs which can be used to determine identity between two sequences include, but are not limited to, the suite of BLAST programs, e.g., BLASTN, BLASTX, and TBLASTX, BLASTP and TBLASTN, publicly available on the Internet at the NCBI website. See also Altschul et al., J. Mol. Biol. 215:403-10, 1990 (with special reference to the published default setting, i.e., parameters w=4, t=17) and Altschul et al., Nucleic Acids Res., 25:3389-3402, 1997. Sequence searches are typically carried out using the BLASTP program when evaluating a given amino acid sequence relative to amino acid sequences in the GenBank Protein Sequences and other public databases. The BLASTX program is preferred for searching nucleic acid sequences that have been translated in all reading frames against amino acid sequences in the GenBank Protein Sequences and other public databases. Both BLASTP and BLASTX are run using default parameters of an open gap penalty of 11.0, and an extended gap penalty of 1.0, and utilize the BLOSUM-62 matrix. See Id.

In addition to calculating percent sequence identity, the BLAST algorithm also performs a statistical analysis of the similarity between two sequences (see, e.g., Karlin & Altschul, Proc. Nat'l. Acad. Sci. USA, 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is, *e.g.,* less than about 0.1, less than about 0.01, or less than about 0.001.

The terms "substantial similarity" or "substantially similar," in the context of polypeptide sequences, indicate that a polypeptide region has a sequence with at least 70%, typically at least 80%, more typically at least 85%, or at least 90% or at least 95% sequence similarity to a reference sequence. For example, a polypeptide is substantially similar to a second polypeptide, for example, where the two peptides differ by one or more conservative substitution(s).

"Polynucleotide" refers to a polymer composed of nucleotide units. Polynucleotides include naturally occurring nucleic acids, such as deoxyribonucleic acid ("DNA") and ribonucleic acid ("RNA") as well as nucleic acid analogs. Nucleic acid analogs include those which include non-naturally occurring bases, nucleotides that engage in linkages with other nucleotides other than the naturally occurring phosphodiester bond or which include bases attached through linkages other than phosphodiester bonds. Thus, nucleotide analogs include, for example and without limitation, phosphorothioates, phosphorodithioates, phosphorotriesters, phosphoramidates, boranophosphates, methylphosphonates, chiral-methyl phosphonates, 2-O-methyl ribonucleotides, peptide-nucleic acids (PNAs), and the like. Such polynucleotides can be synthesized, for example, using an automated DNA synthesizer. The term "nucleic acid" typically refers to large polynucleotides. The term "oligonucleotide" typically refers to short polynucleotides, generally no greater than about 50 nucleotides. It will be understood that when a nucleotide sequence is represented by a DNA sequence (i.e., A, T, G, C), this also includes an RNA sequence (i.e., A, U, G, C) in which "U" replaces "T."

Conventional notation is used herein to describe polynucleotide sequences: the left-hand end of a single-stranded polynucleotide sequence is the 5'-end; the left-hand direction of a double-stranded polynucleotide sequence is referred to as the 5'-direction. The direction of 5' to 3' addition of nucleotides to nascent RNA transcripts is referred to as the transcription direction. The DNA strand having the same sequence as an mRNA is referred to as the "coding strand"; sequences on the DNA strand having the same sequence as an mRNA transcribed from that DNA and which are located 5' to the 5'-end of the RNA transcript are referred to as "upstream sequences"; sequences on the DNA strand having the same sequence as the RNA and which are 3' to the 3' end of the coding RNA transcript are referred to as "downstream sequences."

"Complementary" refers to the topological compatibility or matching together of interacting surfaces of two polynucleotides. Thus, the two molecules can be described as complementary, and furthermore, the contact surface characteristics are complementary to each other. A first polynucleotide is complementary to a second polynucleotide if the nucleotide sequence of the first polynucleotide is substantially identical to the nucleotide sequence of the polynucleotide binding partner of the second polynucleotide, or if the first polynucleotide can hybridize to the second polynucleotide under stringent hybridization conditions.

"Hybridizing specifically to" or "specific hybridization" or "selectively hybridize to", refers to the binding, duplexing, or hybridizing of a nucleic acid molecule preferentially to a particular nucleotide sequence under stringent conditions when that sequence is present in a complex mixture (e.g., total cellular) DNA or RNA. The term "stringent conditions" refers to conditions under which a probe will hybridize preferentially to its target subsequence, and to a lesser extent to, or not at all to, other sequences. "Stringent hybridization" and "stringent hybridization wash conditions" in the context of nucleic acid hybridization experiments such as Southern and northern hybridizations are sequence-dependent and are different under different environmental parameters. An extensive guide to the hybridization of nucleic acids can be found in Tijssen, 1993, Laboratory Techniques in Biochemistry and Molecular Biology--Hybridization with Nucleic Acid Probes, part I, chapter 2, "Overview of principles of hybridization and the strategy of nucleic acid probe assays", Elsevier, N.Y.; Sambrook et al., 2001, Molecular Cloning: A Laboratory Manual, Cold Spring Harbor Laboratory, 3.sup.rd ed., NY; and Ausubel et al., eds., Current Edition, Current Protocols in Molecular Biology, Greene Publishing Associates and Wiley Interscience, NY.

Generally, highly stringent hybridization and wash conditions are selected to be about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Very stringent conditions are selected to be equal to the Tm for a particular probe. An example of stringent hybridization conditions for hybridization of complementary nucleic acids which have more than about 100 complementary residues on a filter in a Southern or northern blot is 50% formalin with 1 mg of heparin at 42°C, with the hybridization being carried out overnight. An example of highly stringent wash conditions is 0.15 M NaCl at 72°C for about 15 minutes. An example of stringent wash conditions is a 0.2 x SSC wash at 65°C for 15 minutes. See Sambrook et al. for a description of SSC buffer. A high stringency wash can be preceded by a low stringency wash to remove background probe signal. An exemplary medium stringency wash for a duplex of, e.g., more than about 100 nucleotides, is 1 x SSC at 45°C for 15 minutes. An exemplary low stringency wash for a duplex of, e.g., more than about 100 nucleotides, is 4-6 x SSC at 40°C for 15 minutes. In general, a signal to noise ratio of 2 x (or higher) than that observed for an unrelated probe in the particular hybridization assay indicates detection of a specific hybridization.

"Primer" refers to a polynucleotide that is capable of specifically hybridizing to a designated polynucleotide template and providing a point of initiation for synthesis of a complementary polynucleotide. Such synthesis occurs when the polynucleotide primer is placed under conditions in which synthesis is induced, i.e., in the presence of nucleotides, a complementary polynucleotide template, and an agent for polymerization such as DNA polymerase. A primer is typically single-stranded but may be double-stranded. Primers are typically deoxyribonucleic acids, but a wide variety of synthetic and naturally occurring primers are useful for many applications. A primer is complementary to the template to which it is designed to hybridize to serve as a site for the initiation of synthesis but need not reflect the exact sequence of the template. In such a case, specific hybridization of the primer to the template depends on the stringency of the hybridization conditions. Primers can be labeled with, e.g., chromogenic, radioactive, or fluorescent moieties and used as detectable moieties.

"Probe," when used in reference to a polynucleotide, refers to a polynucleotide that is capable of specifically hybridizing to a designated sequence of another polynucleotide. A probe specifically hybridizes to a target complementary polynucleotide but need not reflect the exact complementary sequence of the template. In such a case, specific hybridization of the probe to the target depends on the stringency of the hybridization conditions. Probes can be labeled with, e.g., chromogenic, radioactive, or fluorescent moieties and used as detectable moieties. In instances where a probe provides a point of initiation for synthesis of a complementary polynucleotide, a probe can also be a primer.

A "vector" is a polynucleotide that can be used to introduce another nucleic acid linked to it into a cell. One type of vector is a "plasmid," which refers to a linear or circular double stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) are integrated into the genome of a host cell upon introduction into the host cell, and thereby are replicated along with the host genome. An "expression vector" is a type of vector that can direct the expression of a chosen polynucleotide.

A "regulatory sequence" is a nucleic acid that affects the expression (e.g., the level, timing, or location of expression) of a nucleic acid to which it is operably linked. The regulatory sequence can, for example, exert its effects directly on the regulated nucleic acid, or through the action of one or more other molecules (e.g., polypeptides that bind to the regulatory sequence and/or the nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology 185, Academic Press, San Diego, Calif. and Baron et al., 1995, Nucleic Acids Res. 23:3605-06. A nucleotide sequence is "operably linked" to a regulatory sequence if the regulatory sequence affects the expression (e.g., the level, timing, or location of expression) of the nucleotide sequence.

A "host cell" is a cell that can be used to express a polynucleotide of the disclosure. A host cell can be a prokaryote, for example, *E. coli,* or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Typically, a host cell is a cultured cell that can be transformed or transfected with a polypeptide-encoding nucleic acid, which can then be expressed in the host cell. The phrase "recombinant host cell" can be used to denote a host cell that has been transformed or transfected with a nucleic acid to be expressed. A host cell also can be a cell that comprises the nucleic acid but does not express it at a desired level unless a regulatory sequence is introduced into the host cell such that it becomes operably linked with the nucleic acid. It is understood that the term host cell refers not only to the particular subject cell but to the progeny or potential progeny of such a cell. Because certain modifications may occur in succeeding generations due to, e.g., mutation or environmental influence, such progeny may not, in fact, be identical to the parent cell, but are still included within the scope of the term as used herein.

The term "isolated molecule" (where the molecule is, for example, a polypeptide or a polynucleotide) is a molecule that by virtue of its origin or source of derivation (1) is not associated with naturally associated components that accompany it in its native state, (2) is substantially free of other molecules from the same species (3) is expressed by a cell from a different species, or (4) does not occur in nature. Thus, a molecule that is chemically synthesized, or expressed in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by a number of means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

A protein or polypeptide is "substantially pure," "substantially homogeneous," or "substantially purified" when at least about 60% to 75% of a sample exhibits a single species of polypeptide. The polypeptide or protein may be monomeric or multimeric. A substantially pure polypeptide or protein will typically comprise about 50%, 60%, 70%, 80% or 90% W/W of a protein sample, more usually about 95%, and preferably will be over 99% pure. Protein purity or homogeneity may be indicated by a number of means well known in the art, such as polyacrylamide gel electrophoresis of a protein sample, followed by visualizing a single polypeptide band upon staining the gel with a stain well known in the art. For certain purposes, higher resolution may be provided by using HPLC or other means well known in the art for purification.

The terms "label" or "labeled" as used herein refers to incorporation of another molecule in the antibody. In one embodiment, the label is a detectable marker, e.g., incorporation of a radiolabeled amino acid or attachment to a polypeptide of biotinyl moieties that can be detected by marked avidin (e.g., streptavidin containing a fluorescent marker or enzymatic activity that can be detected by optical or calorimetric methods). In another embodiment, the label or marker can be therapeutic, e.g., a drug conjugate or toxin. Various methods of labeling polypeptides and glycoproteins are known in the art and may be used. Examples of labels for polypeptides include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent labels (e.g., FITC, rhodamine, lanthanide phosphors), enzymatic labels (e.g., horseradish peroxidase, β-galactosidase, luciferase, alkaline phosphatase), chemiluminescent markers, biotinyl groups, predetermined polypeptide epitopes recognized by a secondary reporter (e.g., leucine zipper pair sequences, binding sites for secondary antibodies, metal binding domains, epitope tags), magnetic agents, such as gadolinium chelates, toxins such as pertussis toxin, taxol, cytochalasin B, gramicidin D, ethidium bromide, emetine, mitomycin, etoposide, tenoposide, vincristine, vinblastine, colchicine, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. In some embodiments, labels are attached by spacer arms of various lengths to reduce potential steric hindrance.

As used herein, the term "immunotherapy" refers to cancer treatments which include, but are not limited to, treatment using depleting antibodies to specific tumor antigens; treatment using antibody-drug conjugates; treatment using agonistic, antagonistic, or blocking antibodies to co-stimulatory or co-inhibitory molecules (immune checkpoints) such as PD-1, PD-L1, OX-40, CD137, GITR, LAG3, TIM-3, and VISTA; treatment using bispecific T cell engaging antibodies (BiTE^{®}) such as blinatumomab: treatment involving administration of biological response modifiers such as IL-2, IL-12, IL-15, IL-21, GM-CSF, IFN-α, IFN-β and IFN-γ; treatment using therapeutic vaccines such as sipuleucel-T; treatment using dendritic cell vaccines, or tumor antigen peptide vaccines; treatment using chimeric antigen receptor (CAR)-T cells; treatment using CAR-NK cells; treatment using tumor infiltrating lymphocytes (TILs); treatment using adoptively transferred anti-tumor T cells (ex vivo expanded and/or TCR transgenic); treatment using TALL-104 cells; and treatment using immunostimulatory agents such as Toll-like receptor (TLR) agonists CpG and imiquimod.

"Pharmaceutical composition" refers to a composition suitable for pharmaceutical use in an animal. A pharmaceutical composition comprises a pharmacologically effective amount of an active agent and a pharmaceutically acceptable carrier. "Pharmacologically effective amount" refers to that amount of an agent effective to produce the intended pharmacological result. "Pharmaceutically acceptable carrier" refers to any of the standard pharmaceutical carriers, vehicles, buffers, and excipients, such as a phosphate buffered saline solution, 5% aqueous solution of dextrose, and emulsions, such as an oil/water or water/oil emulsion, and various types of wetting agents and/or adjuvants. Suitable pharmaceutical carriers and formulations are described in Remington's Pharmaceutical Sciences, 21st Ed. 2005, Mack Publishing Co, Easton. A "pharmaceutically acceptable salt" is a salt that can be formulated into a compound for pharmaceutical use including, e.g., metal salts (sodium, potassium, magnesium, calcium, etc.) and salts of ammonia or organic amines.

The terms "growth suppression" or "growth inhibition" of a cell expressing HER2 or Trop-2, as used herein, refer to the ability of anti-HER2/Trop-2 binding molecule, e.g., an anti-HER2/Trop-2 antibody or a HER2/Trop-2-binding molecule comprising an antigen-binding fragment thereof, to statistically significantly decrease proliferation of cells expressing HER2 or Trop-2 relative to the proliferation in the absence of the anti-HER2/Trop-2 binding molecule. In one aspect, the proliferation of cells expressing HER2 or Trop-2 (e.g., cancer cells) can be decreased by at least 10%, or at least 20%, or at least 30%, or at least 40%, or at least 50%, or at least 60%, or at least 70%, or at least 80%, or at least 90%, or about 100% when cells are contacted with an anti-HER2/Trop-2 binding molecule, e.g. , an anti-HER2/Trop-2 antibody or a HER2/Trop-2- binding molecule comprising an antigen-binding fragment thereof, relative to the proliferation measured in the absence of the anti-HER2/Trop-2 binding molecule (control conditions). Cell proliferation can be measured according to various methods known in the art, e.g., by counting the number of viable cells, identifying the presence of markers of growth markers, measuring the incorporation of molecules (e.g., radioactively labelled molecules such a H-thymidine), by measuring the size of a tumor (e.g, by volume or by weight), etc. In certain aspects, growth suppression refers to reduction in the number, size, or distribution of metastases.

As used herein, "treatment" (and grammatical variations thereof such as "treat" or "treating") refers to clinical intervention in an attempt to alter the natural course of a disease in the individual being treated and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. As used herein, to "alleviate" a disease, disorder or condition means reducing the severity and/or occurrence frequency of the symptoms of the disease, disorder, or condition. Further, references herein to "treatment" include references to curative, palliative and prophylactic treatment.

The term "effective amount" or "therapeutically effective amount" as used herein refers to an amount of a compound or composition sufficient to treat a specified disorder, condition or disease such as ameliorate, palliate, lessen, and/or delay one or more of its symptoms. In reference to cancers or other unwanted cell proliferation, an effective amount comprises an amount sufficient to: (i) reduce the number of cancer cells; (ii) reduce tumor size; (iii) inhibit, retard, slow to some extent and preferably stop cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer. An effective amount can be administered in one or more administrations.

The phrase "administering" or "cause to be administered" refers to the actions taken by a medical professional (*e.g.,* a physician), or a person controlling medical care of a patient, that control and/or permit the administration of the agent(s)/compound(s) at issue to the patient. Causing to be administered can involve diagnosis and/or determination of an appropriate therapeutic regimen, and/or prescribing particular agent(s)/compounds for a patient. Such prescribing can include, for example, drafting a prescription form, annotating a medical record, and the like. Where administration is described herein, "causing to be administered" is also contemplated.

The terms "patient," "individual," and "subject" may be used interchangeably and refer to a mammal, preferably a human or a non-human primate, but also domesticated mammals (*e.g.,* canine or feline), laboratory mammals (*e.g.,* mouse, rat, rabbit, hamster, guinea pig), and agricultural mammals (*e.g.,* equine, bovine, porcine, ovine). In various embodiments, the patient can be a human (*e.g.,* adult male, adult female, adolescent male, adolescent female, male child, female child) under the care of a physician or other health worker in a hospital, psychiatric care facility, as an outpatient, or other clinical context. In various embodiments, the patient may be an immunocompromised patient or a patient with a weakened immune system including, but not limited to patients having primary immune deficiency, AIDS; cancer and transplant patients who are taking certain immunosuppressive drugs; and those with inherited diseases that affect the immune system (e.g., congenital agammaglobulinemia, congenital IgA deficiency). In various embodiments, the patient has an immunogenic cancer, including, but not limited to bladder cancer, lung cancer, melanoma, and other cancers reported to have a high rate of mutations (Lawrence et al., Nature, 499(7457): 214-218, 2013).

"Resistant or refractory cancer" refers to tumor cells or cancer that do not respond to previous anti-cancer therapy including, e.g., chemotherapy, surgery, radiation therapy, stem cell transplantation, and immunotherapy. Tumor cells can be resistant or refractory at the beginning of treatment, or they may become resistant or refractory during treatment. Refractory tumor cells include tumors that do not respond at the onset of treatment or respond initially for a short period but fail to respond to treatment. Refractory tumor cells also include tumors that respond to treatment with anticancer therapy but fail to respond to subsequent rounds of therapies. For purposes of this application, refractory tumor cells also encompass tumors that appear to be inhibited by treatment with anticancer therapy but recur up to five years, sometimes up to ten years or longer after treatment is discontinued. The anticancer therapy can employ chemotherapeutic agents alone, radiation alone, targeted therapy alone, surgery alone, or combinations thereof. For ease of description and not limitation, it will be understood that the refractory tumor cells are interchangeable with resistant tumor.

It is understood that aspects and embodiments of the application described herein include "consisting" and/or "consisting essentially of" aspects and embodiments.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the application described herein include "consisting" and/or "consisting essentially of" aspects and variations.

### HER2 Antigen

HER2 (also known as CD340 or ERBB2), a member of the human epidermal growth factor receptor family, is aberrantly expressed in certain malignancies, including breast gastric, and lung cancer, primarily due to HER2 genomic amplification. HER2 over-expression is associated with increased risk of recurrence and poorer prognosis in these cancers. In HER2-positive malignancies, this protein can stimulate downstream RAS-RAF-ERK and PI3K-PTEN-AKT signaling and play a key role in cell proliferation. Therefore, HER2 is a preferred therapeutic target in cancers. Numerous antibodies targeting HER2 have been developed. For example, anti-HER2 monoclonal antibodies such as trastuzumab and pertuzumab and HER2 tyrosine kinase inhibitors such as lapatinib have been shown to improve survival and are used as standard therapies for HER2-positive cancers, including those of the breast and stomach.

ADCs comprising an anti-HER2 monoclonal antibody, a linker and a cytotoxic agent payload are also employed as a targeted therapy for HER2-positive malignancies. The trastuzumab emtansine (T-DM1), which consists of the anti-HER2 monoclonal antibody trastuzumab conjugated to the tubulin polymerization inhibitor DM1, is already in use for the treatment of HER2-positive breast cancer. In its phase III clinical trial (the EMILIA study), T-DM1 was found to improve the survival and quality of life of HER2-positive breast cancer patients. Furthermore, 44% of patients responded to T-DM1 treatment. However, its efficacy was limited in other cases, and all patients eventually develop T-DM1 resistance. There is a need for better understanding of the mechanism underlying this limitation which could improve the treatment of patients with HER2-positive cancer.

Trastuzumab deruxtecan (DS-8201a) is another HER2-targeting ADC prepared using a novel linker-payload system with a potent topoisomerase I inhibitor, exatecan derivative (DX-8951 derivative, DXd). It was effective against T-DM1-insensitive patient-derived xenograft models with both high and low HER2 expression. DS-8201a has a potent bystander effect due to a highly membrane-permeable payload and is beneficial in treating tumors with HER2 heterogeneity that are unresponsive to T-DM1. DS-8201a was recently approved by FDA for unresectable or pretreated metasatic HER2-positive breast cacner. The approval was based on the phase II DESTINY-Breast01 trial which included HER2-positive breast cancer patients that had been previously treated with other HER2 therapies. Overall, 60.9% patients responded to this new HER2 ADC. Besides breast cancer, DS-8201a has demonstrated good clinical efficacy in other HER2 positive cancer types such as lung and gastric cancers. Although there is the high rate of durable responses observed, the clinical benefit on overall survival remains to be dertermined with DS-8201a.

The term "HER2" as used herein includes human HER2 (hHER2), variants, isoforms, and species homologs of hHER2, and analogs having at least one common epitope with hHER2. In various embodiments, a hHER2 polypeptide as used herein may comprise the amino acid sequence set forth in NCBI Reference Sequence: NP_001005862 (SEQ ID NO: 1):

MKLRLPASPETHLDMLRHLYQGCQVVQGNLELTYLPTNASLSFLQDIQEVQGYVLIA
HNQVRQVPLQRLRIVRGTQLFEDNYALAVLDNGDPLNNTTPVTGASPGGLRELQLRS
LTEILKGGVLIQRNPQLCYQDTILWKDIFHKNNQLALTLIDTNRSRACHPCSPMCKGS
RCWGESSEDCQSLTRTVCAGGCARCKGPLPTDCCHEQCAAGCTGPKHSDCLACLHF
NHSGICELHCPALVTYNTDTFESMPNPEGRYTFGASCVTACPYNYLSTDVGSCTLVC
PLHNQEVTAEDGTQRCEKCSKPCARVCYGLGMEHLREVRAVTSANIQEFAGCKKIF
GSLAFLPESFDGDPASNTAPLQPEQLQVFETLEEITGYLYISAWPDSLPDLSVFQNLQV
IRGRILHNGAYSLTLQGLGISWLGLRSLRELGSGLALIHHNTHLCFVHTVPWDQLFRN
PHQALLHTANRPEDECVGEGLACHQLCARGHCWGPGPTQCVNCSQFLRGQECVEE
CRVLQGLPREYVNARHCLPCHPECQPQNGSVTCFGPEADQCVACAHYKDPPFCVAR
CPSGVKPDLSYMPIWKFPDEEGACQPCPINCTHSCVDLDDKGCPAEQRASPLTSIISA
VVGILLVVVLGVVFGILIKRRQQKIRKYTMRRLLQETELVEPLTPSGAMPNQAQMRIL
KETELRKVKVLGSGAFGTVYKGIWIPDGENVKIPVAIKVLRENTSPKANKEILDEAYV
MAGVGSPYVSRLLGICLTSTVQLVTQLMPYGCLLDHVRENRGRLGSQDLLNWCMQI
AKGMSYLEDVRLVHRDLAARNVLVKSPNHVKITDFGLARLLDIDETEYHADGGKVPI

KWMALESILRRRFTHQSDVWSYGVTVWELMTFGAKPYDGIPAREIPDLLEKGERLP
QPPICTIDVYMIMVKCWMIDSECRPRFRELVSEFSRMARDPQRFVVIQNEDLGPASPL
DSTFYRSLLEDDDMGDLVDAEEYLVPQQGFFCPDPAPGAGGMVHHRHRSSSTRSGG
GDLTLGLEPSEEEAPRSPLAPSEGAGSDVFDGDLGMGAAKGLQSLPTHDPSPLQRYS
EDPTVPLPSETDGYVAPLTCSPQPEYVNQPDVRPQPPSPREGPLPAARPAGATLERPK
TLSPGKNGVVKDVFAFGGAVENPEYLTPQGGAAPQPHPPPAFSPAFDNLYYWDQDP
PERGAPPSTFKGTPTAENPEYLGLDVPV (SEQ ID NO: 1)

In various embodiments, a HER2 polypeptide comprises an amino acid sequence that shares an observed homology of, *e.g.,* at least 70%, at least 75%, at least 80%, at least 85%, at least 90%, at least 95%, at least 96%, at least 97%, at least 98%, or at least 99% with the human HER2 sequence of SEQ ID NO: 1. Polypeptide variants of HER2 may be described herein by reference to the addition, deletion, or substitution of amino acid residue present at a given position in the 223 amino acid sequence of SEQ ID NO: 1. Thus, for example, the term "P21W" indicates that the "P" (proline, in standard single letter code) residue at position 21 in SEQ ID NO: 1 has been substituted with a "W" (tryptophan, in standard single letter code).

### Trop-2 Antigen

Trophoblast cell-surface antigen-2 (Trop-2) (also known as tumor-associated calcium signal transducer 2 (TACSTD1), membrane component chromosome 1 surface marker 1 (M1S1), gastrointestinal antigen 733-1 (GA733-1), and epithelial glycoprotein-1 (EGP-1)), belongs to the TACSTD family including at least two type I membrane proteins. Trop-2 belongs to the TACSTD family including at least two type I membrane proteins. It transduces an intracellular calcium signal and acts as a cell surface receptor. It has 323 amino acids, comprised of one large extracellular domain, one single transmembrane domain, and a short cytoplasmic tail.

Although it was first discovered as a cell surface marker of trophoblast cells, subsequent reports reveal Trop-2 is also expressed at a low level in limited normal tissues such as the nasal, breast, skin, and bronchial epithelial cells. Further studies have suggested that Trop-2 is overexpressed in many different cancer types including oral, head-and-neck, thyroid, lung, breast, gastric, colorectal, pancreatic, renal, prostate, ovarian, uterine, cervical cancers, and glioma. The elevated expression is associated with disease progression and a poor prognosis in cancer patients. Overexpression of Trop-2 in cancer cells has been shown to stimulate tumor growth both in vitro and in vivo. Similarly, inhibition of Trop-2 expression by means of siRNA has been shown to inhibit tumor cell proliferation. In addition to being critical for tumor growth, Trop-2 is also involved in metastasis. There are at least six major signaling pathways involving Trop-2, including IGF, ErbB3, ERK, MAPK, Notch-Wnt, and Raf pathways. However, its precise role in these pathways, and which downstream pathways are critical in different cancers and in different therapeutic approaches remain to be elucidated.

Based on its differential expression in tumors vs. normal tissues, its role in promoting tumor growth and metastasis, and the negative prognostic value, Trop-2 has been proposed as a promising diagnostic/therapeutic target. Blockade of Trop-2 signaling may be a means for treating cancer. A Trop-2-targeted antigen-binding fragment (Fab) has been shown to induce apoptosis and have inhibitory effects on breast cancer cell proliferation. Although several anti-Trop-2 antibodies have been developed, none was amenable to therapeutic use as naked antibodies. Trop-2-targeted antibody-drug conjugates (ADC) have been developed recently. Sacituzumab govitecan (IMMU-132) is a conjugate of humanized anti-Trop-2 antibody hRS7 with SN-38, the active metabolite of irinotecan. It has been developed and tested in many pre-clinical studies and was able to inhibit growth of a wide range of tumors. It also showed encouraging efficacy in the early phase clinical trials in non-small cell lung cancer, small cell lung cancer, metastatic triple-negative breast cancer, and pancreatic cancer patients. In the phase II IMMU-132-01 clinical trial, IMMU-132 showed 33.3% overall response rate in patients with metastatic triple-negative breast cancer who had failed previous lines of treatments. The median duration of response was 7.7 months. Based on the results, IMMU-132 became the first FDA-approved anti-Trop-2 ADC. Although this anti-Trop-2- ADC demonstrates good clinical effect, there remains a need for improved therapeutic agents using anti-Trop-2 antibodies for the treatment of cancer and other diseases associated with Trop-2 activity.

The term "Trop-2" as used herein includes human Trop-2 (hTrop-2), variants, isoforms, and species homologs of hTrop-2, and analogs having at least one common epitope with hTrop-2. In various embodiments, a hTrop-2 polypeptide as used herein may comprise the amino acid sequence set forth in NCBI Reference Sequence: NP_002344.2 (SEQ ID NO: 2):

MARGPGLAPPPLRLPLLLLVLAAVTGHTAAQDNCTCPTNKMTVCSPDGPGGR
CQCALGSGMAVDCSTLTSKCLLLKARMSAPKNARTLVRPSEHALVDNDGLYDPDC
DPEGRFKARQCNQTSVCWCVNSVGVRRTDKGDLSLRCDELVRTHHILIDLRHRPTA
GAFNHSDLDAELRRLFRERYRLHPKFVAAVHYEQPTIQIELRQNTSQKAAGDVDIGD
AAYYFERDIKGESLFQGRGGLDLRVRGEPLQVERTLIYYLDEIPPKFSMKRLTAGLIA
VIVV VVVALVAGMAVLVITNRRKS GKYKKVEIKELGELRKEPSL (SEQ ID NO: 2)

In various embodiments, a Trop-2 polypeptide comprises an amino acid sequence that shares an observed homology of, *e.g.,* at least 70%, at least 75%, at least 80%, at least 85%, at least 90%, at least 95%, at least 96%, at least 97%, at least 98%, or at least 99% with the human Trop-2 sequence of SEQ ID NO: 2. Polypeptide variants of Trop-2 may be described herein by reference to the addition, deletion, or substitution of amino acid residue present at a given position in the 223 amino acid sequence of SEQ ID NO: 2. Thus, for example, the term "P21W" indicates that the "P" (proline, in standard single letter code) residue at position 21 in SEQ ID NO: 2 has been substituted with a "W" (tryptophan, in standard single letter code).

### Antibodies

Antibodies can be engineered in numerous ways. They can be made as single-chain antibodies (including small modular immunopharmaceuticals or SMIPs^{™}), Fab and F(ab')₂ fragments, etc. Antibodies can be humanized, chimerized, deimmunized, or fully human. Numerous publications set forth the many types of antibodies and the methods of engineering such antibodies. For example, see U.S. Pat. Nos. 6,355,245; 6,180,370; 5,693,762; 6,407,213; 6,548,640; 5,565,332; 5,225,539; 6,103,889; and 5,260,203.

Methods of generating novel antibodies that bind to human Trop-2 are known to those skilled in the art. The present inventors have generated novel antibodies that bind to human Trop-2. Briefly, a method for generating a monoclonal antibody that binds specifically to Trop-2 may comprise administering to a mouse an amount of an immunogenic composition comprising the Trop-2 effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monoclonal antibody that binds specifically to the Trop-2. Once obtained, a hybridoma can be propagated in a cell culture, optionally in culture conditions where the hybridoma-derived cells produce the monoclonal antibody that binds specifically to Trop-2. The monoclonal antibody may be purified from the cell culture. Chimeric antibodies are produced by recombinant DNA techniques. A CDR grafting and back mutation method was used to prepare humanized antibodies.

Other suitable methods of producing or isolating antibodies of the requisite specificity can used, including, for example, methods which select recombinant antibody from a library, or which rely upon immunization of transgenic animals (e.g., mice) capable of producing a full repertoire of human antibodies. See e.g., Jakobovits et al., Proc. Natl. Acad. Sci. USA, 90: 2551-2555, 1993; Jakobovits et al., Nature, 362:255-258, 1993; Lonberg et al., U.S. Pat. No. 5,545,806; Surani et al., U.S. Patent No. 5,545,807.

### Exemplary Constructs

The present invention provides constructs comprising a first antigen-binding domain that specifically binds to human HER2 , and a second antigen-binding domain that specifically binds to human Trop-2 .

In some embodiments, the present invention provides a construct comprising a first antigen-binding domain that specifically binds to human HER2 comprising a first heavy chain variable region (V_{H-1}) and a first light chain variable region (V_{L-1}), and/or a second antigen-binding domain that specifically binds human Trop-2 comprising a second heavy chain variable region (V_{H-2}) and a second light chain variable region (V_{L-2}), wherein the first antigen-binding domain comprises a scFv comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a full-length antibody with a Fc fragment comprising 1) the V_{H-2} comprising a HC-CDR1 comprising the amino acid sequence set forth in SEQ ID NO: 26, a HC-CDR2 comprising the amino acid sequence set forth in SEQ ID NO: 27, and a HC-CDR3 comprising the amino acid sequence set forth in SEQ ID NO: 28, and 2) the V_{L-2} comprising a LC-CDR1 comprising the amino acid sequence set forth in SEQ ID NO: 29, a LC-CDR2 comprising the amino acid sequence set forth in SEQ ID NO: 30, and a LC-CDR3 comprising the amino acid sequence set forth in SEQ ID NO: 31, and the first antigen-binding domain is fused to N-terminus of both heavy chains of the full-length antibody. In some embodiments, the first antigen-binding domain comprises a scFv comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a full-length antibody with a Fc fragment comprising 1) the V_{H-2} comprising the amino acid sequence set forth in SEQ ID NO: 4, and 2) the V_{L-2} comprising the amino acid sequence set forth in SEQ ID NO: 5, and the first antigen-binding domain is fused to N-terminus of both heavy chains of the full-length antibody. In some embodiments, the scFv and the full-length antibody are fused with a linker (e.g., a peptide linker, e.g., a GS linker, e.g., a linker comprising an amino acid sequence selected from the group consisting of SEQ ID NOs: 24, 25, and 44-54). In some embodiments, the V_{H} and V_{L} within the scFv are fused with a linker (e.g., a peptide linker, e.g., a GS linker, e.g., a linker comprising an amino acid sequence selected from the group consisting of SEQ ID NOs: 24, 25, and 44-54). In some embodiments, the construct comprises a) two heavy chains comprising the amino acid sequence set forth in SEQ ID NO: 14, 60, or 62, or a variant comprising an amino acid sequence having at least (such as at least about any one of 80%, 85%, 90%, 95%, 96%, 97%, 98%, or 99%) ; b) two light chains comprising the amino acid sequence set forth in SEQ ID NO: 15, or a variant comprising an amino acid sequence having at least (such as at least about any one of 80%, 85%, 90%, 95%, 96%, 97%, 98%, or 99%) .

In some embodiments, the present application provides a construct comprising a first antigen-binding domain that specifically binds to human HER2 comprising a first heavy chain variable region (V_{H-1}) and a first light chain variable region (V_{L-1}), and a second antigen-binding domain that specifically binds human Trop-2 comprising a second heavy chain variable region (V_{H-2}) and a second light chain variable region (V_{L-2}), wherein the first antigen-binding domain comprises a full-length antibody with a Fc fragment comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a scFv comprising 1) the V_{H-2} comprising a HC-CDR1 comprising the amino acid sequence set forth in SEQ ID NO: 26, a HC-CDR2 comprising the amino acid sequence set forth in SEQ ID NO: 27, and a HC-CDR3 comprising the amino acid sequence set forth in SEQ ID NO: 28, and 2) the V_{L-2} comprising a LC-CDR1 comprising the amino acid sequence set forth in SEQ ID NO: 29, a LC-CDR2 comprising the amino acid sequence set forth in SEQ ID NO: 30, and a LC-CDR3 comprising the amino acid sequence set forth in SEQ ID NO: 31, and the second antigen-binding domain is fused to N-terminus of both heavy chains of the full-length antibody. In some embodiments, the first antigen-binding domain comprises a full-length antibody with a Fc fragment comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a scFv comprising 1) the V_{H-2} comprising the amino acid sequence set forth in SEQ ID NO: 4, and 2) the V_{L-2} comprising the amino acid sequence set forth in SEQ ID NO: 5, and the second antigen-binding domain is fused to N-terminus of both heavy chains of the full-length antibody. In some embodiments, the scFv and the full-length antibody are fused with a linker (e.g., a peptide linker, e.g., a GS linker, e.g., a linker comprising an amino acid sequence selected from the group consisting of SEQ ID NOs: 24, 25, and 44-54). In some embodiments, the V_{H} and V_{L} within the scFv are fused with a linker (e.g., a peptide linker, e.g., a GS linker, e.g., a linker comprising an amino acid sequence selected from the group consisting of SEQ ID NOs: 24, 25, and 44-54). In some embodiments, the construct comprises a) two heavy chains comprising the amino acid sequence set forth in SEQ ID NO: 16, 59, or 61, or a variant comprising an amino acid sequence having at least (such as at least about any one of 80%, 85%, 90%, 95%, 96%, 97%, 98%, or 99%) ; and b) two light chains comprising the amino acid sequence set forth in SEQ ID NO: 17, or a variant comprising an amino acid sequence having at least (such as at least about any one of 80%, 85%, 90%, 95%, 96%, 97%, 98%, or 99%) .

In some embodiments, the present application provides a construct comprising a first antigen-binding domain that specifically binds to human HER2 comprising a first heavy chain variable region (V_{H-1}) and a first light chain variable region (V_{L-1}), and a second antigen-binding domain that specifically binds human Trop-2 comprising a second heavy chain variable region (V_{H-2}) and a second light chain variable region (V_{L-2}), wherein the first antigen-binding domain comprises a full-length antibody with a Fc fragment comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a scFv comprising 1) the V_{H-2} comprising comprises a HC-CDR1 comprising the amino acid sequence set forth in SEQ ID NO: 38, a HC-CDR2 comprising the amino acid sequence set forth in SEQ ID NO: 39, and a HC-CDR3 comprising the amino acid sequence set forth in SEQ ID NO: 40, and 2) the V_{L-2} comprising a LC-CDR1 comprising the amino acid sequence set forth in SEQ ID NO: 41, a LC-CDR2 comprising the amino acid sequence set forth in SEQ ID NO: 42, and a LC-CDR3 comprising the amino acid sequence set forth in SEQ ID NO: 43, and the second antigen-binding domain is fused to C-terminus of both heavy chains of the full-length antibody. In some embodiments, the first antigen-binding domain comprises a full-length antibody with a Fc fragment comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a scFv comprising 1) the V_{H-2} comprising the amino acid sequence set forth in SEQ ID NO: 10 or 58, and 2) the V_{L-2} comprising the amino acid sequence set forth in SEQ ID NO: 11, and the second antigen-binding domain is fused to C-terminus of both heavy chains of the full-length antibody. In some embodiments, the scFv and the full-length antibody are fused with a linker (e.g., a peptide linker, e.g., a GS linker, e.g., a linker comprising an amino acid sequence selected from the group consisting of SEQ ID NOs: 24, 25, and 44-54). In some embodiments, the V_{H} and V_{L} within the scFv are fused with a linker (e.g., a peptide linker, e.g., a GS linker, e.g., a linker comprising an amino acid sequence selected from the group consisting of SEQ ID NOs: 24, 25, and 44-54). In some embodiments, the construct comprises a) two heavy chains comprising the amino acid sequence set forth in SEQ ID NO: 18, or a variant comprising an amino acid sequence having at least (such as at least about any one of 80%, 85%, 90%, 95%, 96%, 97%, 98%, or 99%) ; and b) two light chains comprising the amino acid sequence set forth in SEQ ID NO: 19, or a variant comprising an amino acid sequence having at least (such as at least about any one of 80%, 85%, 90%, 95%, 96%, 97%, 98%, or 99%) .

In some embodiments, the present application provides a construct comprising a first antigen-binding domain that specifically binds to human HER2 comprising a first heavy chain variable region (V_{H-1}) and a first light chain variable region (V_{L-1}), and a second antigen-binding domain that specifically binds human Trop-2 comprising a second heavy chain variable region (V_{H-2}) and a second light chain variable region (V_{L-2}), wherein the first antigen-binding domain comprises a scFv comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a full-length antibody with a Fc fragment comprising 1) the V_{H-2} comprising comprises a HC-CDR1 comprising the amino acid sequence set forth in SEQ ID NO: 38, a HC-CDR2 comprising the amino acid sequence set forth in SEQ ID NO: 39, and a HC-CDR3 comprising the amino acid sequence set forth in SEQ ID NO: 40, and 2) the V_{L-2} comprising a LC-CDR1 comprising the amino acid sequence set forth in SEQ ID NO: 41, a LC-CDR2 comprising the amino acid sequence set forth in SEQ ID NO: 42, and a LC-CDR3 comprising the amino acid sequence set forth in SEQ ID NO: 43, and the first antigen-binding domain is fused to C-terminus of both heavy chains of the full-length antibody. In some embodiments, the first antigen-binding domain comprises a scFv comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a full-length antibody with a Fc fragment comprising 1) the V_{H-2} comprising the amino acid sequence set forth in SEQ ID NO: 10 or 58, and 2) the V_{L-2} comprising the amino acid sequence set forth in SEQ ID NO: 11, and the first antigen-binding domain is fused to C-terminus of both heavy chains of the full-length antibody. In some embodiments, the scFv and the full-length antibody are fused with a linker (e.g., a peptide linker, e.g., a GS linker, e.g., a linker comprising an amino acid sequence selected from the group consisting of SEQ ID NOs: 24, 25, and 44-54). In some embodiments, the V_{H} and V_{L} within the scFv are fused with a linker (e.g., a peptide linker, e.g., a GS linker, e.g., a linker comprising an amino acid sequence selected from the group consisting of SEQ ID NOs: 24, 25, and 44-54). In some embodiments, the construct comprises a) two heavy chains comprising the amino acid sequence set forth in SEQ ID NO: 20, or a variant comprising an amino acid sequence having at least (such as at least about any one of 80%, 85%, 90%, 95%, 96%, 97%, 98%, or 99%) ; and b) two light chains comprising the amino acid sequence set forth in SEQ ID NO: 21, or a variant comprising an amino acid sequence having at least (such as at least about any one of 80%, 85%, 90%, 95%, 96%, 97%, 98%, or 99%) .

In some embodiments, the present application provides a construct comprising a first antigen-binding domain that specifically binds to human HER2 comprising a first heavy chain variable region (V_{H-1}) and a first light chain variable region (V_{L-1}), and a second antigen-binding domain that specifically binds human Trop-2 comprising a second heavy chain variable region (V_{H-2}) and a second light chain variable region (V_{L-2}), wherein the first antigen-binding domain comprises a scFv comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a full-length antibody with a Fc fragment comprising 1) the V_{H-2} comprising comprises a HC-CDR1 comprising the amino acid sequence set forth in SEQ ID NO: 38, a HC-CDR2 comprising the amino acid sequence set forth in SEQ ID NO: 39, and a HC-CDR3 comprising the amino acid sequence set forth in SEQ ID NO: 40, and 2) the V_{L-2} comprising a LC-CDR1 comprising the amino acid sequence set forth in SEQ ID NO: 41, a LC-CDR2 comprising the amino acid sequence set forth in SEQ ID NO: 42, and a LC-CDR3 comprising the amino acid sequence set forth in SEQ ID NO: 43, and the first antigen-binding domain is fused to N-terminus of both heavy chains of the full-length antibody. In some embodiments, the first antigen-binding domain comprises a scFv comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a full-length antibody with a Fc fragment comprising 1) the V_{H-2} comprising the amino acid sequence set forth in SEQ ID NO: 10 or 58, and 2) the V_{L-2} comprising the amino acid sequence set forth in SEQ ID NO: 11, and the first antigen-binding domain is fused to N-terminus of both heavy chains of the full-length antibody. In some embodiments, the scFv and the full-length antibody are fused with a linker (e.g., a peptide linker, e.g., a GS linker, e.g., a linker comprising an amino acid sequence selected from the group consisting of SEQ ID NOs: 24, 25, and 44-54). In some embodiments, the V_{H} and V_{L} within the scFv are fused with a linker (e.g., a peptide linker, e.g., a GS linker, e.g., a linker comprising an amino acid sequence selected from the group consisting of SEQ ID NOs: 24, 25, and 44-54). In some embodiments, the construct comprises a) two heavy chains comprising the amino acid sequence set forth in SEQ ID NO: 22, or a variant comprising an amino acid sequence having at least (such as at least about any one of 80%, 85%, 90%, 95%, 96%, 97%, 98%, or 99%) ; and b) two light chains comprising the amino acid sequence set forth in SEQ ID NO: 23, or a variant comprising an amino acid sequence having at least (such as at least about any one of 80%, 85%, 90%, 95%, 96%, 97%, 98%, or 99%) . In some embodiments, the Fc fragment comprises a heavy chain constant domain which optionally comprises an IgG constant domain (e.g., comprising SEQ ID NO: 12) and a light chain constant domain which optionally comprises a kappa constant region or a lambda constant region (e.g., comprising SEQ ID NO: 13). In some embodiments, the construct is or comprises a bispecific antibody or binding fragment thereof, optionally wherein the bispecific antibody is a humanized antibody. In some embodiments, the construct comprises an antibody-drug conjugate (ADC) having the formula: Ab-(L-D)n, wherein Ab is the bispecific antibody or the binding fragment thereof, L is a linker or a direct bond, D is a therapeutic agent, and n is an integer from 1 to 10.

### Bispecific Anti-HER2/Trop-2 Binding Molecules

In one aspect, the present application provides constructs comprising bispecific anti-HER2/Trop-2 antibodies, or antigen-binding fragment thereof. An antibody of the application, or antigen-binding fragment thereof, can be derivatized or linked to another functional molecule, e.g., another peptide or protein (e.g., another antibody or ligand for a receptor) to generate a bispecific molecule that binds to at least two different binding sites or target molecules. The antibody of the application may in fact be derivatized or linked to more than one other functional molecule to generate multispecific molecules that bind to more than two different binding sites and/or target molecules; such multispecific molecules are also intended to be encompassed by the term "bispecific molecule" as used herein. To create a bispecific molecule of the application, an antibody of the application can be functionally linked (e.g., by chemical coupling, genetic fusion, noncovalent association or otherwise) to one or more other binding molecules, such as another antibody, antibody fragment, peptide or binding mimetic, such that a bispecific molecule is prepared. In various embodiments, the application includes bispecific molecules capable of binding both to FcyR or FcαR expressing effector cells (e.g., monocytes, macrophages or polymorphonuclear cells (PMNs)), and to target cells expressing Trop-2. In such embodiments, the bispecific molecules target Trop-2 expressing cells to effector cell and trigger Fc receptor-mediated effector cell activities, e.g., phagocytosis of a Trop-2 expressing cells, antibody dependent cell-mediated cytotoxicity (ADCC), cytokine release, or generation of superoxide anion. Methods of preparing the bispecific molecules of the present application are well known in the art.

In various embodiments, the bispecific anti-HER2/Trop-2 antibody comprises a first immunoglobulin antigen-binding domain and a second immunoglobulin antigen-binding domain, wherein the first immunoglobulin antigen-binding domain specifically binds to HER2 antibody binding sites, and the second immunoglobulin antigen-binding domain specifically binds to Trop-2 antibody binding site. In various embodiments, the bispecific anti-HER2/Trop-2 antibody comprises a first immunoglobulin antigen-binding domain and a second immunoglobulin antigen-binding domain, wherein the first immunoglobulin antigen-binding domain specifically binds to Trop-2 antibody binding sites, and the second immunoglobulin antigen-binding domain binds to HER2 antibody binding sites.

In various embodiments, the first or second immunoglobulin antigen binding domain of the bispecific anti-HER2/Trop-2 antibody comprises or consists of: (a) a V_{H} and a V_{L}; (b) a V_{H} further comprising a heavy chain constant region or a fragment thereof, and a V_{L} comprising a light chain constant region (LC) or a fragment thereof; (c) a single chain Fv ("scFv"); (d) a diabody; (e) a minibody; (f) an F(ab')2; and (g) an F(ab).

In various embodiments, the first immunoglobulin antigen-binding domain of the bispecific anti-HER2/Trop-2 antibody comprises an scFv antibody fragment, and the second antigen-binding domain comprises a V_{H} and a V_{L}. In various embodiments, the first antigen-binding domain of the bispecific anti-HER2/Trop-2 antibody comprises a V_{H} and a V_{L}, and the second immunoglobulin antigen-binding domain comprises an scFv antibody fragment.

In various embodiments, the first or second immunoglobulin antigen-binding domain of the bispecific anti-HER2/Trop-2 antibody comprises a V_{H} further comprising a heavy chain constant region or fragment thereof and a V_{L} further comprising a light chain constant region or fragment thereof. In various embodiments, the heavy chain constant region or fragment thereof is an IgG constant region. In various embodiments, the IgG constant region or fragment thereof is an IgG1 constant region. In various embodiments, the LC constant region is a kappa constant region. In various embodiments, the light chain constant region is a lambda constant region. In various embodiments, the first or second immunoglobulin antigen-binding domain of the bispecific anti-HER2/Trop-2 antibody comprises a heavy chain which can include an Fc domain comprising a CH2 and a CH3 region. In various embodiments, the Fc domain is an IgG1 Fc domain. In various embodiments, the IgG1 Fc domain is a native IgG1 Fc domain.

In various embodiments, the first or second immunoglobulin antigen-binding domain is a monoclonal antibody. In various embodiments, the first or second immunoglobulin antigen-binding domain is a humanized antibody. In various embodiments, the first or second immunoglobulin antigen-binding domain is a human antibody. In various embodiments, the first or second immunoglobulin antigen-binding domain is a chimeric antibody. In various embodiments, the first or second immunoglobulin antigen-binding domain is an affinity optimized antibody. In various embodiments, the human antibody is expressed in a transgenic mouse (see, for example, Bruggemann, "Human antibody expression in transgenic mice, " Arch. Immunol. Therap. Exper. 49: 203-208, 2001).

In various embodiments, the second immunoglobulin antigen-binding domain of the bispecific anti-HER2/Trop-2 antibody is covalently linked to the carboxy-terminus of the heavy chain of the first immunoglobulin antigen-binding domain. In various embodiments, the bispecific anti- HER2/Trop-2 antibody comprises a linker interposed between the second immunoglobulin antigen binding domain and the carboxy-terminus of the heavy chain of the first immunoglobulin antigen-binding domain. In various embodiments, the peptide linker II comprises the amino acid sequence of SEQ ID NO: 25. In various embodiments, the second immunoglobulin antigen-binding domain of the bispecific anti-HER2/Trop-2 antibody is covalently linked to the amino-terminus of the heavy chain of the first immunoglobulin antigen-binding domain. In various embodiments, the bispecific anti-HER2/Trop-2 antibody comprises a linker interposed between the second immunoglobulin antigen-binding domain and the amino-terminus of the heavy chain of the first immunoglobulin antigen-binding domain. In various embodiments, the peptide linker II comprises the amino acid sequence of SEQ ID NO: 25.

The scFv disclosed herein are obtainable from or produced by any suitable source, whether natural or not, or it may be a recombinant scFv, a synthetic scFv, a semi synthetic scFv, a derivatized scFv, a fermentation optimized scFv, a fusion protein or equivalents, mutants and derivatives thereof as long as it retains the required binding specificity of the scFv's of the present disclosure. These include a scFv with binding specificity which has amino acid substitutions or has sugars or other molecules attached to amino acid functional groups. The term "derivative" or "derivatized" as used herein with respect to an scFv includes chemical modification of an scFv. Illustrative of such modifications would be replacement of hydrogen by an alkyl, acyl, or amino group.

### Linkers

**In** some embodiments, the constructs described herein comprise one or more linkers between two moieties or domains (*e.g.,* between a scFv specifically binding to Trop2 and a full-length antibody of Herceptin, e.g., between a Herceptin scFv and a full-length antibody that binds to Trop2, e.g., between the V_{H} or V_{L} in an scFv). The length, the degree of flexibility and/or other properties of the linker(s) used in the constructs may have some influence on properties, including but not limited to the affinity, specificity or avidity for one or more particular antigens or epitopes. For example, longer linkers may be selected to ensure that two adjacent domains do not sterically interfere with one another. In some embodiment, a linker (such as peptide linker) comprises flexible residues (such as glycine and serine) so that the adjacent domains are free to move relative to each other. For example, a glycine-serine doublet can be a suitable peptide linker. In some embodiments, the linker is a non-peptide linker. In some embodiments, the linker is a peptide linker. In some embodiments, the linker is a non-cleavable linker. In some embodiments, the linker is a cleavable linker.

Other linker considerations include the effect on physical or pharmacokinetic properties of the resulting compound, such as solubility, lipophilicity, hydrophilicity, hydrophobicity, stability (more or less stable as well as planned degradation), rigidity, flexibility, immunogenicity, modulation of antibody binding, the ability to be incorporated into a micelle or liposome, and the like.

### Peptide linkers

The peptide linker may have a naturally occurring sequence, or a non-naturally occurring sequence. For example, a sequence derived from the hinge region of heavy chain only antibodies may be used as the linker. *See,* for example, WO1996/34103.

The peptide linker can be of any suitable length. In some embodiments, the peptide linker is at least about any of 1, 2, 3, 4, 5, 6, 7, 8, 9, 10, 11, 12, 13, 14, 15, 16, 17, 18, 19, 20, 25, 30, 35, 40, 50, 75, 100 or more amino acids long. In some embodiments, the peptide linker is no more than about any of 100, 75, 50, 40, 35, 30, 25, 20, 19, 18, 17, 16, 15, 14, 13, 12, 11, 10, 9, 8, 7, 6, 5 or fewer amino acids long. In some embodiments, the length of the peptide linker is any of about 1 amino acid to about 10 amino acids, about 1 amino acid to about 20 amino acids, about 1 amino acid to about 30 amino acids, about 5 amino acids to about 15 amino acids, about 10 amino acids to about 25 amino acids, about 5 amino acids to about 30 amino acids, about 10 amino acids to about 30 amino acids long, about 30 amino acids to about 50 amino acids, about 50 amino acids to about 100 amino acids, or about 1 amino acid to about 100 amino acids.

An essential technical feature of such peptide linker is that said peptide linker does not comprise any polymerization activity. The characteristics of a peptide linker, which comprise the absence of the promotion of secondary structures, are known in the art and described, *e.g.,* in Dall'Acqua et al. (Biochem. (1998) 37, 9266-9273), Cheadle et al. (Mol Immunol (1992) 29, 21-30) and Raag and Whitlow (FASEB (1995) 9(1), 73-80). A particularly preferred amino acid in context of the "peptide linker" is Gly. Furthermore, peptide linkers that also do not promote any secondary structures are preferred. The linkage of the domains to each other can be provided by, *e.g.,* genetic engineering. Methods for preparing fused and operatively linked bispecific single chain constructs and expressing them in mammalian cells or bacteria are well-known in the art (*e.g.* WO 99/54440, Ausubel, Current Protocols in Molecular Biology, Green Publishing Associates and Wiley Interscience, N. Y. 1989 and 1994 or Sambrook et al., Molecular Cloning: A Laboratory Manual, Cold Spring Harbor Laboratory Press, Cold Spring Harbor, N. Y., 2001).

The peptide linker can be a stable linker, which is not cleavable by proteases, especially by Matrix metalloproteinases (MMPs).

The linker can also be a flexible linker. Exemplary flexible linkers include glycine polymers (G)ₙ (SEQ ID NO: 48), glycine-serine polymers (including, for example, (GS)ₙ (SEQ ID NO: 49), (GSGGS)ₙ (SEQ ID NO: 50), (GGGGS)ₙ (SEQ ID NO: 51), and (GGGS)ₙ (SEQ ID NO: 52), where n is an integer of at least one), glycine-alanine polymers, alanine-serine polymers, and other flexible linkers known in the art. Glycine and glycine-serine polymers are relatively unstructured, and therefore may be able to serve as a neutral tether between components. Glycine accesses significantly more phi-psi space than even alanine, and is much less restricted than residues with longer side chains (*See* Scheraga, Rev. Computational Chem. 11 173-142 (1992)). The ordinarily skilled artisan will recognize that design of an antibody fusion protein can include linkers that are all or partially flexible, such that the linker can include a flexible linker portion as well as one or more portions that confer less flexible structure to provide a desired antibody fusion protein structure.

Furthermore, exemplary linkers also include the amino acid sequence of such as (GGGGS)ₙ(SEQ ID NO: 51), wherein n is an integer between 1 and 8, *e.g.* (GGGGS)₃ (SEQ ID NO: 46), (GGGGS)₄ (SEQ ID NO: 47), or (GGGGS)₆ (SEQ ID NO: 53). In some embodiments, the peptide linker comprises the amino acid sequence of (GSTSGSGKPGSGEGS)ₙ (SEQ ID NO: 54), wherein n is an integer between 1 and 3.

### Non-peptide linkers

Coupling of two moieties may be accomplished by any chemical reaction that will bind the two molecules so long as both components retain their respective activities, e.g., binding to HER2 and Trop-2 in a construct described herein, respectively. This linkage can include many chemical mechanisms, for instance covalent binding, affinity binding, intercalation, coordinate binding and complexation. In some embodiments, the binding is covalent binding. Covalent binding can be achieved either by direct condensation of existing side chains or by the incorporation of external bridging molecules. Many bivalent or polyvalent linking agents may be useful in coupling protein molecules in this context. For example, representative coupling agents can include organic compounds such as thioesters, carbodiimides, succinimide esters, diisocyanates, glutaraldehyde, diazobenzenes and hexamethylene diamines. This listing is not intended to be exhaustive of the various classes of coupling agents known in the art but, rather, is exemplary of the more common coupling agents (*See* Killen and Lindstrom, Jour. Immun. 133:1335-2549 (1984); Jansen et al., Immunological Reviews 62:185-216 (1982); and Vitetta et al., Science 238:1098 (1987)).

Linkers that can be applied in the present application are described in the literature (*see, for example,* Ramakrishnan, S. et al., Cancer Res. 44:201-208 (1984) describing use of MBS (M-maleimidobenzoyl-N-hydroxysuccinimide ester). In some embodiments, non-peptide linkers used herein include: (i) EDC (1-ethyl-3-(3-dimethylamino-propyl) carbodiimide hydrochloride; (ii) SMPT (4-succinimidyloxycarbonyl-alpha-methyl-alpha-(2-pridyl-dithio)-toluene (Pierce Chem. Co., Cat. (21558G); (iii) SPDP (succinimidyl-6 [3-(2-pyridyldithio) propionamido] hexanoate (Pierce Chem. Co., Cat #21651G); (iv) Sulfo-LC-SPDP (sulfosuccinimidyl 6 [3-(2-pyridyldithio)-propianamide] hexanoate (Pierce Chem. Co. Cat. #2165-G); and (v) sulfo-NHS (N-hydroxysulfo-succinimide: Pierce Chem. Co., Cat. #24510) conjugated to EDC.

The linkers described above contain components that have different attributes, thus may lead to bispecific antibodies with differing physio-chemical properties. For example, sulfo-NHS esters of alkyl carboxylates are more stable than sulfo-NHS esters of aromatic carboxylates. NHS-ester containing linkers are less soluble than sulfo-NHS esters. Further, the linker SMPT contains a sterically hindered disulfide bond, and can form antibody fusion protein with increased stability. Disulfide linkages, are in general, less stable than other linkages because the disulfide linkage is cleaved *in vitro,* resulting in less antibody fusion protein available. Sulfo-NHS, in particular, can enhance the stability of carbodimide couplings. Carbodimide couplings (such as EDC) when used in conjunction with sulfo-NHS, forms esters that are more resistant to hydrolysis than the carbodimide coupling reaction alone.

### Exemplary Embodiments

In various embodiments of the present application, the isolated bispecific anti-HER2/Trop-2 antibody comprises a first immunoglobulin antigen-binding domain that specifically binds to HER2 and a second immunoglobulin antigen-binding domain that specifically binds to Trop-2, wherein the first immunoglobulin antigen-binding domain comprises a scFv comprising the amino acid sequence set forth in SEQ ID NO: 3: 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKGGGGSGGG
GSGGGGSEVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRWGGDGFY
AMDYWGQGTLVTVSS (SEQ ID NO: 3)
 and wherein the second immunoglobulin antigen-binding domain comprises a V_{H} comprising the amino acid sequence set forth in SEQ ID NO: 4: 
QVQLQESGPGLVKPSETLSLTCTVSGFSLTSYGVNWIRQPPGKGLEWIGVMWAGGST
NYNSALMSRLTISKDTSKNQFSLKLSSVTAADTAVYYCARDENWDGAWFAYWGQG
TLVTVSS (SEQ ID NO: 4)
 and a V_{L} comprising the amino acid sequence set forth in SEQ ID NO: 5:

DIVMTQSPDSLAVSLGERATINCKSSQSLLNSGTRKNYLAWYQQKPGQPPKLLISWA
SSRESGVPDRFSGSGSGTDFTLTISSLQAEDVAVYYCKQSYNLFTFGGGTKVEIK
(SEQ ID NO: 5)

In various embodiments of the present application, the isolated bispecific anti-HER2/Trop-2 antibody comprises a first immunoglobulin antigen-binding domain that specifically binds to HER2 and a second immunoglobulin antigen-binding domain that specifically binds to Trop-2, wherein the first immunoglobulin antigen-binding domain comprises a scFv comprising the amino acid sequence set forth in SEQ ID NO: 3; and wherein the second immunoglobulin antigen-binding domain comprises a V_{H} comprising the amino acid sequence set forth in SEQ ID NO: 10: 
QVQLQQSGSELKKPGASVKVSCKASGYTFTNYGMNWVKQAPGQGLKWMGWINTY
TGEPTYTDDFKGRFAFSLDTSVSTAYLQISSLKADDTAVYFCARGGFGSSYWYFDV
WGQGSLVTVSS (SEQ ID NO: 10)
 and a V_{L} comprising the amino acid sequence set forth in SEQ ID NO: 11:

DIQLTQSPSSLSASVGDRVSITCKASQDVSIAVAWYQQKPGKAPKLLIYSASYRYTGV
PDRFSGSGSGTDFTLTISSLQPEDFAVYYCQQHYITPLTFGAGTKVDIK (SEQ ID NO:
11)

In various embodiments of the present application, the isolated bispecific anti-HER2/Trop-2 antibody comprises a first immunoglobulin antigen-binding domain that specifically binds to Trop-2 and a second immunoglobulin antigen-binding domain that specifically binds to HER2, wherein the first immunoglobulin antigen-binding domain comprises a scFv comprising the amino acid sequence set forth in SEQ ID NO: 6: 
DIVMTQSPDSLAVSLGERATINCKSSQSLLNSGTRKNYLAWYQQKPGQPPKLLISWA
SSRESGVPDRFSGSGSGTDFTLTISSLQAEDVAVYYCKQSYNLFTFGGGTKVEIKGGG
GSGGGGSGGGGSQVQLQESGPGLVKPSETLSLTCTVSGFSLTSYGVNWIRQPPGKGL
EWIGVMWAGGSTNYNSALMSRLTISKDTSKNQFSLKLSSVTAADTAVYYCARDEN
WDGAWFAYWGQGTLVTVSS (SEQ ID NO: 6)
 and wherein the second immunoglobulin antigen-binding domain comprises a V_{H} comprising the amino acid sequence set forth in SEQ ID NO: 7: 


 (SEQ ID NO: 7)
 and a V_{L} comprising the amino acid sequence set forth in SEQ ID NO: 8:


 (SEQ ID NO:
8)

In various embodiments of the present application, the isolated bispecific anti-HER2/Trop-2 antibody comprises a first immunoglobulin antigen-binding domain that specifically binds to Trop-2 and a second immunoglobulin antigen-binding domain that specifically binds to HER2, wherein the first immunoglobulin antigen-binding domain comprises a scFv comprising the amino acid sequence set forth in SEQ ID NO: 9: 
DIQLTQSPSSLSASVGDRVSITCKASQDVSIAVAWYQQKPGKAPKLLIYSASYRYTGV
PDRFSGSGSGTDFTLTISSLQPEDFAVYYCQQHYITPLTFGAGTKVDIKGGGGSGGGG
SGGGGSQVQLQQSGSELKKPGASVKVSCKASGYTFTNYGMNWVKQAPGQGLKWM
GWINTYTGEPTYTDDFKGRFAFSLDTSVSTAYLQISSLKADDTAVYFCARGGFGSSY
WYFDVWGQGSLVTVSS (SEQ ID NO: 9)
 and wherein the second immunoglobulin antigen-binding domain comprises a V_{H} comprising the amino acid sequence set forth in SEQ ID NO: 7 and a V_{L} comprising the amino acid sequence set forth in SEQ ID NO: 8.

In various embodiments of the present application, the isolated bispecific anti-HER2/Trop-2 antibody comprises a first immunoglobulin antigen-binding domain that specifically binds to HER2 and a second immunoglobulin antigen-binding domain that specifically binds to Trop-2, wherein the first immunoglobulin antigen-binding domain comprises a V_{H} comprising the amino acid sequence of SEQ ID NO: 7 and a V_{L} comprising the amino acid sequence set forth in SEQ ID NO: 8; and wherein the second icomprises a scFv comprising the amino acid sequence set forth in SEQ ID NO: 9.

In various embodiments of the present application, the isolated bispecific anti-HER2/Trop-2 antibody comprises a first immunoglobulin antigen-binding domain that specifically binds to HER2 and a second immunoglobulin antigen-binding domain that specifically binds to Trop-2, wherein the first immunoglobulin antigen-binding domain comprises a scFv comprising the amino acid sequence set forth in SEQ ID NO: 3; and wherein the second immunoglobulin antigen-binding domain comprises a heavy chain (HC) comprising the amino acid sequence set forth in SEQ ID NO: 14: 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKGGGGSGGG
GSGGGGSEVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRWGGDGFY
AMDYWGQGTLVTVSSGGGGSGGGGSGGGSQVQLQESGPGLVKPSETLSLTCTVSGF
SLTSYGVNWIRQPPGKGLEWIGVMWAGGSTNYNSALMSRLTISKDTSKNQFSLKLSS
VTAADTAVYYCARDENWDGAWFAYWGQGTLVTVSSASTKGPSVFPLAPSSKSTSG
GTAALGCLVKDYFPEPVTVSWNSGALTSGVHTFPAVLQSSGLYSLSSVVTVPSSSLGT
QTYICNVNHKPSNTKVDKRVEPKSCDKTHTCPPCPAPELLGGPSVFLFPPKPKDTLMI
SRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREEMTKNQVSLT
CLVKGFYPSDIAVEWESNGQPENNYKTTPPVLDSDGSFFLYSKLTVDKSRWQQGNVF
SCSVMHEALHNHYTQKSLSLSPGK (SEQ ID NO: 14)
 and a light chain (LC) comprising the amino acid sequence set forth in SEQ ID NO: 15:

DIVMTQSPDSLAVSLGERATINCKSSQSLLNSGTRKNYLAWYQQKPGQPPKLLISWA
SSRESGVPDRFSGSGSGTDFTLTISSLQAEDVAVYYCKQSYNLFTFGGGTKVEIKRTV
AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO:
15)

In various embodiments of the present application, the isolated bispecific anti-HER2/Trop-2 antibody comprises a first immunoglobulin antigen-binding domain that specifically binds to HER2 and a second immunoglobulin antigen-binding domain that specifically binds to Trop-2, wherein the first immunoglobulin antigen-binding domain comprises a scFv comprising the amino acid sequence set forth in SEQ ID NO: 3; and wherein the second immunoglobulin antigen-binding domain comprises a heavy chain (HC) comprising the amino acid sequence set forth in SEQ ID NO: 22: 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKGGGGSGGG
GSGGGGSEVQLVESGGGLVQPGGSLRLSCMSGFNIKDTYIHWVRQAPGKGLEWVAR
IYPTNGYTRYADSVKGRFTISADTSKNTAYLQMNSLRAEDTAVYYCSRWGGDGFYA
MDYWGQGTLVTVSSGGGGSGGGGSGGGSQVQLQQSGSELKKPGASVKVSCKASGY
TFTNYGMNWVKQAPGQGLWMGWINTYTGEPTYTDDFKGRFAFSLDTSVSTAYLQI
SSLKADDTAVYFCARGGFGSSYWYFDVWGQGSLVTVSSASTKGPSVFPLAPSSKSTS
GGTAALGCLVKDYFPEPVTVSWNSGALTSGVHTFPAVLQSSGLYSLSSVVTVPSSSLG
TQTYICWNHKPSNTKVDKRVEPKSCDKTHTCPPCPAPELLGGPSVFLFPPKPKDTLMI
SRTPEVTCVVVDVSHEDPEVKFNWYVDGVEVHNAKTKPREEQYNSTYRVVSVLTVL
HQDWLNGKEYKCKVSNKALPAPTEKTTSKAKGQPREPQVYTLPPSREEMTKNQVSL
TCI.VKGFYPSDIAVEWESNGQPENNYKTTPPVLDSDGSFFLYSKLTVDKSRWQQGNV
FSCSVMHEALRNHYTQKSLSLSPG (SEQ ID NO: 22)
 and a light chain (LC) comprising the amino acid sequence set forth in SEQ ID NO: 23:

DIQLTQSPSSLSASVGDRVSITCKASQDVSIAVAWYQQKPGKAPKLLIYSASYRYTGV
PDRFSGSGSGTDFTLTISSLQPEDFAVYYCQQHYITPLTFGAGTKVDIKRTVAAPSVFI
FPPSDEQLKSGTASVVCLLNNFYPREAKVQWKVDNALQSGNSQESVTEQDSKDSTY
SLSSTLTLSKADYEKHKVYACÉVTIIQGLSSPVTKSFNRGEC (SEQ ID NO: 23)

In various embodiments of the present application, the isolated bispecific anti-HER2/Trop-2 antibody comprises a first immunoglobulin antigen-binding domain that specifically binds to Trop-2 and a second immunoglobulin antigen-binding domain that specifically binds to HER2, wherein the first immunoglobulin antigen-binding domain comprises a scFv comprising the amino acid sequence set forth in SEQ ID NO: 6; and wherein the second immunoglobulin antigen-binding domain comprises a heavy chain (HC) comprising the amino acid sequence set forth in SEQ ID NO: 16: 
DIVMTQSPDSLAVSLGERATINCKSSQSLLNSGTRKNYLAWYQQKPGQPPKLLISWA
SSRESGVPDRFSGSGSGTDFTLTISSLQAEDVAVYYCKQSYNLFTFGGGTKVEIKGGG
GSGGGGSGGGGSQVQLQESGPGLVKPSETLSLTCTVSGFSLTSYGVNWIRQPPGKGL
EWIGVMWAGGSTNYNSALMSRLTISKDTSKNQFSLKLSSVTAADTAVYYCARDEN
WDGAWFAYWGQGTLVTVSSGGGGSGGGGSGGGSEVQLVESGGGLVQPGGSLRLS
CAASGFNIKDTYIHWVRQAPGKGLEWVARIYPTNGYTRYADSVKGRFTISADTSKNT
AYLQMNSLRAEDTAVYYCSRWGGDGFYAMDYWGQGTLVTVSSASTKGPSVFPLAP
SSKSTSGGTAALGCLVKDYFPEPVTVSWNSGALTSGVHTFPAVLQSSGLYSLSSVVTV
PSSSLGTQTYICNVNHKPSNTKVDKRVEPKSCDKTHTCPPCPAPELLGGPSVFLFPPKP
KDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTK
 
NQVSLTCLVKGFYPSDIAVEWESNGQPENNYKTTPPVLDSDGSFFLYSKLTVDKSRW
QQGNVFSCSVMHEALHNHYTQKSLSLSPGK
(SEQ ID NO: 16)
 and a light chain (LC) comprising the amino acid sequence set forth in SEQ ID NO: 17:

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSVF
IFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO: 17)

In various embodiments of the present application, the isolated bispecific anti-HER2/Trop-2 antibody comprises a first immunoglobulin antigen-binding domain that specifically binds to Trop-2 and a second immunoglobulin antigen-binding domain that specifically binds to HER2, wherein the first immunoglobulin antigen-binding domain comprises a scFv comprising the amino acid sequence set forth in SEQ ID NO: 9; and wherein the second immunoglobulin antigen-binding domain comprises a heavy chain (HC) comprising the amino acid sequence set forth in SEQ ID NO: 20: 
DIQLTQSPSSLSASVGDRVSITCKASQDVSIAVAWYQQKPGKAPKLLIYSASYRYT
GVPDRFSGSGSGTDFTLTISSLQPEDFAVYYCQQHYITPLTFGAGTKVDIKGGGGSGG
GGSGGGGSQVQLQQSGSELKKPGASVKVSCKASGYTFTNYGMNWVKQAPGQGLK
WMGWINTYTGEPTYTDDFKGRFAFSLDTSVSTAYLQISSLKADDTAVYFCARGGFG
SSYWYFDVWGQGSLVTVSSEVQLVESGGGLVQPGGSLRLSCAASGFNIKDTYIHWV
RQAPGKGLEWVARIYPTNGYTRYADSVKGRFTISADTSKNTAYLQMNSLRAEDTAV
YYCSRWGGDGFYAMDYWGQGTLVTVSSASTKGPSVFPLAPSSKSTSGGTAALGCLV
KDYFPEPVTVSWNSGALTSGVHTFPAVLQSSGLYSLSSVVTVPSSSLGTQTYICNVNH
KPSNTKVDKRVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGK
EYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEA
LHNHYTQKSLSLSPG (SEQ ID NO: 20)
 and a light chain (LC) comprising the amino acid sequence set forth in SEQ ID NO: 21:

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSVF
IFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO: 21)

In various embodiments of the present application, the isolated bispecific anti-HER2/Trop-2 antibody comprises a first immunoglobulin antigen-binding domain that specifically binds to HER2 and a second immunoglobulin antigen-binding domain that specifically binds to Trop-2, and wherein the first immunoglobulin antigen-binding domain comprises a heavy chain (HC) comprising the amino acid sequence set forth in SEQ ID NO: 18: 
EVQLVESGGGLVQPGGSLRLSCAASGFNIKDTYIHWVRQAPGKGLEWVARIYPTNG
YTRYADSVKGRFTISADTSKNTAYLQMNSLRAEDTAVYYCSRWGGDGFYAMDYW
GQGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKRVEPKSCDK
THTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGGGG
GSGGGGSDIQLTQSPSSLSASVGDRVSITCKASQDVSIAVAWYQQKPGKAPKLLIYSA
SYRYTGVPDRFSGSGSGTDFTLTISSLQPEDFAVYYCQQHYITPLTFGAGTKVDIKGG
GGSGGGGSGGGGSQVQLQQSGSELKKPGASVKVSCKASGYTFTNYGMNWVKQAP
GQGLKWMGWINTYTGEPTYTDDFKGRFAFSLDTSVSTAYLQISSLKADDTAVYFCA
RGGFGSSYWYFDVWGQGSLVTVSS (SEQ ID NO: 18)
 and a light chain (LC) comprising the amino acid sequence set forth in SEQ ID NO: 19: 
DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSVF
IFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO: 19)
 wherein the second immunoglobulin antigen-binding domain comprises a scFv comprising the amino acid sequence set forth in SEQ ID NO: 9.

**In** various embodiments, the bispecific antibody is an antibody comprising a scFv domain, a heavy chain and a light chain, wherein the scFv domain comprises a sequence that has at least about 80%, at least about 85%, at least about 90%, 91%, 92%, 93%, 94%, 95%, 96%, 97%, 98%, or at least about 99% identity to the amino acid sequence as set forth in SEQ ID NO: 3, 6, 9, the heavy chain comprises a sequence that has at least about 80%, at least about 85%, at least about 90%, 91%, 92%, 93%, 94%, 95%, 96%, 97%, 98%, or at least about 99% identity to the amino acid sequence as set forth in SEQ ID NO: 14, 16, 18, 20, 22, and wherein the light chain comprises a sequence that has at least about 75%, at least about 80%, at least about 85%, at least about 90%, 91%, 92%, 93%, 94%, 95%, 96%, 97%, 98%, or at least about 99% identity to the amino acid sequence as set forth in SEQ ID NO: 15, 17,19,21, 23.

In various embodiments, the second immunoglobulin antigen-binding domain of the bispecific anti-HER2/Trop-2 antibody is covalently linked to the carboxy-terminus of the HC of the first immunoglobulin antigen-binding domain. In various embodiments, the bispecific anti-HER2/Trop-2 antibody comprises at least one linker interposed between the second immunoglobulin antigen binding domain and the carboxy-terminus of the HC of the first immunoglobulin antigen-binding domain. In various embodiments, one linker is interposed between the second immunoglobulin antigen binding domain and the carboxy-terminus of the HC of the first immunoglobulin antigen-binding domain.

In various embodiments, the second immunoglobulin antigen-binding domain is covalently linked to the amino-terminus of the HC of the first immunoglobulin antigen-binding domain. In various embodiments, the bispecific anti-HER2/Trop-2 antibody comprises at least one linker interposed between the second immunoglobulin antigen-binding domain and the amino-terminus of the HC of the first immunoglobulin antigen-binding domain. In various embodiments, one linker is interposed between the second immunoglobulin antigen-binding domain and the amino-terminus of the HC of the first immunoglobulin antigenbinding domain.

In various embodiments of the present disclosure the bispecific antibody may be an anti-HER2/Trop-2 antibody that has the same or higher antigen-binding affinity as that of the antibody comprising the heavy chain sequence as set forth in any of SEQ ID NOs: 14, 16, 18, 20, and 22. In various embodiments, the antibody may be an anti-HER2/Trop-2 antibody which binds to the same epitopes as the antibody comprising the heavy chain sequence as set forth in any of SEQ ID NOs: 14, 16, 18, 20, and 22. In various embodiments, the antibody is an anti-HER2/Trop-2 antibody which competes with the antibody comprising the heavy chain sequence as set forth in any of SEQ ID NOs: 14, 16, 18, 20, and 22. In various embodiments, the antibody may be an anti-HER2/Trop-2 antibody which comprises at least one (such as two or three) CDRs of the light chain sequence as set forth in any of SEQ ID NOs: 14, 16, 18, 20, and 22.

In various embodiments of the present disclosure the bispecific antibody may be an anti-HER2/Trop-2 antibody that has the same or higher antigen-binding affinity as that of the antibody comprising the light chain sequence as set forth in any of SEQ ID NOs: 15, 17, 19, 21, and 23. In various embodiments, the antibody may be an anti-HER2/Trop-2 antibody which binds to the same epitope as the antibody comprising the light chain sequence as set forth in any of SEQ ID NOs: 15, 17, 19, 21, and 23. In various embodiments, the antibody is an anti-HER2/Trop-2 antibody which competes with the antibody comprising the light chain sequence as set forth in any of SEQ ID NOs: 15, 17, 19, 21, and 23. In various embodiments, the antibody may be an anti-HER2/Trop-2 antibody which comprises at least one (such as two or three) CDRs of the light chain sequence as set forth in any of SEQ ID NOs: 15, 17, 19, 21, and 23.

The bispecific antibodies or antigen-binding fragments thereof of the application can comprise any constant region known in the art. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, e.g., a IgA-, IgD-, IgE-, IgG- and IgM-type heavy chain constant region. In various embodiments, the light or heavy chain constant region is a fragment, derivative, variant, or mutein of a naturally occurring constant region.

Techniques are known for deriving an antibody of a different subclass or isotype from an antibody of interest, i.e., subclass switching. Thus, IgG antibodies may be derived from an IgM antibody, for example, and vice versa. Such techniques allow the preparation of new antibodies that possess the antigen-binding properties of a given antibody (the parent antibody), but also exhibit biological properties associated with an antibody isotype or subclass different from that of the parent antibody. Recombinant DNA techniques may be employed. Cloned DNA encoding particular antibody polypeptides may be employed in such procedures, e.g., DNA encoding the constant domain of an antibody of the desired isotype. See also Lanitto et al., Methods Mol. Biol. 178:303-16, 2002.

Antibodies of the present application may also be described or specified in terms of their cross-reactivity. Antibodies that bind HER2 and Trop-2, which have at least 95%, at least 90%, at least 85%, at least 80%, at least 75%, at least 70%, at least 65%, at least 60%, at least 55%, and at least 50% identity (as calculated using methods known in the art and described herein) to human HER2 and Trop-2are also included in the present application.

In various embodiments, the antibodies of the present application can be engineered by modifying one or more residues within one or both variable regions (i.e., V_{H} and/or V_{L}), or by modifying residues within the constant region(s), e.g., to alter the effector function(s) of the antibody. In various embodiments, the variable region of the antibody will by modified by performing CDR grafting using framework sequences can be obtained from public DNA databases or published references that include germline antibody gene sequences (e.g., Tomlinson, I. M., et al., J. Mol. Biol. 227:776-798, 1992; and Cox, J. P. L. et al., Eur. J. Immunol. 24:827-836, 1994). In various embodiments, the antibodies may be modified using site-directed mutagenesis or PCR-mediated mutagenesis to introduce a mutation(s) in the V_{H} and/or V_{L} which improves binding affinity and/or decreases immunogenicity. In various embodiments, the antibodies may be modified in the Fc region for purposes of altering the serum half-life, complement fixation, Fc receptor binding, and/or antigen-dependent cellular cytotoxicity of the antibody. In various embodiments, the antibodies may be modified for purposes of modifying the glycosylation of the antibody. Methods for performing each of the modifications described herein, and others, are wll known to the skilled artisan.

### Characterization of Bispecific Antibody Binding to Antigen

Bispecific antibodies of the present application can be tested for binding to human HER2 and Trop-2 expressed on cell surface by, for example, flow cytometry. Briefly, antibody can be added to cells in FACS buffer and incubated for 30 min at 4°C. After incubation, the cells are washed to remove unbound antibody. The cells are then dissociated and stained with fluorescent-conjugated secondary antibody for 30 minutes on ice before being analyzed using backman flow cytometry system. The flueorescent intensity and cell binding percentage can be analyzed by Beckman flow cytometric software.

### Pharmaceutical Compositions

In another aspect, the present application provides a pharmaceutical composition comprising an antibody or antigen-binding fragment thereof as described above. The pharmaceutical compositions, methods and uses of the application thus also encompass embodiments of combinations (co-administration) with other active agents, as detailed below.

Generally, the antibodies, or antigen-binding fragments thereof antibodies of the present application are suitable to be administered as a formulation in association with one or more pharmaceutically acceptable excipient(s). The term 'excipient' is used herein to describe any ingredient other than the compound(s) of the application. The choice of excipient(s) will to a large extent depend on factors such as the particular mode of administration, the effect of the excipient on solubility and stability, and the nature of the dosage form. As used herein, "pharmaceutically acceptable excipient" includes any and all solvents, dispersion media, coatings, antibacterial and antifungal agents, isotonic and absorption delaying agents, and the like that are physiologically compatible. Some examples of pharmaceutically acceptable excipients are water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Additional examples of pharmaceutically acceptable substances are wetting agents or minor amounts of auxiliary substances such as wetting or emulsifying agents, preservatives or buffers, which enhance the shelf life or effectiveness of the antibody. Pharmaceutical compositions of the present application and methods for their preparation will be readily apparent to those skilled in the art. Such compositions and methods for their preparation may be found, for example, in Remington's Pharmaceutical Sciences, 19th Edition (Mack Publishing Company, 1995). Pharmaceutical compositions are preferably manufactured under GMP conditions.

A pharmaceutical composition of the application may be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

Any method for administering peptides, proteins or antibodies accepted in the art may suitably be employed for the antibodies and portions of the application.

The pharmaceutical compositions of the application are typically suitable for parenteral administration. As used herein, "parenteral administration" of a pharmaceutical composition includes any route of administration characterized by physical breaching of a tissue of a subject and administration of the pharmaceutical composition through the breach in the tissue, thus generally resulting in the direct administration into the blood stream, into muscle, or into an internal organ. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intrasternal, intravenous, intraarterial, intrathecal, intraventricular, intraurethral, intracranial, intrasynovial injection or infusions, and kidney dialytic infusion techniques. Various embodiments include the intravenous and the subcutaneous routes.

Formulations of a pharmaceutical composition suitable for parenteral administration typically generally comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules or in multi-dose containers containing a preservative. Formulations for parenteral administration include, but are not limited to, suspensions, solutions, emulsions in oily or aqueous vehicles, pastes, and the like. Such formulations may further comprise one or more additional ingredients including, but not limited to, suspending, stabilizing, or dispersing agents. In one embodiment of a formulation for parenteral administration, the active ingredient is provided in dry (i.e. powder or granular) form for reconstitution with a suitable vehicle (e.g. sterile pyrogen-free water) prior to parenteral administration of the reconstituted composition. Parenteral formulations also include aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water. Exemplary parenteral administration forms include solutions or suspensions in sterile aqueous solutions, for example, aqueous propylene glycol or dextrose solutions. Such dosage forms can be suitably buffered, if desired. Other parentally-administrable formulations which are useful include those which comprise the active ingredient in microcrystalline form, or in a liposomal preparation. Formulations for parenteral administration may be formulated to be immediate and/or modified release. Modified release formulations include delayed-, sustained-, pulsed-, controlled-, targeted and programmed release.

For example, in one aspect, sterile injectable solutions can be prepared by incorporating the bispecific antibody in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The antibodies of the application can also be administered intranasally or by inhalation, typically in the form of a dry powder (either alone, as a mixture, or as a mixed component particle, for example, mixed with a suitable pharmaceutically acceptable excipient) from a dry powder inhaler, as an aerosol spray from a pressurized container, pump, spray, atomizer (preferably an atomizer using electrohydrodynamics to produce a fine mist), or nebulizer, with or without the use of a suitable propellant, or as nasal drops.

The pressurized container, pump, spray, atomizer, or nebulizer generally contains a solution or suspension of an antibody of the application comprising, for example, a suitable agent for dispersing, solubilizing, or extending release of the active, a propellant(s) as solvent.

Prior to use in a dry powder or suspension formulation, the drug product is generally micronized to a size suitable for delivery by inhalation (typically less than 5 microns). This may be achieved by any appropriate comminuting method, such as spiral jet milling, fluid bed jet milling, supercritical fluid processing to form nanoparticles, high pressure homogenization, or spray drying.

Capsules, blisters and cartridges for use in an inhaler or insufflator may be formulated to contain a powder mix of the compound of the application, a suitable powder base and a performance modifier.

Suitable flavours, such as menthol and levomenthol, or sweeteners, such as saccharin or saccharin sodium, may be added to those formulations of the application intended for inhaled/intranasal administration.

Formulations for inhaled/intranasal administration may be formulated to be immediate- and/or modified release. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the application are typically arranged to administer a metered dose or "puff" of an antibody of the application. The overall daily dose will typically be administered in a single dose or, more usually, as divided doses throughout the day.

The antibodies and antibody portions of the application may also be formulated for an oral route administration. Oral administration may involve swallowing, so that the compound enters the gastrointestinal tract, and/or buccal, lingual, or sublingual administration by which the compound enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Pharmaceutical compositions intended for oral use may be prepared according to any method known to the art for the manufacture of pharmaceutical compositions and such compositions may contain one or more agents selected from the group consisting of sweetening agents in order to provide a pharmaceutically elegant and palatable preparation. For example, to prepare orally deliverable tablets, the antibody or antigen-binding fragment thereof is mixed with at least one pharmaceutical excipient, and the solid formulation is compressed to form a tablet according to known methods, for delivery to the gastrointestinal tract. The tablet composition is typically formulated with additives, e.g. a saccharide or cellulose carrier, a binder such as starch paste or methyl cellulose, a filler, a disintegrator, or other additives typically usually used in the manufacture of medical preparations. To prepare orally deliverable capsules, DHEA is mixed with at least one pharmaceutical excipient, and the solid formulation is placed in a capsular container suitable for delivery to the gastrointestinal tract. Compositions comprising antibodies or antigen-binding fragments thereof may be prepared as described generally in Remington's Pharmaceutical Sciences, 18th Ed. 1990 (Mack Publishing Co. Easton Pa. 18042) at Chapter 89.

In various embodiments, the pharmaceutical compositions are formulated as orally deliverable tablets containing antibodies or antigen-binding fragments thereof in admixture with non-toxic pharmaceutically acceptable excipients which are suitable for manufacture of tablets. These excipients may be inert diluents, such as calcium carbonate, sodium carbonate, lactose, calcium phosphate or sodium phosphate, granulating and disintegrating agents, for example, maize starch, gelatin or acacia, and lubricating agents, for example, magnesium stearate, stearic acid, or talc. The tablets may be uncoated, or they may be coated with known techniques to delay disintegration and absorption in the gastrointestinal track and thereby provide a sustained action over a longer period of time. For example, a time delay material such as glyceryl monostearate or glyceryl distearate alone or with a wax may be employed.

In various embodiments, the pharmaceutical compositions are formulated as hard gelatin capsules wherein the antibody or antigen-binding fragment thereof is mixed with an inert solid diluent, for example, calcium carbonate, calcium phosphate, or kaolin or as soft gelatin capsules wherein the antibody or antigen-binding fragment thereof is mixed with an aqueous or an oil medium, for example, arachis oil, peanut oil, liquid paraffin or olive oil.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

### Therapeutic Uses

In another aspect disclosed herein, the present application provides methods for treating cancer in a subject, comprising administering to said subject a therapeutically effective amount (either as monotherapy or in a combination therapy regimen) of an antibody or antigen-binding fragment thereof of the present application. Such disorders include, but are not limited to solid tumors, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, and combinations of said cancers. In various embodiments, the subject previously responded to treatment with an anti-cancer therapy, but, upon cessation of therapy, suffered relapse (hereinafter "a recurrent cancer"). In various embodiments, the subject has resistant or refractory cancer. In various embodiments, the cancerous cells are immunogenic tumors (e.g., those tumors for which vaccination using the tumor itself can lead to immunity to tumor challenge). In various embodiments, the cancer is selected from the group consisting of colorectal cancer (CRC), renal cancer, non-small-cell lung cancer (NSCLC), prostate cancer, breast cancer, ovarian cancer, pancreatic cancer, gastric cancer, and glioma.

In some embodiments, the cancer is selected from the group consisting of prostate cancer, breast cancer, colon cancer, pancreatic cancer, and gastric cancer.

In various embodiments, the present antibodies and antigen-binding fragments thereof can be utilized to directly kill or ablate cancerous cells *in vivo.* Direct killing involves administering the antibodies (which are optionally fused to a cytotoxic drug) to a subject requiring such treatment. In various embodiments, the cancer comprises cancer cells expressing Trop-2 at a higher level than noncancerous cells of a comparable tissue. Since the antibodies recognize Trop-2 on cancer cells, any such cells to which the antibodies bind are destroyed. Where the antibodies are used alone to kill or ablate cancer cells, such killing or ablation can be affected by initiating endogenous host immune functions, such as CDC and/or ADCC. Assays for determining whether an antibody kills cells in this manner are within the purview of those skilled in the art.

In some embodiments, the cancer is Trop2 positive and/or HER2 positive.

In some embodiments, the cancer is Trop2 medium or high and/or HER2 medium or high.

In some embodiments, the cancer is Trop2 low and/or HER2 low.

The expression level of Trop2 or HER2 can be assessed via suitable methods, such as immunohistochemistry (IHC) staining on e.g., tumor sections from specimens from the subject, and the catogorization of low, medium and high expression of Trop2 and/or HER2 is based upon the type of cancer and/or criteria widely acceptable in the field, for example, those illustrated in Zhang et al., BMC Cancer. 2020 Aug 27;20(1):815; Ahn et al., J Pathol Transl Med. 2020 Jan; 54(1): 34-44; and Inamura et al., Oncotarget. 2017 Apr 25; 8(17): 28725-28735.

In some embodiments, the cancer is Trop2 low when it has a similar level of Trop 2 expression as that on Colo205 cells or BxPC-3 cells (e.g., within 25% or 50% less or more of the level of Trop 2 expression on Colo205 or BxPC3). In some embodiments, the cancer is Trop 2 high when it has a similar level of Trop 2 expression as that on NCI-N87 cells or SK-BR-3 cells (e.g., within 25% or 50% less or more of the level of Trop 2 expression on NCI-N87 cells or SK-BR-3 cells). In some embodiments, the cancer is Trop 2 medium when it is higher than 25%, 50%, 75%, or 100% more of the level of Trop 2 expression on Colo205 or BxPC3 and lower than 100%, 75%, 50% or 25% less of the level of Trop 2 expression on NCI-N87 cells or SK-BR-3 cells.

In some embodiments, the cancer is HER2 low when it has a similar level of HER2 expression as that on Colo205 cells (e.g., within 25% or 50% less or more of the level of HER2 expression on Colo205). In some embodiments, the cancer is HER2 medium when it has a similar level of HER2 expression as that on NCI-N87 or MDA-MB-468 cells (e.g., within 25% or 50% less or more of the level of HER2 expression on NCI-N87 or MDA-MB-468 cells). In some embodiments, the cancer is HER2 high when it has a similar level of HER2 expression as that on BxPC-3 cells (e.g., within 25% or 50% less or more of the level of HER2 expression on BxPC-3 cells).

In various embodiments, the present antibodies and antigen-binding fragments thereof can be utilized to promote growth inhibition and/or proliferation of a cancerous tumor cell. These methods may inhibit or prevent the growth of the cancer cells of said subject, such as for example, by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95%. As a result, where the cancer is a solid tumor, the modulation may reduce the size of the solid tumor by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95%.

The inhibition of the cancer cell proliferation can be measured by cell-based assays, such as bromodeoxyuridine (BRDU) incorporation (Hoshino et al., Int. J. Cancer 38, 369, 1986; Campana et al., J. Immunol. Meth. 107:79, 1988; [³H]-thymidine incorporation (Chen, J., Oncogene 13:1395-403, 1996; Jeoung, J., J. Biol. Chem. 270:18367-73, 1995; the dye Alamar Blue (available from Biosource International) (Voytik-Harbin et al., In Vitro Cell Dev Biol Anim 34:239-46, 1998). The anchorage independent growth of cancer cells is assessed by colony formation assay in soft agar, such as by counting the number of cancer cell colonies formed on top of the soft agar (see Examples and Sambrook et al., Molecular Cloning, Cold Spring Harbor, 1989).

The inhibition of cancer cell growth in a subject may be assessed by monitoring the cancer growth in a subject, for example in an animal model or in human subjects. One exemplary monitoring method is tumorigenicity assays. In one example, a xenograft comprises human cells from a pre-existing tumor or from a tumor cell line. Tumor xenograft assays are known in the art and described herein (see, e.g., Ogawa et al., Oncogene 19:6043-6052, 2000). In another embodiment, tumorigenicity is monitored using the hollow fiber assay, which is described in U.S. Patent No. 5,698,413.

The percentage of the inhibition is calculated by comparing the cancer cell proliferation, anchorage independent growth, or cancer cell growth under modulator treatment with that under negative control condition (typically without modulator treatment). For example, where the number of cancer cells or cancer cell colonies (colony formation assay), or PRDU or [³H]-thymidine incorporation is A (under the treatment of modulators) and C (under negative control condition), the percentage of inhibition would be (C-A)/Cx100%.

Examples of tumor cell lines derived from human tumors and available for use in the *in vitro* and *in vivo* studies include, but are not limited to, leukemia cell lines (e.g., CCRF-CEM, HL-60(TB), K-562, MOLT-4, RPM1-8226, SR, P388 and P388/ADR, H292, MV-411); non-small cell lung cancer cell lines (e.g., A549/ATCC, EKVX, HOP-62, HOP-92, NCI-H226, NCI-H23, NCI-H322M, NCI-H460, NCI-H522 and LXFL 529); small cell lung cancer cell lines (e.g., DMS 114 and SHP-77); colon cancer cell lines (e.g., COLO 205, HCC-2998, HCT-116, HCT-15, HT29, KM12, SW-620, DLD-1 and KM20L2); central nervous system (CNS) cancer cell lines (e.g., SF-268, SF-295, SF-539, SNB-19, SNB-75, U251, SNB-78 and XF 498); melanoma cell lines (e.g., LOX I MVI, MALME-3M, M14, SK-MEL-2, SK-MEL-28, SK-MEL-5, UACC-257, UACC-62, RPMI-7951 and M19-MEL); ovarian cancer cell lines (e.g., IGROV1, OVCAR-3, OVCAR-4, OVCAR-5, OVCAR-8 and SK-OV-3); renal cancer cell lines (e.g., 786-0, A498, ACHN, CAKI-1, RXF 393, SN12C, TK-10, UO-31, RXF-631 and SN12K1); prostate cancer cell lines (e.g., PC-3 and DU-145); breast cancer cell lines (e.g., MCF7, NCI/ADR-RES, MDA-MB-231/ATCC, HS 578T, MDA-MB-435, BT-549, T-47D and MDA-MB-468); and thyroid cancer cell lines (e.g., SK-N-SH).

In another aspect disclosed herein, the present application provides methods for treating an infectious disease in a subject, comprising administering to the subject a therapeutically effective amount (either as monotherapy or in a combination therapy regimen) of an isolated antibody or antigen-binding fragment of the present application. In various embodiments, the subject has an infectious disease that was caused by a pathogenic virus. In various embodiments, the subject has an infectious disease that was caused by a pathogenic bacteria. In various embodiments, the subject has an infectious disease that was caused by a pathogenic fungi. In various embodiments, the subject has an infectious disease that was caused by a pathogenic parasite. In various embodiments, the subject has an infectious disease that is resistant to, or ineffectively treated by, treatment using conventional vaccines

"Therapeutically effective amount" or "therapeutically effective dose" refers to that amount of the therapeutic agent being administered which will relieve to some extent one or more of the symptoms of the disorder being treated.

A therapeutically effective dose can be estimated initially from cell culture assays by determining an IC₅₀. A dose can then be formulated in animal models to achieve a circulating plasma concentration range that includes the IC₅₀ as determined in cell culture. Such information can be used to more accurately determine useful doses in humans. Levels in plasma may be measured, for example, by HPLC. The exact composition, route of administration and dosage can be chosen by the individual physician in view of the subject's condition.

Dosage regimens can be adjusted to provide the optimum desired response (e.g., a therapeutic or prophylactic response). For example, a single bolus can be administered, several divided doses (multiple or repeat or maintenance) can be administered over time and the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the present disclosure will be dictated primarily by the unique characteristics of the antibody and the particular therapeutic or prophylactic effect to be achieved.

Thus, the skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a subject may also be determined, as can the temporal requirements for administering each agent to provide a detectable therapeutic benefit to the subject. Accordingly, while certain dose and administration regimens are exemplified herein, these examples in no way limit the dose and administration regimen that may be provided to a subject in practicing the present disclosure.

It is to be noted that dosage values may vary with the type and severity of the condition to be alleviated and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. Further, the dosage regimen with the compositions of this disclosure may be based on a variety of factors, including the type of disease, the age, weight, sex, medical condition of the subject, the severity of the condition, the route of administration, and the particular antibody employed. Thus, the dosage regimen can vary widely, but can be determined routinely using standard methods. For example, doses may be adjusted based on pharmacokinetic or pharmacodynamic parameters, which may include clinical effects such as toxic effects and/or laboratory values. Thus, the present disclosure encompasses intra-subject dose-escalation as determined by the skilled artisan. Determining appropriate dosages and regimens are well-known in the relevant art and would be understood to be encompassed by the skilled artisan once provided the teachings disclosed herein.

For administration to human subjects, the total monthly dose of the antibodies or antigen-binding fragments thereof of the disclosure can be in the range of 0.5-1200 mg per subject, 0.5-1100 mg per subject, 0.5-1000 mg per subject, 0.5-900 mg per subject, 0.5-800 mg per subject, 0.5-700 mg per subject, 0.5-600 mg per subject, 0.5-500 mg per subject, 0.5-400 mg per subject, 0.5-300 mg per subject, 0.5-200 mg per subject, 0.5-100 mg per subject, 0.5-50 mg per subject, 1-1200 mg per subject, 1-1100 mg per subject, 1-1000 mg per subject, 1-900 mg per subject, 1-800 mg per subject, 1-700 mg per subject, 1-600 mg per subject, 1-500 mg per subject, 1-400 mg per subject, 1-300 mg per subject, 1-200 mg per subject, 1-100 mg per subject, or 1-50 mg per subject depending, of course, on the mode of administration. For example, an intravenous monthly dose can require about 1-1000 mg/subject. In various embodiments, the antibodies or antigen-binding fragments thereof of the disclosure can be administered at about 1-200 mg per subject, 1-150 mg per subject or 1-100 mg/subject. The total monthly dose can be administered in single or divided doses and can, at the physician's discretion, fall outside of the typical ranges given herein.

In some embodiments, the construct is administered to a subject at a dose of at least about 5mg/kg. In some embodiments, the construct is administered to a human at a dose equivalent to a dose of at least 5mg/kg in mice. See Nair, A. B., & Jacob, S. (2016). A simple practice guide for dose conversion between animals and human. Journal of basic and clinical pharmacy, 7(2), 27.

For repeated administrations over several days or longer, depending on the condition, the treatment is sustained until a desired suppression of symptoms occurs or until sufficient therapeutic levels are achieved, for example, to reduce pain. An exemplary dosing regimen comprises administering an initial dose of about 2 mg/kg, followed by a weekly maintenance dose of about 1 mg/kg of the bispecific anti-HER2/Trop-2 antibody, or followed by a maintenance dose of about 1 mg/kg every other week. However, other dosage regimens may be useful, depending on the pattern of pharmacokinetic decay that the practitioner wishes to achieve. For example, in some embodiments, dosing from one-four times a week is contemplated. The progress of this therapy is easily monitored by conventional techniques and assays. The dosing regimen (including the HER2/Trop-2 antagonist(s) used) can vary over time. In various embodiments, the appropriate dosage of an HER2/Trop-2 antagonist antibody will depend on the bispecific anti-HER2/Trop-2 antibody (or compositions thereof) employed, the type and severity of headache (e.g., migraine) to be treated, whether the agent is administered for preventive or therapeutic purposes, previous therapy, the patient's clinical history and response to the agent, and the discretion of the attending physician. Typically, the clinician will administer a bispecific anti-HER2/Trop-2 antibody, until a dosage is reached that achieves the desired result. Dose and/or frequency can vary over course of treatment.

It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

In various embodiments, the total dose administered will achieve a plasma antibody concentration in the range of, e.g., about 1 to 1000 µg/ml, about 1 to 750 µg/ml, about 1 to 500 µg/ml, about 1 to 250 µg/ml, about 10 to 1000 µg/ml, about 10 to 750 µg/ml, about 10 to 500 µg/ml, about 10 to 250 µg/ml, about 20 to 1000 µg/ml, about 20 to 750 µg/ml, about 20 to 500 µg/ml, about 20 to 250 µg/ml, about 30 to 1000 µg/ml, about 30 to 750 µg/ml, about 30 to 500 µg/ml, about 30 to 250 µg/ml.

Toxicity and therapeutic index of the pharmaceutical compositions of the application can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effective dose is the therapeutic index and it can be expressed as the ratio LD₅₀/ED₅₀. Compositions that exhibit large therapeutic indices are generally preferred.

In various embodiments, single or multiple administrations of the pharmaceutical compositions are administered depending on the dosage and frequency as required and tolerated by the subject. In any event, the composition should provide a sufficient quantity of at least one of the antibodies or antigen-binding fragments thereof disclosed herein to effectively treat the subject. The dosage can be administered once but may be applied periodically until either a therapeutic result is achieved or until side effects warrant discontinuation of therapy.

The dosing frequency of the administration of the antibody or antigen-binding fragment thereof pharmaceutical composition depends on the nature of the therapy and the particular disease being treated. The subject can be treated at regular intervals, such as weekly or monthly, until a desired therapeutic result is achieved. Exemplary dosing frequencies include, but are not limited to: once weekly without break; once weekly, every other week; once every 2 weeks; once every 3 weeks; weakly without break for 2 weeks, then monthly; weakly without break for 3 weeks, then monthly; monthly; once every other month; once every three months; once every four months; once every five months; or once every six months, or yearly.

### Combination Therapy

As used herein, the terms "co-administration", "co-administered" and "in combination with", referring to the antibodies or antigen-binding fragments thereof of the disclosure and one or more other therapeutic agents, is intended to mean, and does refer to and include the following: simultaneous administration of such combination of antibodies or antigen-binding fragments thereof of the disclosure and therapeutic agent(s) to a subject in need of treatment, when such components are formulated together into a single dosage form which releases said components at substantially the same time to said subject; substantially simultaneous administration of such combination of antibodies or antigen-binding fragments thereof of the disclosure and therapeutic agent(s) to a subject in need of treatment, when such components are formulated apart from each other into separate dosage forms which are taken at substantially the same time by said subject, whereupon said components are released at substantially the same time to said subject; sequential administration of such combination of antibodies or antigen-binding fragments thereof of the disclosure and therapeutic agent(s) to a subject in need of treatment, when such components are formulated apart from each other into separate dosage forms which are taken at consecutive times by said subject with a significant time interval between each administration, whereupon said components are released at substantially different times to said subject; and sequential administration of such combination of antibodies or antigen-binding fragments thereof of the disclosure and therapeutic agent(s) to a subject in need of treatment, when such components are formulated together into a single dosage form which releases said components in a controlled manner whereupon they are concurrently, consecutively, and/or overlappingly released at the same and/or different times to said subject, where each part may be administered by either the same or a different route.

In another aspect, the present application relates to combination therapies designed to treat a cancer, or an infectious disease, in an subject, comprising administering to the subject a therapeutically effective amount of an isolated antibody or antigen-binding fragment of the present application, and b) one or more additional therapies selected from the group consisting of immunotherapy, chemotherapy, small molecule kinase inhibitor targeted therapy, surgery, radiation therapy, vaccination protocols, and stem cell transplantation, wherein the combination therapy provides increased cell killing of tumor cells, i.e., a synergy exists between the isolated antibody or antigen-binding fragment and the additional therapies when co-administered.

In various embodiments, the immunotherapy is selected from the group consisting of: treatment using agonistic, antagonistic, or blocking antibodies to co-stimulatory or co-inhibitory molecules (immune checkpoints) such as PD-1, PD-L1, OX-40, CD137, GITR, LAG3, TIM-3, and VISTA; treatment using bispecific T cell engaging antibodies (BiTE^{®}) such as blinatumomab: treatment involving administration of biological response modifiers such as IL-2, IL-12, IL-15, IL-21, GM-CSF, IFN-α, IFN-β and IFN-γ; treatment using therapeutic vaccines such as sipuleucel-T; treatment using dendritic cell vaccines, or tumor antigen peptide vaccines; treatment using chimeric antigen receptor (CAR)-T cells; treatment using CAR-NK cells; treatment using tumor infiltrating lymphocytes (TILs); treatment using adoptively transferred anti-tumor T cells (ex vivo expanded and/or TCR transgenic); treatment using TALL-104 cells; and treatment using immunostimulatory agents such as Toll-like receptor (TLR) agonists CpG and imiquimod.

A wide array of conventional compounds has been shown to have anti-neoplastic activities. These compounds have been used as pharmaceutical agents in chemotherapy to shrink solid tumors, prevent metastases and further growth, or decrease the number of malignant T-cells in leukemic or bone marrow malignancies. Although chemotherapy has been effective in treating various types of malignancies, many anti-neoplastic compounds induce undesirable side effects. It has been shown that when two or more different treatments are combined, the treatments may work synergistically and allow reduction of dosage of each of the treatments, thereby reducing the detrimental side effects exerted by each compound at higher dosages. In other instances, malignancies that are refractory to a treatment may respond to a combination therapy of two or more different treatments.

When the bispecific antibodies disclosed herein are administered in combination with another conventional anti-neoplastic agent, either concomitantly or sequentially, such antibody or antigen-binding fragment may enhance the therapeutic effect of the anti-neoplastic agent or overcome cellular resistance to such anti-neoplastic agent. This allows decrease of dosage of an anti-neoplastic agent, thereby reducing the undesirable side effects, or restores the effectiveness of an anti-neoplastic agent in resistant T-cells.

Pharmaceutical compounds that may be used for combinatory anti-tumor therapy include, merely to illustrate: aminoglutethimide, amsacrine, anastrozole, asparaginase, bcg, bicalutamide, bleomycin, buserelin, busulfan, campothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These chemotherapeutic anti-tumor compounds may be categorized by their mechanism of action into, for example, following groups: anti-metabolites/anti-cancer agents, such as pyrimidine analogs (5-fluorouracil, floxuridine, capecitabine, gemcitabine and cytarabine) and purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristin, vinblastin, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamineoxaliplatin, iphosphamide, melphalan, merchlorehtamine, mitomycin, mitoxantrone, nitrosourea, plicamycin, procarbazine, taxol, taxotere, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes-dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); anti-angiogenic compounds (TNP-470, genistein) and growth factor inhibitors (vascular endothelial growth factor (VEGF) inhibitors, fibroblast growth factor (FGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylpednisolone, prednisone, and prenisolone); growth factor signal transduction kinase inhibitors; mitochondrial dysfunction inducers and caspase activators; and chromatin disruptors.

In various embodiments, the chemotherapy comprises a chemotherapeutic agent selected from the group consisting of: daunorubicin, dactinomycin, doxorubicin, bleomycin, mitomycin, nitrogen mustard, chlorambucil, melphalan, cyclophosphamide, 6-mercaptopurine, 6-thioguanine, bendamustine, cytarabine (CA), 5-fluorouracil (5-FU), floxuridine (5-FUdR), methotrexate (MTX), colchicine, vincristine, vinblastine, etoposide, teniposide, cisplatin, carboplatin, oxaliplatin, pentostatin, cladribine, cytarabine, gemcitabine, pralatrexate, mitoxantrone, diethylstilbestrol (DES), fluradabine, ifosfamide, hydroxyureataxanes (such as paclitaxel and doxetaxel) and/or anthracycline antibiotics, as well as combinations of agents such as, but not limited to, DA-EPOCH, CHOP, CVP or FOLFOX.

In various embodiments, the small molecule kinase inhibitor targeted therapy comprises a small molecule kinase inhibitor selected from the group consisting of Bruton's tyrosine kinase (BTK) inhibitor, phosphatidylinositol-3-kinase (PI3K) inhibitor, SYK inhibitor (e.g., entospletinib), AKT inhibitor, mTOR inhibitor, Src inhibitor, JAK/STAT inhibitor, Ras/Raf/MEK/ERK inhibitor, and Aurora inhibitor (see, D'Cruz et al, Expert Opin Pharmacother, 14(6): 707-21, 2013).

In various embodiments, the combination therapy comprises administering the antibody or antigen-binding fragment thereof and the one or more additional therapies simultaneously. In various embodiments, antibody or antigen-binding fragment thereof composition and the one or more additional therapies are administered sequentially, i.e., the antibody or antigen-binding fragment thereof composition is administered either prior to or after the administration of the one or more additional therapies.

In various embodiments, the administrations of the antibody or antigen-binding fragment thereof composition and the one or more additional therapies are concurrent, i.e., the administration period of the antibody or antigen-binding fragment thereof composition and the one or more additional therapies overlap with each other.

In various embodiments, the administrations of the antibody or antigen-binding fragment thereof composition and the one or more additional therapies are non-concurrent. For example, in various embodiments, the administration of the antibody or antigen-binding fragment thereof composition is terminated before the one or more additional therapies is administered. In various embodiments, the administration of the one or more additional therapies is terminated before the antibody or antigen-binding fragment thereof composition is administered.

When the bispecific antibody disclosed herein is administered in combination with one or more additional therapies, either concomitantly or sequentially, such antibody or antigen-binding fragment thereof may enhance the therapeutic effect of the one or more additional therapies or overcome cellular resistance to the one or more additional therapies. This allows for decreased dosage or duration of the one or more additional therapies, thereby reducing the undesirable side effects, or restores the effectiveness of the one or more additional therapies.

### Diagnostic Uses

In another aspect disclosed herein, the present application provides a method for detecting in vitro or in vivo the presence of human HER2 and Trop-2 peptides in a sample, e.g., for diagnosing a human HER2- and Trop-2-related disorder. In some methods, this is achieved by contacting a sample to be tested, along with a control sample, a bispecific antibody, under conditions that allow for formation of a complex between the antibody and human HER2 and Trop-2. Complex formation is then detected (e.g., using an ELISA) in both samples, and any statistically significant difference in the formation of complexes between the samples is indicative the presence of human HER2 and Trop-2 antigen in the test sample.

In various embodiments disclosed herein, methods are provided for detecting cancer or confirming the diagnosis of cancer in a subject. The method includes contacting a biological sample from the subject with an isolated antibody or antigen-biding fragment thereof of the application and detecting binding of the isolated human monoclonal antibody or antigen-binding fragment thereof to the sample. An increase in binding of the isolated human monoclonal antibody or antigen-binding fragment thereof to the sample as compared to binding of the isolated human monoclonal antibody or antigen-binding fragment thereof to a control sample detects cancer in the subject or confirms the diagnosis of cancer in the subject. The control can be a sample from a subject known not to have cancer, or a standard value. The sample can be any sample, including, but not limited to, tissue from biopsies, autopsies and pathology specimens. Biological samples also include sections of tissues, for example, frozen sections taken for histological purposes. Biological samples further include body fluids, such as blood, serum, plasma, sputum, and spinal fluid.

In one embodiment, a kit is provided for detecting HER2 and Trop-2 in a biological sample, such as a blood sample. Kits for detecting a polypeptide will typically comprise a human antibody that specifically binds HER2 and Trop-2, such as any of the antibodies disclosed herein. In some embodiments, an antibody fragment, such as an Fv fragment is included in the kit. For *in vivo* uses, the antibody can be a scFv fragment. In a further embodiment, the antibody is labeled (for example, with a fluorescent, radioactive, or an enzymatic label).

In one embodiment, a kit includes instructional materials disclosing means of use of an antibody that specifically binds HER2 and Trop-2. The instructional materials may be written, in an electronic form (such as a computer diskette or compact disk) or may be visual (such as video files). The kits may also include additional components to facilitate the particular application for which the kit is designed. Thus, for example, the kit may additionally contain means of detecting a label (such as enzyme substrates for enzymatic labels, filter sets to detect fluorescent labels, appropriate secondary labels such as a secondary antibody, or the like). The kits may additionally include buffers and other reagents routinely used for the practice of a particular method. Such kits and appropriate contents are well known to those of skill in the art.

In one embodiment, the diagnostic kit comprises an immunoassay. Although the details of the immunoassays may vary with the particular format employed, the method of detecting HER2 and Trop-2 in a biological sample generally includes the steps of contacting the biological sample with an antibody which specifically reacts, under immunologically reactive conditions, to HER2 and Trop-2. The antibody is allowed to specifically bind under immunologically reactive conditions to form an immune complex, and the presence of the immune complex (bound antibody) is detected directly or indirectly.

In various embodiments, the antibodies or antigen-binding fragments can be labeled or unlabeled for diagnostic purposes. Typically, diagnostic assays entail detecting the formation of a complex resulting from the binding of an antibody to HER2 or Trop-2. The antibodies can be directly labeled. A variety of labels can be employed, including, but not limited to, radionuclides, fluorescers, enzymes, enzyme substrates, enzyme cofactors, enzyme inhibitors and ligands (e.g., biotin, haptens). Numerous appropriate immunoassays are known to the skilled artisan (see, for example, U.S. Patent Nos. 3,817,827; 3,850,752; 3,901,654; and 4,098,876). When unlabeled, the antibodies can be used in assays, such as agglutination assays. Unlabeled antibodies can also be used in combination with another (one or more) suitable reagent which can be used to detect antibody, such as a labeled antibody (e.g., a second antibody) reactive with the first antibody (e.g., anti-idiotype antibodies or other antibodies that are specific for the unlabeled immunoglobulin) or other suitable reagent (e.g., labeled protein A).

The antibody or antigen-binding fragment provided herein may also be used in a method of detecting the susceptibility of a mammal to certain diseases. To illustrate, the method can be used to detect the susceptibility of a mammal to diseases which progress based on the amount of HER2 and Trop-2 present on cells and/or the number of HER2- and Trop-2-positive cells in a mammal. In one embodiment, the application provides a method of detecting susceptibility of a mammal to a tumor. In this embodiment, a sample to be tested is contacted with an antibody which binds to HER2 and Trop-2 or portion thereof under conditions appropriate for binding of said antibody thereto, wherein the sample comprises cells which express HER2 and Trop-2 in normal individuals. The binding of antibody and/or amount of binding is detected, which indicates the susceptibility of the individual to a tumor, wherein higher levels of receptor correlate with increased susceptibility of the individual to a tumor.

In various embodiments, the antibodies or antigen-binding fragments are attached to a label that is able to be detected (e.g., the label can be a radioisotope, fluorescent compound, enzyme or enzyme co-factor). The active moiety may be a radioactive agent, such as: radioactive heavy metals such as iron chelates, radioactive chelates of gadolinium or manganese, positron emitters of oxygen, nitrogen, iron, carbon, or gallium, ⁴³K, ⁵²Fe, ⁵⁷Co, ⁸⁷Cu, ⁶⁷Ga, ⁶⁸Ga, ¹²³I, ¹²⁵I, ¹³¹I, ¹³²I, or ⁹⁹Tc. A binding agent affixed to such a moiety may be used as an imaging agent and is administered in an amount effective for diagnostic use in a mammal such as a human and the localization and accumulation of the imaging agent is then detected. The localization and accumulation of the imaging agent may be detected by radioscintigraphy, nuclear magnetic resonance imaging, computed tomography or positron emission tomography.

Immunoscintigraphy using antibodies or antigen-binding fragments directed at HER2 and Trop-2 may be used to detect and/or diagnose cancers and vasculature. For example, monoclonal antibodies against the HER2 and Trop-2 marker labeled with ⁹⁹Technetium, ¹¹¹Indium, or ¹²⁵Iodine may be effectively used for such imaging. As will be evident to the skilled artisan, the amount of radioisotope to be administered is dependent upon the radioisotope. Those having ordinary skill in the art can readily formulate the amount of the imaging agent to be administered based upon the specific activity and energy of a given radionuclide used as the active moiety. Typically, 0.1-100 millicuries per dose of imaging agent, or 1-10 millicuries, or 2-5 millicuries are administered. Thus, the compositions disclosed are useful as imaging agents comprising a targeting moiety conjugated to a radioactive moiety comprise 0.1-100 millicuries, in some embodiments 1-10 millicuries, in some embodiments 2-5 millicuries, in some embodiments 1-5 millicuries.

### Immunoconjugates

The application further provides immunoconjugates comprising at least one of the bispecific anti-HER2/Trop-2 antibody or antigen-binding fragment thereof of the present application conjugated (or linked) directly or indirectly to an effector molecule. In this regard, the term "conjugated" or "linked" refers to making two polypeptides into one contiguous polypeptide molecule. The linkage can be either by chemical or recombinant means. In one embodiment, the linkage is chemical, wherein a reaction between the antibody moiety and the effector molecule has produced a covalent bond formed between the two molecules to form one molecule. A peptide linker (short peptide sequence) can optionally be included between the antibody and the effector molecule. In various embodiments, an antibody or antigen-binding fragment is joined to an effector molecule. In other embodiments, an antibody or antigen-binding fragment joined to an effector molecule is further joined to a lipid, a protein or peptide to increase its half-life in the body. Accordingly, in various embodiments, the antibodies of the present disclosure may be used to deliver a variety of effector molecules.

The effector molecule can be a radioisotope, a radioisotope, an immunomodulator, a cytokine, a lymphokine, a chemokine, a growth factor, a tumor necrosis factor, a hormone, a hormone antagonist, an enzyme, an oligonucleotide, a DNA, an RNA, an siRNA, an RNAi, a microRNA, a photoactive therapeutic agent, an anti-angiogenic agent, a pro-apoptotic agent, a peptide a lipid, a carbohydrate, a chelating agentor, therapeutic agent, or chemotherapeutic agent.

Specific, non-limiting examples of immunotoxins include, but are not limited to, abrin, ricin, Pseudomonas exotoxin (PE, such as PE35, PE37, PE38, and PE40), diphtheria toxin (DT), botulinum toxin, cholix toxin, or modified toxins thereof, or other toxic agents that directly or indirectly inhibit cell growth or kill cells.

A "cytokine" is class of proteins or peptides released by one cell population which act on another cell as intercellular mediators. Cytokines can act as an immune-modulating agent. Examples of cytokines include lymphokines, monokines, growth factors and traditional polypeptide hormones. Thus, embodiments may utilize an interferon (e.g., IFN-α, IFN-β, and IFN-y); tumor necrosis factor super family (TNFSF) member; human growth hormone; thyroxine; insulin; proinsulin; relaxin; prorelaxin; follicle stimulating hormone (FSH); thyroid stimulating hormone (TSH); luteinizing hormone (LH); hepatic growth factor; prostaglandin, fibroblast growth factor; prolactin; placental lactogen, OB protein; TNF-α; TNF-β; integrin; thrombopoietin (TPO); a nerve growth factor such as NGF-β.; platelet-growth factor; TGF-α; TGF-β; insulin-like growth factor-I and -II; erythropoietin (EPO); colony stimulating factors (CSFs) such as macrophage-CSF (M-CSF); granulocyte-macrophage-CSF (GM-CSF); and granulocyte-CSF (G-CSF); an interleukin (IL-1 to IL-21), kit-ligand or FLT-3, angiostatin, thrombospondin, or endostatin. These cytokines include proteins from natural sources or from recombinant cell culture and biologically active equivalents of the native sequence cytokines.

Chemokines can also be conjugated to the antibodies disclosed herein. Chemokines are a superfamily of small (approximately about 4 to about 14 kDa), inducible and secreted pro-inflammatory cytokines that act primarily as chemoattractants and activators of specific leukocyte cell subtypes. Chemokine production is induced by inflammatory cytokines, growth factors and pathogenic stimuli. The chemokine proteins are divided into subfamilies (alpha, beta, and delta) based on conserved amino acid sequence motifs and are classified into four highly conserved groups--CXC, CC, C and CX3C, based on the position of the first two cysteines that are adjacent to the amino terminus. To date, more than 50 chemokines have been discovered and there are at least 18 human seven-transmembrane-domain (7TM) chemokine receptors. Chemokines of use include, but are not limited to, RANTES, MCAF, MCP-1, and fractalkine.

The therapeutic agent can be a chemotherapeutic agent. One of skill in the art can readily identify a chemotherapeutic agent of use (e.g. see Slapak and Kufe, Principles of Cancer Therapy, Chapter 86 in Harrison's Principles of Internal Medicine, 14th edition; Perry et al., Chemotherapy, Ch. 17 in Abeloff, Clinical Oncology 2nd ed.®. 2000 Churchill Livingstone, Inc; Baltzer L., Berkery R. (eds): Oncology Pocket Guide to Chemotherapy, 2nd ed. St. Louis, Mosby-Year Book, 1995; Fischer D S, Knobf M F, Durivage H J (eds): The Cancer Chemotherapy Handbook, 4th ed. St. Louis, Mosby-Year Book, 1993). Useful chemotherapeutic agents for the preparation of immunoconjugates include auristatin, dolastatin, MMAE, MMAF, tubulysin, AFP, DM1, DM4, calicheamicin, duocarmycin, pyrrolobenzodiazepine, AEB, doxorubicin, daunorubicin, methotrexate, melphalan, chlorambucil, vinca alkaloids, 5-fluorouridine, mitomycin-C, taxol, L-asparaginase, mercaptopurine, thioguanine, hydroxyurea, cytarabine, cyclophosphamide, ifosfamide, nitrosoureas, cisplatin, carboplatin, mitomycin, dacarbazine, procarbazine, topotecan, nitrogen mustards, cytoxan, etoposide, BCNU, irinotecan, camptothecins, bleomycin, idarubicin, dactinomycin, plicamycin, mitoxantrone, asparaginase, vinblastine, vincristine, vinorelbine, paclitaxel, and docetaxel and salts, solvents and derivatives thereof. In various embodiments, the chemotherapeutic agent is auristatin E (also known in the art as dolastatin-10) or a derivative thereof as well as pharmaceutically salts or solvates thereof. Typical auristatin derivatives include DM1, AEB, AEVB, AFP, MMAF, and MMAE. The synthesis and structure of auristatin E and its derivatives, as well as linkers, are described in, e.g., U.S. Patent Application Publication No. 20030083263; U.S. Patent Application Publication No. 20050238629; and U.S. Patent No. 6,884,869. In various embodiments, the therapeutic agent is an auristatin or an auristatin derivative. In various embodiments, the auristatin derivative is dovaline-valine-dolaisoleunine-dolaproine-phenylalanine (MMAF) or monomethyauristatin E (MMAE). In various embodiments, the therapeutic agent is a maytansinoid or a maytansinol analogue. In various embodiments, the maytansinoid is DM1 and DM4 derivatives. In various embodiments, the therapeutic agent is a calicheamicin, variant, or derivative. In various embodiments, the therapeutic agent is a duocarmycin, variant, or derivative.

The effector molecules can be linked to an antibody or antigen-binding fragment of the present application using any number of means known to those of skill in the art. Both covalent and noncovalent attachment means may be used. The procedure for attaching an effector molecule to an antibody varies according to the chemical structure of the effector molecule. Polypeptides typically contain a variety of functional groups, such as carboxylic acid (COOH), free amine (--NH₂) or sulfhydryl (--SH) groups, which are available for reaction with a suitable functional group on an antibody to result in the binding of the effector molecule. Alternatively, the antibody is derivatized to expose or attach additional reactive functional groups. The derivatization may involve attachment of any of a number of linker molecules such as those available from Pierce Chemical Company, Rockford, Ill. The linker can be any molecule used to join the antibody to the effector molecule. The linker is capable of forming covalent bonds to both the antibody and to the effector molecule. Suitable linkers are well known to those of skill in the art and include, but are not limited to, straight or branched-chain carbon linkers, heterocyclic carbon linkers, or peptide linkers. Where the antibody and the effector molecule are polypeptides, the linkers may be joined to the constituent amino acids through their side groups (such as through a disulfide linkage to cysteine) or to the alpha carbon amino and carboxyl groups of the terminal amino acids.

In some circumstances, it is desirable to free the effector molecule from the antibody when the immunoconjugate has reached its target site. Therefore, in these circumstances, immunoconjugates will comprise linkages that are cleavable in the vicinity of the target site. Cleavage of the linker to release the effector molecule from the antibody may be prompted by enzymatic activity or conditions to which the immunoconjugate is subjected either inside the target cell or in the vicinity of the target site.

Procedures for conjugating the antibodies with the effector molecules have been previously described and are within the purview of one skilled in the art. For example, procedures for preparing enzymatically active polypeptides of the immunotoxins are described in WO84/03508 and WO85/03508. Other techniques are described in Shih et al., Int. J. Cancer 41:832-839 (1988); Shih et al., Int. J. Cancer 46:1101-1106 (1990); Shih et al., U.S. Pat. No. 5,057,313; Shih Cancer Res. 51:4192, International Publication WO 02/088172; U.S. Pat. No. 6,884,869; International Patent Publication WO 2005/081711; U.S. Patent Application Publication No. 2003-0130189 A; and US Patent Application Publication No. 20080305044.

An immunoconjugate of the present application retains the immunoreactivity of the antibody or antigen-binding fragment, e.g., the antibody or antigen-binding fragment has approximately the same, or only slightly reduced, ability to bind the antigen after conjugation as before conjugation.

### Antibody Drug Conjugates (ADCs)

As used herein, an immunoconjugate is also referred to as an antibody drug conjugate (ADC). An antibody drug conjugates (ADC) in accordance with one embodiment of the application has the formula: Ab-(L-D)n, wherein Ab is a bispecific anti-HER2/Trop-2 binding antibody, L is a linker, D is an effector molecule that is an immunotoxin, drug moiety, cytokine, chemokine, therapeutic agent, or chemotherapeutic agent, and n is an integer from 1, 2, 3, 4, 5, 6, 7, 8, 9, 10.

In various embodiments, the ADC comprises two, three, four, five, six, seven, eight, nine or ten therapeutic moieties. In various embodiments, the ADC comprises two, three, or four therapeutic moieties. In various embodiments, all therapeutic moieties are the same. In various embodiments, at least one therapeutic moiety is different from the rest. In various embodiments, all therapeutic moieties are different.

### Polynucleotides and Antibody Expression

The application further provides polynucleotides comprising a nucleotide sequence encoding an anti-HER2/Trop-2 antibody or antigen-binding fragment thereof. Because of the degeneracy of the genetic code, a variety of nucleic acid sequences encode each antibody amino acid sequence. The application further provides polynucleotides that hybridize under stringent or lower stringency hybridization conditions, e.g., as defined herein, to polynucleotides that encode an antibody that binds to human HER2 and Trop-2.

Stringent hybridization conditions include, but are not limited to, hybridization to filter-bound DNA in 6xSSC at about 45°C followed by one or more washes in 0.2xSSC/0.1% SDS at about 50-65°C, highly stringent conditions such as hybridization to filter-bound DNA in 6xSSC at about 45°C followed by one or more washes in 0.1xSSC/0.2% SDS at about 60°C, or any other stringent hybridization conditions known to those skilled in the art (see, for example, Ausubel, F. M. et al., eds. 1989 Current Protocols in Molecular Biology, vol. 1, Green Publishing Associates, Inc. and John Wiley and Sons, Inc., NY at pages 6.3.1 to 6.3.6 and 2.10.3).

The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR. In one embodiment, the codons that are used comprise those that are typical for human or mouse (see, e.g., Nakamura, Y., Nucleic Acids Res. 28: 292 (2000)).

A polynucleotide encoding an antibody may also be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (e.g., an antibody cDNA library, or a cDNA library generated from, or nucleic acid, preferably polyA+RNA, isolated from, any tissue or cells expressing the antibody, such as hybridoma cells selected to express an antibody) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

The present application is also directed to host cells that express the anti-HER2/Trop-2 antibodies of the application. A wide variety of host expression systems known in the art can be used to express an antibody of the present application including prokaryotic (bacterial) and eukaryotic expression systems (such as yeast, baculovirus, plant, mammalian and other animal cells, transgenic animals, and hybridoma cells), as well as phage display expression systems.

A bispecific antibody of the application can be prepared by recombinant expression of immunoglobulin light and heavy chain genes in a host cell. To express an antibody recombinantly, a host cell is transformed, transduced, infected or the like with one or more recombinant expression vectors carrying DNA fragments encoding the immunoglobulin light and/or heavy chains of the antibody such that the light and/or heavy chains are expressed in the host cell. The heavy chain and the light chain may be expressed independently from different promoters to which they are operably-linked in one vector or, alternatively, the heavy chain and the light chain may be expressed independently from different promoters to which they are operably-linked in two vectors one expressing the heavy chain and one expressing the light chain. Optionally, the heavy chain and light chain may be expressed in different host cells.

Additionally, the recombinant expression vector can encode a signal peptide that facilitates secretion of the antibody light and/or heavy chain from a host cell. The antibody light and/or heavy chain gene can be cloned into the vector such that the signal peptide is operably-linked in-frame to the amino terminus of the antibody chain gene. The signal peptide can be an immunoglobulin signal peptide or a heterologous signal peptide. Preferably, the recombinant antibodies are secreted into the medium in which the host cells are cultured, from which the antibodies can be recovered or purified.

An isolated DNA encoding a HCVR can be converted to a full-length heavy chain gene by operably-linking the HCVR-encoding DNA to another DNA molecule encoding heavy chain constant regions. The sequences of human, as well as other mammalian, heavy chain constant region genes are known in the art. DNA fragments encompassing these regions can be obtained e.g., by standard PCR amplification. The heavy chain constant region can be of any type, (e.g., IgG, IgA, IgE, IgM or IgD), class (e.g., IgG₁, IgG₂, IgG₃ and IgG₄) or subclass constant region and any allotypic variant thereof as described in Kabat (supra).

An isolated DNA encoding a LCVR region may be converted to a full-length light chain gene (as well as to a Fab light chain gene) by operably linking the LCVR-encoding DNA to another DNA molecule encoding a light chain constant region. The sequences of human, as well as other mammalian, light chain constant region genes are known in the art. DNA fragments encompassing these regions can be obtained by standard PCR amplification. The light chain constant region can be a kappa or lambda constant region.

In addition to the antibody heavy and/or light chain gene(s), a recombinant expression vector of the application carries regulatory sequences that control the expression of the antibody chain gene(s) in a host cell. The term "regulatory sequence" is intended to include promoters, enhancers and other expression control elements (e.g., polyadenylation signals), as needed, that control the transcription or translation of the antibody chain gene(s). The design of the expression vector, including the selection of regulatory sequences may depend on such factors as the choice of the host cell to be transformed, the level of expression of protein desired. Preferred regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP)) and/or polyoma virus.

Additionally, the recombinant expression vectors of the application may carry additional sequences, such as sequences that regulate replication of the vector in host cells (e.g., origins of replication) and one or more selectable marker genes. The selectable marker gene facilitates selection of host cells into which the vector has been introduced. For example, typically the selectable marker gene confers resistance to drugs, such as G418, hygromycin, or methotrexate, on a host cell into which the vector has been introduced. Preferred selectable marker genes include the dihydrofolate reductase (dhfr) gene (for use in dhfr-minus host cells with methotrexate selection/amplification), the neo gene (for G418 selection), and glutamine synthetase (GS) in a GS-negative cell line (such as NSO) for selection/amplification.

For expression of the light and/or heavy chains, the expression vector(s) encoding the heavy and/or light chains is introduced into a host cell by standard techniques e.g. electroporation, calcium phosphate precipitation, DEAE-dextran transfection, transduction, infection and the like. Although it is theoretically possible to express the antibodies of the application in either prokaryotic or eukaryotic host cells, eukaryotic cells are preferred, and most preferably mammalian host cells, because such cells are more likely to assemble and secrete a properly folded and immunologically active antibody. Preferred mammalian host cells for expressing the recombinant antibodies of the application include Chinese Hamster Ovary (CHO cells) [including dhfr minus CHO cells, as described in Urlaub and Chasin, Proc. Natl. Acad. Sci. USA 77:4216-20, 1980, used with a DHFR selectable marker, e.g. as described in Kaufman and Sharp, J. Mol. Biol. 159:601-21, 1982], NSO myeloma cells, COS cells, and SP2/0 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown under appropriate conditions known in the art. Antibodies can be recovered from the host cell and/or the culture medium using standard purification methods.

The application provides a host cell comprising a nucleic acid molecule of the present application. Preferably a host cell of the application comprises one or more vectors or constructs comprising a nucleic acid molecule of the present application. For example, a host cell of the application is a cell into which a vector of the application has been introduced, said vector comprising a polynucleotide encoding a LCVR of an antibody of the application and/or a polynucleotide encoding a HCVR of the application. The application also provides a host cell into which two vectors of the application have been introduced; one comprising a polynucleotide encoding a LCVR of an antibody of the application and one comprising a polynucleotide encoding a HCVR present in an antibody of the application and each operably-linked to enhancer/promoter regulatory elements (e.g., derived from SV40, CMV, adenovirus and the like, such as a CMV enhancer/AdMLP promoter regulatory element or an SV40 enhancer/AdMLP promoter regulatory element) to drive high levels of transcription of the genes.

Once expressed, the intact antibodies, individual light and heavy chains, or other immunoglobulin forms of the present application can be purified according to standard procedures of the art, including ammonium sulfate precipitation, ion exchange, affinity (e.g., Protein A), reverse phase, hydrophobic interaction column chromatography, hydroxyapatite chromatography, gel electrophoresis, and the like. Standard procedures for purification of therapeutic antibodies are described, for example, by Feng L1, Joe X. Zhou, Xiaoming Yang, Tim Tressel, and Brian Lee in an article entitled "Current Therapeutic Antibody Production and Process Optimization" (BioProcessing Journal, September/October 2005). Additionally, standard techniques for removing viruses from recombinantly expressed antibody preparations are also known in the art (see, for example, Gerd Kern and Mani Krishnan, "Viral Removal by Filtration: Points to Consider" (Biopharm International, October 2006)). The effectiveness of filtration to remove viruses from preparations of therapeutic antibodies is known to be at least in part dependent on the concentration of protein and/or the antibody in the solution to be filtered. The purification process for antibodies of the present application may include a step of filtering to remove viruses from the mainstream of one or more chromatography operations. Preferably, prior to filtering through a pharmaceutical grade nanofilter to remove viruses, a chromatography mainstream containing an antibody of the present application is diluted or concentrated to give total protein and/or total antibody concentration of about 1 g/L to about 3 g/L. Even more preferably, the nanofilter is a DV20 nanofilter (e.g., Pall Corporation; East Hills, N.Y.). Substantially pure immunoglobulins of at least about 90%, about 92%, about 94% or about 96% homogeneity are preferred, and about 98 to about 99% or more homogeneity most preferred, for pharmaceutical uses. Once purified, partially or to homogeneity as desired, the sterile antibodies may then be used therapeutically, as directed herein.

In view of the aforementioned discussion, the present application is further directed to an antibody obtainable by a process comprising the steps of culturing a host cell including, but not limited to a mammalian, plant, bacterial, transgenic animal, or transgenic plant cell which has been transformed by a polynucleotide or a vector comprising nucleic acid molecules encoding antibodies of the application so that the nucleic acid is expressed and, optionally, recovering the antibody from the host cell culture medium.

In certain aspects, the present application provides hybridoma cell lines, as well as disclosed herein the monoclonal antibodies produced by these hybridoma cell lines. The cell lines disclosed have uses other than for the production of the monoclonal antibodies. For example, the cell lines can be fused with other cells (such as suitably drug-marked human myeloma, mouse myeloma, human-mouse heteromyeloma or human lymphoblastoid cells) to produce additional hybridomas, and thus provide for the transfer of the genes encoding the monoclonal antibodies. In addition, the cell lines can be used as a source of nucleic acids encoding the anti-HER2 immunoglobulin chains, which can be isolated and expressed (e.g., upon transfer to other cells using any suitable technique (see e.g., Cabilly et al., U.S. Pat. No. 4,816,567; Winter, U.S. Pat. No. 5,225,539)). For instance, clones comprising a rearranged anti-HER2 light or heavy chain can be isolated (e.g., by PCR) or cDNA libraries can be prepared from mRNA isolated from the cell lines, and cDNA clones encoding an anti-HER2 immunoglobulin chain can be isolated. Thus, nucleic acids encoding the heavy and/or light chains of the antibodies or portions thereof can be obtained and used in accordance with recombinant DNA techniques for the production of the specific immunoglobulin, immunoglobulin chain, or variants thereof (e.g., humanized immunoglobulins) in a variety of host T-cells or in an in vitro translation system. For example, the nucleic acids, including cDNAs, or derivatives thereof encoding variants such as a humanized immunoglobulin or immunoglobulin chain, can be placed into suitable prokaryotic or eukaryotic vectors (e.g., expression vectors) and introduced into a suitable host T-cell by an appropriate method (e.g., transformation, transfection, electroporation, infection), such that the nucleic acid is operably linked to one or more expression control elements (e.g., in the vector or integrated into the host T-cell genome). For production, host T-cells can be maintained under conditions suitable for expression (e.g., in the presence of inducer, suitable media supplemented with appropriate salts, growth factors, antibiotic, nutritional supplements, etc.), whereby the encoded polypeptide is produced. If desired, the encoded protein can be recovered and/or isolated (e.g., from the host T-cells or medium). It will be appreciated that the method of production encompasses expression in a host T-cell of a transgenic animal (see e.g., WO 92/03918, GenPharm International, published Mar. 19, 1992).

Host cells can also be used to produce portions, or fragments, of intact antibodies, e.g., Fab fragments or scFv molecules by techniques that are conventional. For example, it may be desirable to transfect a host cell with DNA encoding either the light chain or the heavy chain of an antibody of this application. Recombinant DNA technology may also be used to remove some or all the DNA encoding either or both of the light and heavy chains that is not necessary for binding to human HER2 and Trop-2. The molecules expressed from such truncated DNA molecules are also encompassed by the antibodies of the application.

Methods for expression of single chain antibodies and/or refolding to an appropriate active form, including single chain antibodies, from bacteria such as *E. coli* have been described and are well-known and are applicable to the antibodies disclosed herein (see, e.g., Buchner et al., Anal. Biochem. 205:263-270, 1992; Pluckthun, Biotechnology 9:545, 1991; Huse et al., Science 246:1275, 1989 and Ward et al., Nature 341:544, 1989).

Often, functional heterologous proteins from *E. coli* or other bacteria are isolated from inclusion bodies and require solubilization using strong denaturants, and subsequent refolding. During the solubilization step, as is well known in the art, a reducing agent must be present to separate disulfide bonds. An exemplary buffer with a reducing agent is: 0.1 M Tris pH 8, 6 M guanidine, 2 mM EDTA, 0.3 M DTE (dithioerythritol). Reoxidation of the disulfide bonds can occur in the presence of low molecular weight thiol reagents in reduced and oxidized form, as described in Saxena et al., Biochemistry 9: 5015-5021, 1970, and especially as described by Buchner et al., supra.

Renaturation is typically accomplished by dilution (for example, 100-fold) of the denatured and reduced protein into refolding buffer. An exemplary buffer is 0.1 M Tris, pH 8.0, 0.5 M L-arginine, 8 mM oxidized glutathione (GSSG), and 2 mM EDTA.

As a modification to the two-chain antibody purification protocol, the heavy and light chain regions are separately solubilized and reduced and then combined in the refolding solution. An exemplary yield is obtained when these two proteins are mixed in a molar ratio such that a 5-fold molar excess of one protein over the other is not exceeded. Excess oxidized glutathione or other oxidizing low molecular weight compounds can be added to the refolding solution after the redox-shuffling is completed.

In addition to recombinant methods, the antibodies, labeled antibodies and antigen-binding fragments thereof that are disclosed herein can also be constructed in whole or in part using standard peptide synthesis. Solid phase synthesis of the polypeptides of less than about 50 amino acids in length can be accomplished by attaching the C-terminal amino acid of the sequence to an insoluble support followed by sequential addition of the remaining amino acids in the sequence. Techniques for solid phase synthesis are described by Barany & Merrifield, The Peptides: Analysis, Synthesis, Biology. Vol. 2: Special Methods in Peptide Synthesis, Part A. pp. 3-284; Merrifield et al., J. Am. Chem. Soc. 85:2149-2156, 1963, and Stewart et al., Solid Phase Peptide Synthesis, 2nd ed., Pierce Chem. Co., Rockford, Ill., 1984. Proteins of greater length may be synthesized by condensation of the amino and carboxyl termini of shorter fragments. Methods of forming peptide bonds by activation of a carboxyl terminal end (such as by the use of the coupling reagent N,N'-dicylohexylcarbodimide) are well known in the art.

The following examples are offered to more fully illustrate the application but are not construed as limiting the scope thereof. As detailed in the specific examples provided below, highly potent bispecific antibodies were generated by combining a Herceptin antibody or a Herceptin scFv with an anti-Trop-2 antibody or anti-Trop-2 scFv. A number of different bispecific antibody configurations were generated and tested. The unique bispecific antibodies provided exhibit better biological activities than any of the mono-specific anti-HER2 and mono-specific anti-Trop-2 antibodies tested. In many assays the bispecific antibodies also demonstrate a synergistic activity over mono-specific anti-HER2 antibodies. The bispecific anti-HER2/Trop-2 antibody when conjugated with a DM1 derivative cytotoxic agent demonstrated superior in vitro and in vivo anti-tumor activities. These data suggest that the bispecific antibodies may have therapeutic use, particularly as ADCs or as ADCC enhanced antibodies, for the treatment of cancers expressing HER2 and/or Trop-2 levels, including patients currently ineligible for treatment with trastuzumab, pertuzumab or T-DM1. In addition, the bispecific antibodies may have therapeutic use for the treatment of cancer patients that have failed existing anti-HER2 and/or anti-Trop-2 therapies.

### Example 1

### Bispecific Antibody Construction

A schematic representation of the anti-HER2/Trop-2 bispecific antibody formats described in this application are presented in FIG.s 1-5. These constructs were designed to simultaneously target HER2 and Trop-2 antigens. The scFv portion in all of the antibody formats was constructed by, e.g., linking the V_{H} domain using a linker (e.g., a synthetic flexible linker, e.g., a GS linker, e.g., a linker sequence set forth in SEQ ID NO: 25). The scFv was covalently linked to the N-terminus of heavy chain, and to the C-terminus of the CH3 domain via, e.g., a flexible linker, e.g., a GS linker, e.g., a linker sequence set forth in SEQ ID NO: 25.

To generate bispecific antibody HT2 (FIG. 1), the heavy chain (HC) was synthesized containing anti-HER2 antibody Herceptin scFv (SEQ ID NO: 3), anti-Trop-2 antibody A1,2X4 V_{H} (SEQ ID NO: 4), and IgG1 constant (CH) regions was synthesized. The scFv and V_{H} was linked via GS linker. The resulting scFv-V_{H}-CH fusion was cloned into pUC57 expression vector. The light chain (LC) containing anti-Trop-2 antibody A1,2X4 V_{L} (SEQ ID NO: 5) and CL regions was also synthesized and cloned into pUC57 expression vector. The HC and LC were then co-transfected into an expression vector. The resulting bispecific antibody is composed of Herceptin as scFv and anti-Trop2 antibody A1,2X4 as Fab, in which Herceptin scFv is orientated as VK-linker-V_{H} when it's fused to the N-terminus of both heavy chains of anti-Trop-2 antibody.

To generate bispecific antibody TH2 (FIG. 2), the HC was synthesized containing anti-Trop-2 antibody A1,2X4 scFv (SEQ ID NO: 6) and anti-HER2 antibody Herceptin V_{H} (SEQ ID NO: 7) and IgG1 CH regions was synthesized. The scFv and V_{H} was linked via a GS linker. The resulting scF_{V}-V_{H}-CH fusion was cloned into pUC57 expression vector. The LC containing anti-HER2 antibody Herceptin V_{L} (SEQ ID NO: 8) and CL (SEQ ID NO: 13) regions was also synthesized and cloned into pUC57 expression vector. The HC and LC were then co-transfected into an expression vector. The resulting bispecific antibody is composed of anti-Trop-2 antibody A1,2X4 as scFv and Herceptin as Fab, in which anti-Trop-2 scFv is orientated as VK-linker-V_{H} when it's fused to the N-terminus of both heavy chains of Herceptin.

To generate bispecific antibody HB1 (FIG. 3), the HC was synthesized containing anti-HER2 antibody Herceptin V_{H} (SEQ ID NO: 7) and IgG1 CH regions and anti-Trop-2 antibody hRS7 scFv (SEQ ID NO: 9) was synthesized. The V_{H} and scFv was linked via a GS linker. The resulting V_{H}-CH-scFv fusion was cloned into pUC57 expression vector. The LC containing anti-HER2 antibody Herceptin V_{L} (SEQ ID NO: 8) and CL regions was also synthesized and cloned into pUC57 expression vector. The HC and LC were then co-transfected into an expression vector. The resulting bispecific antibody is composed of Herceptin as Fab and anti-Trop-2 antibody hRS7 as scFv, in which anti-Trop-2 scFv is orientated as VK-linker-CH when it's fused to the C-terminus of both heavy chains of Herceptin.

To generate bispecific antibody BH1 (FIG. 4), the HC was synthesized containing anti-HER2 antibody Herceptin scFv (SEQ ID NO: 9) and IgG1 constant region was synthesized. The scFv and V_{H} was linked via a GS linker. The resulting scF_{V}-V_{H}-CH fusion was cloned into pUC57 expression vector. The LC containing anti-Trop-2 antibody hRS7 V_{L} (SEQ ID NO: 11) and CL regions was also synthesized and cloned into pUC57 expression vector. The HC and LC were then co-transfected into an expression vector. The resulting bispecific antibody is composed of Herceptin as scFv and anti-Trop-2 antibody hRS7 as Fab, in which anti-Herceptin scFv is orientated as VK-linker-V_{H} when it's fused to the C-terminus of both heavy chains of hRS7 antibody.

To generate bispecific antibody BH2 (FIG. 5), the heavy chain (HC) was synthesized containing anti-HER2 antibody Herceptin scFv (SEQ ID NO: 3) and anti-Trop-2 antibody hRS7 V_{H} (SEQ ID NO: 10) and IgG1 CH region was synthesized. The scFv and V_{H} was linked via a GS linker. The resulting scF_{V}-V_{H}-CH fusion was cloned into pUC57 expression vector. The LC containing anti-Trop-2 antibody hRS7 V_{L} (SEQ ID NO: 11) and CL regions was also synthesized and cloned into pUC57 expression vector. The HC and LC were then co-transfected into an expression vector. The resulting bispecific antibody is composed of Herceptin as scFv and anti-Trop2 antibody hRS7 as Fab, in which Herceptin scFv is orientated as VK-linker-V_{H} when it's fused to the N-terminus of both heavy chains of hRS7 antibody.

All antibody constructs were transiently expressed in HEK293F cells, which resulted in expression levels of 1.08 mg/mL for HT2(HT002), 0.55 mg/mL for TH2(TH002), 0.695 mg/mL for HB1(HT001a). Among those, the expression level of HT2 (HT002) is particularly advantageous.

All bispecific antibodies were purified to homogeneity (> 90%) by standard protein A purification and analyzed by reducing and nonreducing SDS-PAGE.

### Example 2

### Binding Affinity of Bispecific Antibodies

The binding affinity of the purified anti-HER2/Trop-2 bispecific antibodies were determined by FACS analysis. Prostate cancer cells LNCap with HER2 but no Trop-2 expression, breast cancer cells MDA-MB-468 with Trop-2 but no HER2 expression, and colon cancer cells Colo205 that express both HER2 and Trop-2 were harvested and suspended in FACS buffer at 0.5 - 1 x 10⁶ cells/ml. Anti-HER2 antibody Herceptin, anti-Trop-2 antibody A1,2X4, bispecific anti-HER2/Trop-2 antibodies HT2(HT002) and TH2(TH001) at 1.25, 2.5, 5, 10, 20, 40 g/ml were then added to cells and incubated for 30 min at 4°C. After incubation, the cells are washed to remove unbound antibody. The cells are then dissociated and stained with fluorescent-conjugated secondary antibody at 20 g/ml for 30 minutes in dark on ice. After incubation, the cells are washed and resuspended in staining buffer before being analyzed using backman flow cytometry system (BD Accuri C6). The median flueorescent intensity (MFI) was analyzed by Beckman flow cytometric software. The equilibrium binding constant Kd was obtained using "one site-specific binding" analyzing model in GraphPad Prism 7. Kd is the radioligand concentration needed to achieve a half-maximum binding at equilibrium.

FIG. 6B depicts the results on bispecific anti-HER2/Trop-2 antibody HB1. In LNCap cells that express only Her2 and MDA-MB-468 cells that express only Trop-2 on cell surface, HB1 had showed less binding compared to the parental mono-specific antibody Herceptin or hRS7. In Colo205 cells that express both HER2 and Trop-2, HB1 had comparable binding affinity compared to the parental antibodies.

FIG. 6C depicts the results on bispecific anti-HER2/Trop-2 antibody BH1 (TH001a) and BH2 (TH001b). In LNCap cells that express only Her2 on cell surface, bispecific antibody TH001b had similar binding affinity as parental mono-specific antibody Herceptin, whereas bispecific antibody TH001a had moderate binding affinity compared to Herceptin. In MDA-MB-468 cells that express only Trop-2 on cell surface, TH001a showed better binding comparable to parental mono-specific antibody A1,2X4 than TH001b. In Colo205 cells that express both antigens, bispecific antibodies demonstrated comparable or better binding than parental mono-specific antibodies.

FIG. 6A depicts the results on bispecific anti-HER2/Trop-2 antibody HT2 (HT002) and TH2 (TH002). In LNCap cells that express only Her2 on cell surface, HT002 and TH002 bound to the cells with similar binding affinity as the parental mono-specific antibody Herceptin. However, both bispecific antibodies had moderate binding affinity to MDA-MB-468 cells that only express Trop-2 compared to mono-specifc antibody A1,2X4. Strikingly, in Colo205 cells that express both antigens, the amount of bound HT002 and TH002 bispecific antibody detected on the cell surface was increased dramatically compared to either mono-specific antibody alone. Such effects provide particular advantages for treating patients with cancer cells that both Trop2 positive and HER2 positive.

### Example 3

### Internalization of Bispecific Antibody

Internalization and degradation of bispecific anti-HER2/Trop-2 antibody was measured by flow cytometry. In this study, fluorescent-conjugated secondary antibodies were used to detect the primary antibodies left on after internalization.

HER2 low/Trop-2 low expressing colon cancer cells Colo205, HER2 negative/Trop-2 high expressing breast cancer cells MDA-MB-468, and HER2 high/Trop-2 medium expressing ovarian cancer cells SK-BR-3 were seeded in 1×10⁵ cell/well. Next, either bispecific antibodies or parental mono-specific antibodies was added to cells in FACS buffer at concentrations of 400, 1000, 3000, or 10000 ng/ml, respectively, and incubated for 30 min at 4°C. After incubation, the cells were washed three times with FACS buffer to removed unbound antibody. The cells were then incubated at 37°C with 5% CO₂ for antibody internalization. After 2 hrs, the cells were dissociated with Trypsin and stained with fluorescent-conjugated goat anti-human IgG secondary antibodies for 30 min on ice in dark before being analyzed using Beckman flow cytometry system. The fluorescent intensity and cell binding percentage at each time point were analyzed by Beckman flow cytometric software. The amount of antibodies internalized into cells at each concentration was determined using the following formula: (MFI₄°_{C} - MFI₃₇°_{C)} x 10³

FIG. 7 depicts the results from the study. The results indicate that bispecific anti-HER2/Trop-2 antibody HT2 (HT002), BH1 (TH001a), and BH2 (TH001b) of the application can be internalized by cells expressing HER2 and /or Trop-2. The amount of bispecific antibodies internalized was similar to that of parental anti-Trop-2 antibody A1,2X4 or hRS7 and anti-HER2 antibody Herceptin in MDA-MB-468 cells expressing Trop-2 but none HER2 level and the Her2 low and Trop-2 low expressing Colo205 cells. In the SK-BR-3 cells that express both HER2 and Trop-2 at medium or high levels, the bispecific antibodies HT2 and BH2 induces an enhanced internalization than parental mono-specific antibodies, whereas BH1 had similar internalization compared to mono-specific antibodies. The amount of internalization on HT2 and BH2 was more than the sum of mono-specific antibody Herceptin and A1,2X4. These results demonstrate the particular advantages of using HT2 or BH2 for treating patients who have cancer cells that express medium or high levels of HER2 and/or Trops.

### Example 4

### Generation of Bispecific Antibody Drug Conjugates (ADCs)

The constructs described herein include anti-HER2/Trop-2 bispecific antibodies, including HT2(HT002) and HB1(HT001a), conjugated to BI-P203, DM1, or MMAE to form ADCs and evaluated for their ability to inhibit the growth of multiple cancer cell lines expressing different levels of HER2 and Trop-2.

Some embodiments of the application relate to BI-P203 or DM1 or MMAE linked to bispecific antibodies via a non-cleavable linker. In this example, the bispecific anti-HER2/Trop-2 antibodies HT2 (HT002) or HB1 (HT001a) was reduced by adding antibody to TCEP (Tris (2-carboxyethyl) phosphine) dissolved in a pH-adjusted PBS EDTA buffer. The antibody/TCEP solution was incubated at 37°C for 2-3 hrs. The reduced antibodies were buffer exchanged into conjugation reaction buffer. The BI-P203, DM1, or MMAE payload dissolved in DMSO was added to a solution of reduced anti-Trop-2 monoclonal antibody at payload/antibody ratio of 7 - 30:1 in order to achieve different drug to antibody ratios (DARs). The payload/antibody solution was incubated for 1-2 hr at 20°C while the reduced antibody was conjugated to BI-P203 payload via the maleimide group. After conjugation was completed, the reaction mixture was desalted and concentrated to yield anti-HER2/Trop-2-BI-P203 ADCs. The biochemical properties of the resulting ADCs were characterized using size-exclusion chromatography high pressure liquid chromatography (SEC-HPLC) to determine purity and aggregation content, and by using hydrophobic interaction chromatography HPLC (HIC-HPLC) to confirm drug loading (DAR). The conjugation procedure is illustrated in FIG. 8. The final ADC products are comprised of four or six or seven BI-P203 and DM1-linker molecules, and two or four MMAE-linker molecules. The cysteine conjugation method used in the conjugation process produces more homogenouse ADCs compared to lysine conjugation method.

### Example 5

### In Vitro Activity of Bispecific ADCs

ADCs of the application containing cytotoxic payloads may be used to kill target cells, such as killing cancer cells. HT2-BI-P203, HT2-MMAE, and HB1-DM1 ADCs were tested in the *in vitro* cytoxicity assay. Naked HT2 antibody was also included in the assay. For HT2-BI-P203 *in vitro* assay, BxPC-3 pancreatic cancer cells, N87 gastric cancer cells, MDA-MB-468 breast cancer cells, and A549 lung cancer cells were used. BxPC-3 cells have low HER2 and high Trop-2 expression, NCI-N87 cells have high HER2 and medium Trop-2 expression, MDA-MB-468 cells express no HER2 but medium levels of Trop-2, and A549 cells had undetectable levels of HER2 and Trop-2. All cell lines were cultured in a suitable culture medium at 37°C in a humidified incubator atmosphere of 5% CO₂. Cells were plated in 96-well flat bottom plates. Cell seeding number ranged from 500 cells/100 ul/ well to 6,000 cells/100 µl/well. Cells were allowed to adhere overnight at 37°C in a humidified atmosphere of 5% CO₂. HT2-BI-P203 ADC or HT2 antibody were prepared from stock solution and diluted into appropriated working concentration 24 hr after cell seeding. A serial ten-fold dilution for seven points was performed with culture medium. The final concentrations were ranging from 1000 nM to 0.001 nM. The cells were incubated with ADCs for 72 hours. Cell Counting Kit-8 solution (Dojindo China Co., Ltd, lot#PL701) was added to the wells for 1-4 hr at 37°C and the absorbance at 450 nm was measured using a Microplate Reader (SpectraMax M5, Molecular Devices) and SoftMax Pro5.4.1 software. The percent of inhibition was calculated by the following formula: (average absorbance of treated samples/average absorbance of control samples) × 100. For all cellular assays, dose-response curves were generated using GraphPad Prism 7 three-parameter curve fitting.

Table 2 summarizes the HER2 and Trop-2 expression levels in BxPC-3, NCI-N87, MDA-MB-468, and A549 cells and IC50 for each article tested. The killing curves were shown in FIG. 9A. The results demonstrate that the anti-HER2/Trop-2 bispecific antibody HT2-BI-P203 effectively killed all cancer cells that express different levels of HER2 and Trop-2. In the HER2- and Trop-2 negative A549 cells, IC50 could not be calculated with all HT2-BI-P203, indicating no specific killing activity was achieved. Naked HT2 antibody had no specific killing activity in all cell lines tested.

**Table 2**

| **Cell Line** | **HER2 Expression (MFI)** | **Trop-2 Expression (MFI)** | **HT2-BI-P203 IC50 (nM)** | **HT2 IC50 (nM)** |
|---|---|---|---|---|
| BxPC-3 | 1112 | 161823 | 0.092 | - |
| NCI-N87 | 82800 | 40400 | 9.019 | - |
| MDA-MB-468 | 164 | 74943 | 14.06 | - |
| A549 | 609 | -61 | - | - |

For HT2-MMAE *in vitro* assay, MDA-MB-468 breast cancer cells, LNCaP prostate cancer cells, SK-BR-3 ovarian cancer cells, and A549 lung cancer cells were used. HT2(HT002)-MMAE was tested head-to-head with the parental mono-specific antibodies Herceptin- and A1,2X4-MMAE ADCs were. *In vitro* cytotoxicity assay was performed according to the procedures described above.

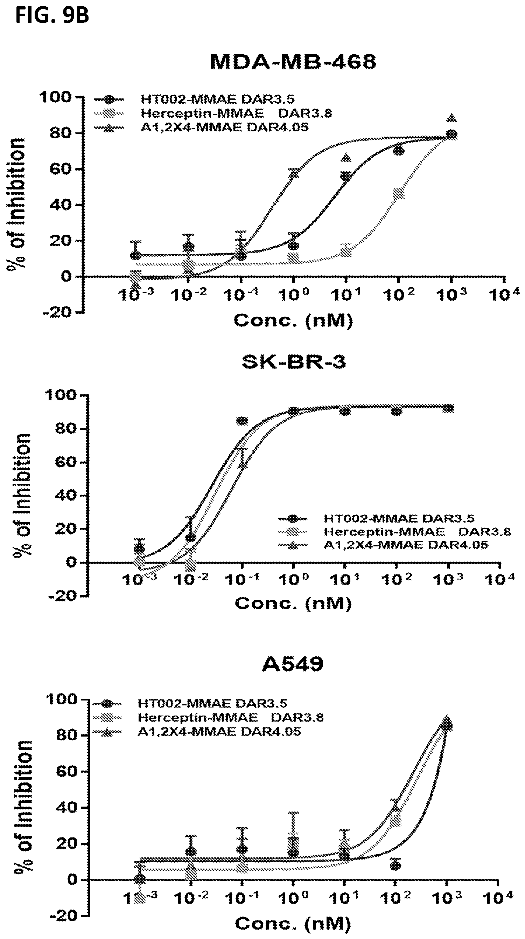

Table 3 summarizes the HER2 and Trop-2 expression levels in MDA-MB-468, SK-BR-3, and A549 cells and IC50 for each article tested. The killing curves were shown in FIG. 9B. In MDA-MB-468 cells that express only Trop-2, Herceptin-MMAE showed no specific killing, while HT002-MMAE had weaker cytotoixity compared to A1,2X4-MMAE. All three ADCs were potent against SK-BR-3 cells that express both HER2 and Trop-2.

The results demonstrate that the anti-HER2/Trop-2 bispecific antibody HT2-BI-P203 effectively killed all cancer cells that express different levels of HER2 and Trop-2. In the HER2- and Trop-2 negative A549 cells, IC50 could not be calculated with all HT2-BI-P203, indicating no specific killing activity was achieved. Naked HT2 antibody had no specific killing activity in all cell lines tested. In the HER2- and Trop-2 negative A549 cells, IC50 was above 200 nM or could not be calculated with all three ADCs, indicating no specific killing activity was achieved.

**Table 3**

| **Cell Line** | **HER2 Expression (MFI)** | **Trop-2 Expression (MFI)** | **HT002-MMAE IC50 (nM)** | **Herceptin-MMAE IC50 (nM)** | **HT002-MMAE IC50 (nM)** |
|---|---|---|---|---|---|
| MDA-MB-468 | ND | 39936 | 6.122 | 102.9 | 0.389 |
| SK-BR-3 | 143427 | 54714 | 0.0263 | 0.0301 | 0.06294 |
| A549 | 1673 | 139 | - | 255.3 | 216.2 |

For HB1-DM1 *in vitro* assay, BxPC-3 pancreatic cancer cells, MDA-MB-468 breast cancer cells, LNCaP prostate cancer cells, and SK-BR-3 ovarian cancer cells were used. MDA-MB-468 cells express only Trop-2, LnCaP cells express only HER2, and SK-BR-3 cells have both HER2 and Trop-2 expression. *In vitro* cytotoxicity assay was performed according to the procedures described above.

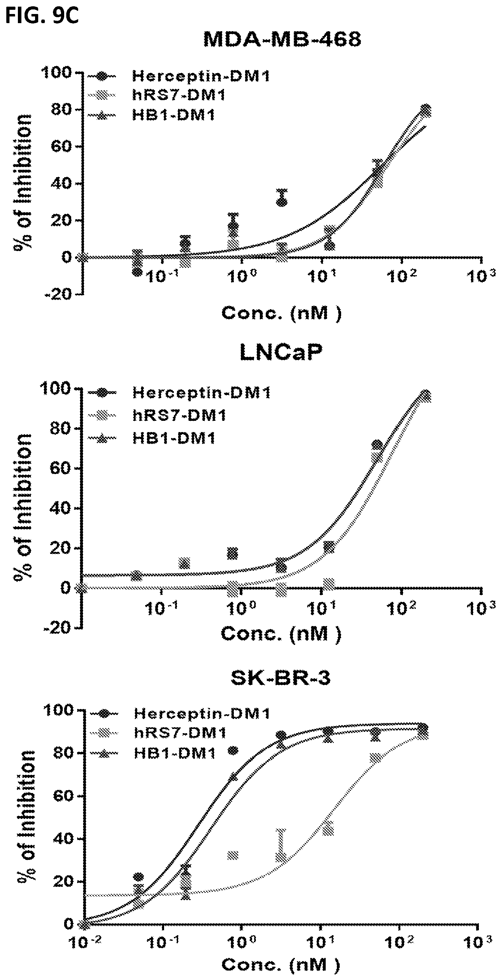

Table 4 summarizes the IC50 for HB1-DM1 in each cell lines. The killing curves were shown in FIG. 9C. HB1-DM1 ADC was most potent against SK-BR-3 cells generating IC50 values less than 1 nM.

**Table 4**

| **Cell Line** | **HB1-DM1 IC50 (nM)** |
|---|---|
| MDA-MB-468 | 57.31 |
| LNCaP | 50.32 |
| SK-BR-3 | 0.4106 |

### Example 6

### In Vivo Activity of Bispecific ADCs

The anti-tumor activities of anti-HER2/Trop-2 bispecific antibody HT002-BI-P203 ADC was assessed using Trop-2-positive / Her2-negative MDA-MB-468 and Trop-2-positive / Her2-positive NCI-N87 xenograft models in mouse subjects. Five million BxPC3 or NCI-N87 cells were harvested from culture flasks and implanted subcutaneously into the right flank of 6- to 7-week-old BALB/c nude mice. Tumors were measured twice a week throughout the course of the experiments, with tumor volume calculated using the following formula: tumor volume (mm³) = (length x width²)/2.

The ADCs tested in BxPC3 model were conjugated with different DARs: A1,2X4-BI-P203 had DAR of 7.2, T-DM1 had DAR of 3.2, and HT002-BI-P203 had DAR of 5.2. ADCs were administered intravenously as a single bolus dose.

FIG. 10A shows the results of the *in vivo* BxPC-3 xenograft study. As shown, single administration of bispecific ADC of the application at 3 mg/kg induced durable stable disease, whereas 3 mg/kg dose of mono-specific antibody Trop-2 ADC or T-DM1 resulted in tumor growth inhibition. There was no significant body weight change with all ADC treatment compared to vehicle control and IgG isotype ADC control as shown in FIG. 10B.

To further assess the antitumor activities of bispecific ADC, HT002-BI-P203 was tested in the N87 xenograft model at two dose levels of 1.5 and 5 mg/kg. The ADC was administered intravenously as a single bolus dose.

FIG. 11A depicts the results of the *in vivo* N87 xenograft study. The result indicates that the bispecific ADC was able to achieve durable stable disease with a single administration of a 5 mg/kg dose. As shown in FIG. 11B, there was no significant body weight change with all ADC treatment compared to vehicle control and IgG isotype ADC control.

All of the articles and methods disclosed and claimed herein can be made and executed without undue experimentation in light of the present disclosure. All patents, patent applications, and publications mentioned in the specification are indicative of the levels of those of ordinary skill in the art to which the application pertains.

### Example 7

HT002-MMAE (DAR 3.5) were tested against T-DM1 in BxPC-3 xenograft model. The unconjugated bispecific antibody HT002 was also included. Testing articles were administered intravenously on day 0 and day 7.

FIG. 12 shows the results of the in vivo BxPC-3 xenograft study. As shown, the administration of bispecific ADC HT002 of the application at 5 mg/kg resulted in tumor regression which was durable through the end of the study (Day 43), whereas 5 mg/kg dose of T-DM1 induced tumor growth inhibition. Tumor growth inhibition was also observed with the administration of the unconjugated bispecific antibody HT002.

### Example 8

The bispecific antibody ADC, HT002-MMAE, along with the parental antibody ADCs, A1,2X4-MMAE and Herceptin-MMAE, were tested in the *in vitro* cytotoxicity assay in JIMT-1 breast cancer cells. Unconjugated HT002 antibody and Herceptin were also included in the assay. JIMT-1 cells were derived from a patient with Herceptin-resistant breast cancer, and express moderate to high levels of Trop-2 and HER2. The killing curves were shown in FIG. 13. The results demonstrates that the bispecific antibody ADC HT002-MMAE effectively inhibited JIMT-1 cell growth with an IC50 value of 0.4301 nM, similar to the two parental antibody ADCs which had IC50 values of 0.3874 and 0.5043 nM, respectively. The unconjugated bispecific antibody HT002 alone was able to inhibit JIMT-1 cell growth with an IC50 value of 0.1853 nM. As expected, Herceptin had minimal antiproliferative activity in this cell line.

**SEQUENCE TABLE**

| SEQ ID NO | Description | Exemplary Sequences |
|---|---|---|
| 1 | human HER2 amino acid sequence | MKLRLPASPETHLDMLRHLYQGCQVVQGNLELTYLPTNASLSFLQ DIQEVQGYVLIAHNQVRQVPLQRLRIVRGTQLFEDNYALAVLDNGD PLNNTTPVTGASPGGLRELQLRSLTEILKGGVLIQRNPQLCYQDTIL WKDIFHKNNQLALTLIDTNRSRACHPCSPMCKGSRCWGESSEDCQ SLTRTVCAGGCARCKGPLPTDCCHEQCAAGCTGPKHSDCLACLHF NHSGICELHCPALVTYNTDTFESMPNPEGRYTFGASCVTACPYNYL STDVGSCTLVCPLHNQEVTAEDGTQRCEKCSKPCARVCYGLGMEH LREVRAVTSANIQEFAGCKKIFGSLAFLPESFDGDPASNTAPLQPEQ LQVFETLEEITGYLYISAWPDSLPDLSVFQNLQVIRGRILHNGAYSL TLQGLGISWLGLRSLRELGSGLALIHHNTHLCFVHTVPWDQLFRNP HQALLHTANRPEDECVGEGLACHQLCARGHCWGPGPTQCVNCSQ FLRGQECVEECRVLQGLPREYVNARHCLPCHPECQPQNGSVTCFGP EADQCVACAHYKDPPFCVARCPSGVKPDLSYMPIWKFPDEEGACQ PCPINCTHSCVDLDDKGCPAEQRASPLTSIISAVVGILLVVVLGVVFG ILIKRRQQKIRKYTMRRLLQETELVEPLTPSGAMPNQAQMRILKETE LRKVKVLGSGAFGTVYKGIWIPDGENVKIPVAIKVLRENTSPKANKE ILDEAYVMAGVGSPYVSRLLGICLTSTVQLVTQLMPYGCLLDHVR ENRGRLGSQDLLNWCMQIAKGMSYLEDVRLVHRDLAARNVLVKSP NHVKITDFGLARLLDIDETEYHADGGKVPIKWMALESILRRRFTHQ SDVWSYGVTVWELMTFGAKPYDGIPAREIPDLLEKGERLPQPPICTI DVYMIMVKCWMIDSECRPRFRELVSEFSRMARDPQRFVVIQNEDL GPASPLDSTFYRSLLEDDDMGDLVDAEEYLVPQQGFFCPDPAPGA GGMVHHRHRSSSTRSGGGDLTLGLEPSEEEAPRSPLAPSEGAGSDVF DGDLGMGAAKGLQSLPTHDPSPLQRYSEDPTVPLPSETDGYVAPLT CSPQPEYVNQPDVRPQPPSPREGPLPAARPAGATLERPKTLSPGKN GVVKDVFAFGGAVENPEYLTPQGGAAPQPHPPPAFSPAFDNLYYW DQDPPERGAPPSTFKGTPTAENPEYLGLDVPV |
| 2 | human TROP-2 amino acid sequence | MARGPGLAPPPLRLPLLLLVLAAVTGHTAAQDNCTCPTNKMTV CSPDGPGGRCQCALGSGMAVDCSTLTSKCLLLKARMSAPKNAR TLVRPSEHALVDNDGLYDPDCDPEGRFKARQCNQTSVCWCVN SVGVRRTDKGDLSLRCDELVRTHHILIDLRHRPTAGAFNHSDLD AELRRLFRERYRLHPKFVAAVHYEQPTIQIELRQNTSQKAAGDV DIGDAAYYFERDIKGESLFQGRGGLDLRVRGEPLQVERTLIYYL DEIPPKFSMKRLTAGLIAVIVVVVVALVAGMAVLVITNRRKS GKYKKVEIKELGELRKEPSL* |
| 3 | Human Herceptin scFv | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKGGGGSGGGGSGGGGSEVQLVESGGGLVQPGG SLRLSCAASGFNIKDTYIHWVRQAPGKGLEWVARIYPTNGYTRYA DSVKGRFTISADTSKNTAYLQMNSLRAEDTAVYYCSRWGGDGFY AMDYWGQGTLVTVSS |
| 4 | A1,2X4 V_{H} | QVQLQESGPGLVKPSETLSLTCTVSGFSLTSYGVNWIRQPPGKGLE WIGVMWAGGSTNYNSALMSRLTISKDTSKNQFSLKLSSVTAADTA VYYCARDENWDGAWFAYWGQGTLVTVSS |
| 5 | A1,2X4 V_{L} | DIVMTQSPDSLAVSLGERATINCKSSQSLLNSGTRKNYLAWYQQKP GQPPKLLISWASSRESGVPDRFSGSGSGTDFTLTISSLQAEDVAVYY CKQSYNLFTFGGGTKVEIK |
| 6 | A1,2X4 scFv | DIVMTQSPDSLAVSLGERATINCKSSQSLLNSGTRKNYLAWYQQKP GQPPKLLISWASSRESGVPDRFSGSGSGTDFTLTISSLQAEDVAVYY CKQSYNLFTFGGGTKVEIKGGGGSGGGGSGGGGSQVQLQESGPGL VKPSETLSLTCTVSGFSLTSYGVNWIRQPPGKGLEWIGVMWAGGS TNYNSALMSRLTISKDTSKNQFSLKLSSVTAADTAVYYCARDENW DGAWFAYWGQGTLVTVSS |
| 7 | Herceptin V_{H} | |
| 8 | Herceptin V_{L} | |
| 9 | hRS7 scFv | DIQLTQSPSSLSASVGDRVSITCKASQDVSIAVAWYQQKPGKAPKL LIYSASYRYTGVPDRFSGSGSGTDFTLTISSLQPEDFAVYYCQQHYI TPLTFGAGTKVDIKGGGGSGGGGSGGGGSQVQLQQSGSELKKPGA SVKVSCKASGYTFTNYGMNWVKQAPGQGLKWMGWINTYTGEPT YTDDFKGRFAFSLDTSVSTAYLQISSLKADDTAVYFCARGGFGSSY WYFDVWGQGSLVTVSS |
| 10 | hRS7 V_{H} | QVQLQQSGSELKKPGASVKVSCKASGYTFTNYGMNWVKQAPGQG LKWMGWINTYTGEPTYTDDFKGRFAFSLDTSVSTAYLQISSLKAD DTAVYFCARGGFGSSYWYFDVWGQGSLVTVSS |
| 11 | hRS7 V_{L} | DIQLTQSPSSLSASVGDRVSITCKASQDVSIAVAWYQQKPGKAPKL LIYSASYRYTGVPDRFSGSGSGTDFTLTISSLQPEDFAVYYCQQHYI TPLTFGAGTKVDIK |
| 12 | IgG1 constant region | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKRVEPKSCDKTHTCPPCPAPELLGGPSVFLFPPKPKDTLMISRTP EVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYK TTPPVLDSDGSFFLYSKLTVDKSRWQQGNVFSCSVMHEALHNHYT QKSLSLSPGK |
| 13 | IgG1 constant region | |
| 14 | bispecific anti-HER2/Trop-2 antibody HT2 heavy chain amino acid sequence | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKGGGGSGGGGSGGGGSEVQLVESGGGLVQPGG SLRLSCAASGFNIKDTYIHWVRQAPGKGLEWVARIYPTNGYTRYA DSVKGRFTISADTSKNTAYLQMNSLRAEDTAVYYCSRWGGDGFY AMDYWGQGTLVTVSSGGGGSGGGGSGGGSQVQLQESGPGLVKPS ETLSLTCTVSGFSLTSYGVNWIRQPPGKGLEWIGVMWAGGSTNYN SALMSRLTISKDTSKNQFSLKLSSVTAADTAVYYCARDENWDGAW FAYWGQGTLVTVSSASTKGPSVFPLAPSSKSTSGGTAALGCLVKDY FPEPVTVSWNSGALTSGVHTFPAVLQSSGLYSLSSVVTVPSSSLGTQ TYICNVNHKPSNTKVDKRVEPKSCDKTHTCPPCPAPELLGGPSVFL FPPKPKDTLMISRTPEVTCVVVDVSHEDPEVKFNWYVDGVEVHNA KTKPREEQYNSTYRVVSVLTVLHQDWLNGKEYKCKVSNKALPAPIE KTISKAKGQPREPQVYTLPPSREEMTKNQVSLTCLVKGFYPSDIAV EWESNGQPENNYKTTPPVLDSDGSFFLYSKLTVDKSRWQQGNVFS CSVMHEALHNHYTQKSLSLSPGK |
| 15 | bispecific anti-HER2/Trop-2 antibody HT2 light chain amino acid sequence | DIVMTQSPDSLAVSLGERATINCKSSQSLLNSGTRKNYLAWYQQKP GQPPKLLISWASSRESGVPDRFSGSGSGTDFTLTISSLQAEDVAVYY CKQSYNLFTFGGGTKVEIKRTVAAPSVFIFPPSDEQLKSGTASVVCL LNNFYPREAKVQWKVDNALQSGNSQESVTEQDSKDSTYSLSSTLT LSKADYEKHKVYACEVTHQGLSSPVTKSFNRGEC |
| 16 | bispecific anti-HER2/Trop-2 antibody TH2 heavy chain amino acid sequence | DIVMTQSPDSLAVSLGERATINCKSSQSLLNSGTRKNYLAWYQQKP GQPPKLLISWASSRESGVPDRFSGSGSGTDFTLTISSLQAEDVAVYY CKQSYNLFTFGGGTKVEIKGGGGSGGGGSGGGGSQVQLQESGPGL VKPSETLSLTCTVSGFSLTSYGVNWIRQPPGKGLEWIGVMWAGGS TNYNSALMSRLTISKDTSKNQFSLKLSSVTAADTAVYYCARDENW DGAWFAYWGQGTLVTVSSGGGGSGGGGSGGGSEVQLVESGGGL VQPGGSLRLSCAASGFNIKDTYIHWVRQAPGKGLEWVARIYPTNG YTRYADSVKGRFTISADTSKNTAYLQMNSLRAEDTAVYYCSRWG GDGFYAMDYWGQGTLVTVSSASTKGPSVFPLAPSSKSTSGGTAAL GCLVKDYFPEPVTVSWNSGALTSGVHTFPAVLQSSGLYSLSSVVTVP SSSLGTQTYICNVNHKPSNTKVDKRVEPKSCDKTHTCPPCPAPELL GGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPSREEMTKNQVSLTCLVKGFY PSDIAVEWESNGQPENNYKTTPPVLDSDGSFFLYSKLTVDKSRWQQ GNVFSCSVMHEALHNHYTQKSLSLSPGK |
| 17 | bispecific anti- | DIQMTQSPSSLSASVGDRVTITCRASQDVNTAVAWYQQKPGKAPK LLIYSASFLYSGVPSRFSGSRSGTDFTLTISSLQPEDFATYYCQQHYT |

| SEQ ID NO | Description | Exemplary Sequences |
|---|---|---|
| | HER2/Trop-2 antibody TH2 light chain amino acid sequence | TPPTFGQGTKVEIKRTVAAPSVFIFPPSDEQLKSGTASVVCLLNNFY PREAKVQWKVDNALQSGNSQESVTEQDSKDSTYSLSSTLTLSKAD YEKHKVYACEVTHQGLSSPVTKSFNRGEC |
| 18 | bispecific anti-HER2/Trop-2 antibody HB1 heavy chain amino acid sequence | EVQLVESGGGLVQPGGSLRLSCAASGFNIKDTYIHWVRQAPGKGL EWVARIYPTNGYTRYADSVKGRFTISADTSKNTAYLQMNSLRAED TAVYYCSRWGGDGFYAMDYWGQGTLVTVSSASTKGPSVFPLAPS SKSTSGGTAALGCLVKDYFPEPVTVSWNSGALTSGVHTFPAVLQSS GLYSLSSVVTVPSSSLGTQTYICNVNHKPSNTKVDKRVEPKSCDKT HTCPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPSREEMTKN QVSLTCLVKGFYPSDIAVEWESNGQPENNYKTTPPVLDSDGSFFLYS KLTVDKSRWQQGNVFSCSVMHEALHNHYTQKSLSLSPGGGGGSG GGGSDIQLTQSPSSLSASVGDRVSITCKASQDVSIAVAWYQQKPGK APKLLIYSASYRYTGVPDRFSGSGSGTDFTLTISSLQPEDFAVYYCQ QHYITPLTFGAGTKVDIKGGGGSGGGGSGGGGSQVQLQQSGSELK KPGASVKVSCKASGYTFTNYGMNWVKQAPGQGLKWMGWINTYT GEPTYTDDFKGRFAFSLDTSVSTAYLQISSLKADDTAVYFCARGGF GSSYWYFDVWGQGSLVTVSS |
| 19 | bispecific anti-HER2/Trop-2 antibody HB1 light chain amino acid sequence | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKRTVAAPSVFIFPPSDEQLKSGTASVVCLLNNFY PREAKVQWKVDNALQSGNSQESVTEQDSKDSTYSLSSTLTLSKAD YEKHKVYACEVTHQGLSSPVTKSFNRGEC |
| 20 | bispecific anti-HER2/Trop-2 antibody BH1 heavy chain amino acid sequence | QVQLQQSGSELKKPGASVKVSCKASGYTFTNYGMNWVKQAPGQG LKWMGWINTYTGEPTYTDDFKGRFAFSLDTSVSTAYLQISSLKAD DTAVYFCARGGFGSSYWYFDVWGQGSLVTVSSASTKGPSVFPLAP SSKSTSGGTAALGCLVKDYFPEPVTVSWNSGALTSGV_{H}TFPAV_{L}QSS GLYSLSSVVTVPSSSLGTQTYICNVNHKPSNTKVDKRVEPKSCDKT HTCPPCPAPELLGGPSVFLFPPKPKDTLMISRTPEVTCVVVDVSHED PEVKFNWYVDGVEVIINAKTKPREEQYNSTYRVVSV_{L}TV_{L}HQDWL NGKEYKCKVSNKALPAPIEKTISKAKGQPREPQVYTLPPSREEMTK NQVSLTCLVKGFYPSDIAVEWESNGQPENWKTTPPV_{L}DSDGSFFLY SKLTVDKSRWQQGNVFSCSVMHEALHNHYTQKSLSLSPGGGGGS GGGGSDIQMTQSPSSISASVGDRVTTTCRASQDVNTAVAWYQQKP GKAPKLLIYSASFLYSGVPSRFSGSRSGTDFTLTISSLQPEDFATYYC QQHYTTPPTFGQGTKVEIKGGGGSGGGGSGGGGSEVQLVESGGGL VQPGGSLRLSCAASGFNIKDTYIHWVRQAPGKGLEWVARIYPTNG |

| SEQ ID NO | Description | Exemplary Sequences |
|---|---|---|
| | | YTRYADSVKGRFTISADTSKNTAYLQNSLRAEDTAVYYCSRWGGD GFYAMDYWGQGTLVTVSS |
| 21 | bispecific anti-HER2/Trop-2 antibody BH1 light chain amino acid sequence | DIQLTQSPSSLSASVGDRVSITCKASQDVSIAVAWYQQKPGKAPKL LIYSASYRYTGVPDRFSGSGSGTDFTLTISSLQPEDFAVYYCQQHYI TPLTFGAGTKVDIKRTVAAPSVFIFPPSDEQLKSGTASVVCLLNNFY PREAKVQWKVDNALQSGNSQESVTEQDSKDSTYSLSSTLTLSKAD YEKHKVYACEVTIIQGLSSPVTKSFNRGEC |
| 22 | bispecific anti-HER2/Trop-2 antibody BH2 heavy chain amino acid sequence | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKGGGGSGGGGSGGGGSEVQLVESGGGLVQPGG SLRLSCMSGFNIKDTYIHWVRQAPGKGLEWVARIYPTNGYTRYAD SVKGRFTISADTSKNTAYLQMNSLRAEDTAVYYCSRWGGDGFYA MDYWGQGTLVTVSSGGGGSGGGGSGGGSQVQLQQSGSELKKPGA SVKVSCKASGYTFTNYGMNWVKQAPGQGLWMGWINTYTGEPTY TDDFKGRFAFSLDTSVSTAYLQISSLKADDTAVYFCARGGFGSSYW YFDVWGQGSLVTVSSASTKGPSVFPLAPSSKSTSGGTAALGCLVKD YFPEPVTVSWNSGALTSGVHTFPAVLQSSGLYSLSSVVTVPSSSLGT QTYICWNHKPSNTKVDKRVEPKSCDKTHTCPPCPAPELLGGPSVFL FPPKPKDTLMISRTPEVTCVVVDVSHEDPEVKFNWYVDGVEVHNA KTKPREEQYNSTYRVVSVLTVLHQDWLNGKEYKCKVSNKALPAPT EKTTSKAKGQPREPQVYTLPPSREEMTKNQVSLTCLVKGFYPSDIA VEWESNGQPENNYKTTPPVLDSDGSFFLYSKLTVDKSRWQQGNVF SCSVMHEALRNHYTQKSLSLSPG |
| 23 | bispecific anti-HER2/Trop-2 antibody BH2 light chain amino acid sequence | DIQLTQSPSSLSASVGDRVSITCKASQDVSIAVAWYQQKPGKAPKL LIYSASYRYTGVPDRFSGSGSGTDFTLTISSLQPEDFAVYYCQQHYI TPLTFGAGTKVDIKRTVAAPSVFIFPPSDEQLKSGTASVVCLLNNFY PREAKVQWKVDNALQSGNSQESVTEQDSKDSTYSLSSTLTLSKAD YEKHKVYACEVTIIQGLSSPVTKSFNRGEC |
| 24 | Linker | GGGGSGGGGS |
| 25 | Linker | GGGGSGGGGSGGGS |
| 26 | A1,2X4 V_{H} CDR1 | SYGVN |
| 27 | A1,2X4 V_{H} CDR2 | VMWAGGSTNYNSALMS |
| 28 | A1,2X4 V_{H} CDR3 | DENWDGAWFAY |

| SEQ ID NO | Description | Exemplary Sequences |
|---|---|---|
| 29 | A1,2X4 V_{L} CDR1 | KSSQSLLNSGTRKNYLA |
| 30 | A1,2X4 V_{L} CDR2 | WASSRES |
| 31 | A1,2X4 V_{L} CDR3 | KQSYNLFT |
| 32 | Herceptin V_{H} CDR1 | DTYIH |
| 33 | Herceptin V_{H} CDR2 | RIYPTNGYTRYADSVKG |
| 34 | Herceptin V_{H} CDR3 | WGGDGFYAMDY |
| 35 | Herceptin V_{L} CDR1 | RASQDVNTAVA |
| 36 | Herceptin V_{L} CDR2 | SASFLYS |
| 37 | Herceptin V_{L} CDR3 | QQHYTTPPT |
| 38 | hRS7 V_{H} CDR1 | NYGMN |
| 39 | hRS7 V_{H} CDR2 | WINTYTGEPTYTDDFKG |
| 40 | hRS7 V_{H} CDR3 | GGFGSSYWYFDV |
| 41 | hRS7 V_{L} CDR1 | KASQDVSIAVA |
| 42 | hRS7 V_{L} CDR2 | SASYRYT |
| 43 | hRS7 V_{L} CDR3 | QQHYITPLT |
| 44 | Linker | GGGGS |
| 45 | Linker | GGGGSGGGGS |
| 46 | Linker | GGGGSGGGGSGGGGS |
| 47 | Linker | GGGGSGGGGSGGGGSGGGGS |
| 48 | Linker | (G)ₙ, n>=1 |
| 49 | Linker | (GS)ₙ, 8>=n>=1 |
| 50 | Linker | (GSGGS)ₙ, 8>=n>=1 |

| SEQ ID NO | Description | Exemplary Sequences |
|---|---|---|
| 51 | Linker | (GGGGS)ₙ, 8>=n>=1 |
| 52 | Linker | (GGGS)ₙ, 8>=n>=1 |
| 53 | Linker | (GGGGS)₆ |
| 54 | Linker | (GSTSGSGKPGSGEGS)ₙ 3>=n>=1 |
| 55 | Anti-Her2 V_{H} (alternative) | EVQLVESGGGLVQPGGSLRLSCAASGFNIKDTYIHWVRQAPGKGL EWVARIYPTNGYTRYADSVKGRFTISADTSKNTAYLQNSLRAEDT AVYYCSRWGGDGFYAMDYWGQGTLVTVSS |
| 56 | Anti-Her2 V_{H} (alternative) | EVQLVESGGGLVQPGGSLRLSCMSGFNIKDTYIHWVRQAPGKGLE WVARIYPTNGYTRYADSVKGRFTISADTSKNTAYLQMNSLRAEDT AVYYCSRWGGDGFYAMDYWGQGTLVTVSS |
| 57 | Anti-Her2 V_{L} (alternative) | DIQMTQSPSSISASVGDRVTTTCRASQDVNTAVAWYQQKPGKAPK LLIYSASFLYSGVPSRFSGSRSGTDFTLTISSLQPEDFATYYCQQHYT TPPTFGQGTKVEIK |
| 58 | hRS7 V_{H} (alternative) | QVQLQQSGSELKKPGASVKVSCKASGYTFTNYGMNWVKQAPGQG LWMGWINTYTGEPTYTDDFKGRFAFSLDTSVSTAYLQISSLKADDT AVYFCARGGFGSSYWYFDVWGQGSLVTVSS |
| 59 | TH2 Heavy chain (alternative) | DIVMTQSPDSLAVSLGERATINCKSSQSLLNSGTRKNYLAWYQQKP GQPPKLLISWASSRESGVPDRFSGSGSGTDFTLTISSLQAEDVAVYY CKQSYNLFTFGGGTKVEIKGGGGSGGGGSGGGGSQVQLQESGPGL VKPSETLSLTCTVSGFSLTSYGVNWIRQPPGKGLEWIGVMWAGGS TNYNSALMSRLTISKDTSKNQFSLKLSSVTAADTAVYYCARDENW DGAWFAYWGQGTLVTVSSGGGGSGGGGSGGGGSGGGGSGGGGS GGGGSEVQLVESGGGLVQPGGSLRLSCAASGFNIKDTYIHWVRQA PGKGLEWVARIYPTNGYTRYADSVKGRFTISADTSKNTAYLQMNS LRAEDTAVYYCSRWGGDGFYAMDYWGQGTLVTVSSASTKGPSVF PLAPSSKSTSGGTAALGCLVKDYFPEPVTVSWNSGALTSGVHTFPA VLQSSGLYSLSSVVTVPSSSLGTQTYICNVNHKPSNTKVDKRVEPKS CDKTHTCPPCPAPELLGGPSVFLFPPKPKDTLMISRTPEVTCVVVDV SHEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 60 | HT2 heavy chain (alternative) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKGGGGSGGGGSGGGGSEVQLVESGGGLVQPGG SLRLSCAASGFNIKDTYIHWVRQAPGKGLEWVARIYPTNGYTRYA DSVKGRFTISADTSKNTAYLQMNSLRAEDTAVYYCSRWGGDGFY AMDYWGQGTLVTVSSGGGGSGGGGSGGGGSGGGGSGGGGSGGG GSQVQLQESGPGLVKPSETLSLTCTVSGFSLTSYGVNWIRQPPGKG LEWIGVMWAGGSTNYNSALMSRLTISKDTSKNQFSLKLSSVTAAD TAVYYCARDENWDGAWFAYWGQGTLVTVSSASTKGPSVFPLAPS SKSTSGGTAALGCLVKDYFPEPVTVSWNSGALTSGVHTFPAVLQSS |

| SEQ ID NO | Description | Exemplary Sequences |
|---|---|---|
| | | GLYSLSSVVTVPSSSLGTQTYICNVNHKPSNTKVDKRVEPKSCDKT HTCPPCPAPELLGGPSVFLFPPKPKDTLMISRTPEVTCVVVDVSHED PEVKFNWYVDGVEV_{H}NAKTKPREEQYNSTYRVVSV_{L}TV_{L}HQDWLN GKEYKCKVSNKALPAPIEKTISKAKGQPREPQVYTLPPSREEMTKN QVSLTCLVKGFYPSDIAVEWESNGQPENNYKTTPPV_{L}DSDGSFFLYS KLTVDKSRWQQGNVFSCSVMHEALHNHYTQKSLSLSPGK |
| 61 | TH2 Heavy chain (alternative) | DIVMTQSPDSLAVSLGERATINCKSSQSLLNSGTRKNYLAWYQQKP GQPPKLLISWASSRESGVPDRFSGSGSGTDFTLTISSLQAEDVAVYY CKQSYNLFTFGGGTKVEIKGGGGSGGGGSGGGGSGGGGSGGGGS GGGGSQVQLQESGPGLVKPSETLSLTCTVSGFSLTSYGVNWIRQPP GKGLEWIGVMWAGGSTNYNSALMSRLTISKDTSKNQFSLKLSSVT AADTAVYYCARDENWDGAWFAYWGQGTLVTVSSGGGGSGGGGS GGGGSGGGGSGGGGSGGGGSEVQLVESGGGLVQPGGSLRLSCAAS GFNIKDTYIHWVRQAPGKGLEWVARIYPTNGYTRYADSVKGRFTIS ADTSKNTAYLQMNSLRAEDTAVYYCSRWGGDGFYAMDYWGQGT LVTVSSASTKGPSVFPLAPSSKSTSGGTAALGCLVKDYFPEPVTVS WNSGALTSGVHTFPAVLQSSGLYSLSSVVTVPSSSLGTQTYICNVNH KPSNTKVDKRVEPKSCDKTHTCPPCPAPELLGGPSVFLFPPKPKDTL MISRTPEVTCVVVDVSHEDPEVKFNWYVDGVEVHNAKTKPREEQY NSTYRVVSVLTVLHQDWLNGKEYKCKVSNKALPAPIEKTISKAKGQ PREPQVYTLPPSREEMTKNQVSLTCLVKGFYPSDIAVEWESNGQPE NNYKTTPPVLDSDGSFFLYSKLTVDKSRWQQGNVFSCSVMHEALH NHYTQKSLSLSPGK |
| 62 | HT2 heavy chain (alternative) | DIQMTQSPSSLSASVGDRVTITCRASQDVNTAVAWYQQKPGKAPK LLIYSASFLYSGVPSRFSGSRSGTDFTLTISSLQPEDFATYYCQQHYT TPPTFGQGTKVEIKGGGGSGGGGSGGGGSGGGGSGGGGSGGGGSE VQLVESGGGLVQPGGSLRLSCAASGFNIKDTYIHWVRQAPGKGLE WVARIYPTNGYTRYADSVKGRFTISADTSKNTAYLQMNSLRAEDT AVYYCSRWGGDGFYAMDYWGQGTLVTVSSGGGGSGGGGSGGGG SGGGGSGGGGSGGGGSQVQLQESGPGLVKPSETLSLTCTVSGFSLT SYGVNWIRQPPGKGLEWIGVMWAGGSTNYNSALMSRLTISKDTSK NQFSLKLSSVTAADTAVYYCARDENWDGAWFAYWGQGTLVTVS SASTKGPSVFPLAPSSKSTSGGTAALGCLVKDYFPEPVTVSWNSGA LTSGVHTFPAVLQSSGLYSLSSVVTVPSSSLGTQTYICNVNHKPSNTK VDKRVEPKSCDKTHTCPPCPAPELLGGPSVFLFPPKPKDTLMISRTP EVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYK TTPPVLDSDGSFFLYSKLTVDKSRWQQGNVFSCSVMHEALHNHYT QKSLSLSPGK |

## Claims

1. A construct comprising a first antigen-binding domain that specifically binds to human HER2, and a second antigen-binding domain that specifically binds to human Trop-2, wherein the first antigen-binding domain that specifically binds to human HER2 comprises a first heavy chain variable region (V_{H-1}) and a first light chain variable region (V_{L-1}), and wherein the second antigen-binding domain that specifically binds human Trop-2 comprises a second heavy chain variable region (V_{H-2}) and a second light chain variable region (V_{L-2}), wherein:
a) the V_{H-2} comprises a HC-CDR1 comprising the amino acid sequence set forth in SEQ ID NO: 26, a HC-CDR2 comprising the amino acid sequence set forth in SEQ ID NO: 27, and a HC-CDR3 comprising the amino acid sequence set forth in SEQ ID NO: 28, and
the V_{L-2} comprises a LC-CDR1 comprising the amino acid sequence set forth in SEQ ID NO: 29, a LC-CDR2 comprising the amino acid sequence set forth in SEQ ID NO: 30, and a LC-CDR3 comprising the amino acid sequence set forth in SEQ ID NO: 31; and
the V_{H-1} comprises a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and
the V_{L-1} comprises a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37 or;
b) the V_{H-2} comprises a HC-CDR1 comprising the amino acid sequence set forth in SEQ ID NO: 38, a HC-CDR2 comprising the amino acid sequence set forth in SEQ ID NO: 39, and a HC-CDR3 comprising the amino acid sequence set forth in SEQ ID NO: 40, and
the V_{L-2} comprises a LC-CDR1 comprising the amino acid sequence set forth in SEQ ID NO: 41, a LC-CDR2 comprising the amino acid sequence set forth in SEQ ID NO: 42, and a LC-CDR3 comprising the amino acid sequence set forth in SEQ ID NO: 43, and: the V_{H-1} comprises a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and
the V_{L-1} comprises a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37.

2. The construct of claim 1, wherein the first antigen-binding domain and/or the second antigen-binding domain is selected from the group consisting of a full-length antibody, a Fab, a Fab', a (Fab')2, a Fv, a single chain Fv (scFv) fragment, an scFv-scFv, a minibody, or a diabody.

3. The construct of claim 2, wherein the first antigen-binding domain comprises a full-length antibody comprising a Fc fragment,
optionally wherein the second antigen-binding domain comprises a scFv comprising the V_{H-2} and V_{L-2},
optionally wherein the second antigen-binding domain is fused to one or both of the heavy chains of the full-length antibody, or optionally
wherein the second antigen-binding domain is fused to one or both of the light chains of the full-length antibody,
optionally wherein the second antigen-binding domain is fused to N-terminus of the one or both of the heavy chains or light chains of the full-length antibody,
optionally wherein the second antigen-binding domain is fused to C-terminus of the one or both of the heavy chains or light chains of the full-length antibody,
optionally wherein the second antigen-binding domain is fused to the full-length antibody via a first linker, optionally wherein the first linker is a GS linker selected from the group consisting of SEQ ID NOs: 24, 25, and 44-54.

4. The construct of claim 2, wherein the first antigen-binding domain comprises a scFv comprising the V_{H-1} and V_{L-1},
optionally wherein the second antigen-binding domain comprises a full-length antibody comprising a Fc fragment,
optionally wherein the first antigen-binding domain is fused to one or both of the heavy chains of the full-length antibody, or
optionally wherein the first antigen-binding domain is fused to one or both of the light chains of the full-length antibody,
optionally wherein the first antigen-binding domain is fused to N-terminus of the one or both of the heavy chains or light chains of the full-length antibody,
optionally wherein the first antigen-binding domain is fused to C-terminus of the one or both of the heavy chains or light chains of the full-length antibody,
optionally wherein the second antigen-binding domain is fused to the full-length antibody via a first linker,
optionally wherein the first linker is a GS linker selected from the group consisting of SEQ ID NOs: 24, 25, and 44-54.

5. The construct of claims 3 or 4, wherein the second antigen-binding domain comprises a scFv comprising the V_{H-2} and V_{L-2}, and wherein the V_{H-1} and V_{L-1} in the scFv in the first antigen-binding domain, or the V_{H-2} and V_{L-2} in the scFv in the second antigen-binding domain are linked to each other via a second linker,
optionally wherein the second linker is a GS linker selected from the group consisting of SEQ ID NOs: 24, 25, and 44-54.

6. The construct of any one of claims 2-5, wherein:
a) the first antigen-binding domain comprises a scFv comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a full-length antibody with a Fc fragment comprising 1) the V_{H-2} comprising a HC-CDR1 comprising the amino acid sequence set forth in SEQ ID NO: 26, a HC-CDR2 comprising the amino acid sequence set forth in SEQ ID NO: 27, and a HC-CDR3 comprising the amino acid sequence set forth in SEQ ID NO: 28, and 2) the V_{L-2} comprising a LC-CDR1 comprising the amino acid sequence set forth in SEQ ID NO: 29, a LC-CDR2 comprising the amino acid sequence set forth in SEQ ID NO: 30, and a LC-CDR3 comprising the amino acid sequence set forth in SEQ ID NO: 31, and the first antigen-binding domain is fused to N-terminus of both heavy chains of the full-length antibody,
b) the first antigen-binding domain comprises a full-length antibody with a Fc fragment comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a scFv comprising 1) the V_{H-2} comprising a HC-CDR1 comprising the amino acid sequence set forth in SEQ ID NO: 26, a HC-CDR2 comprising the amino acid sequence set forth in SEQ ID NO: 27, and a HC-CDR3 comprising the amino acid sequence set forth in SEQ ID NO: 28, and 2) the V_{L-2} comprising a LC-CDR1 comprising the amino acid sequence set forth in SEQ ID NO: 29, a LC-CDR2 comprising the amino acid sequence set forth in SEQ ID NO: 30, and a LC-CDR3 comprising the amino acid sequence set forth in SEQ ID NO: 31, and the second antigen-binding domain is fused to N-terminus of both heavy chains of the full-length antibody,
c) the first antigen-binding domain comprises a full-length antibody with a Fc fragment comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a scFv comprising 1) the V_{H-2} comprising comprises a HC-CDR1 comprising the amino acid sequence set forth in SEQ ID NO: 38, a HC-CDR2 comprising the amino acid sequence set forth in SEQ ID NO: 39, and a HC-CDR3 comprising the amino acid sequence set forth in SEQ ID NO: 40, and 2) the V_{L-2} comprising a LC-CDR1 comprising the amino acid sequence set forth in SEQ ID NO: 41, a LC-CDR2 comprising the amino acid sequence set forth in SEQ ID NO: 42, and a LC-CDR3 comprising the amino acid sequence set forth in SEQ ID NO: 43, and the second antigen-binding domain is fused to C-terminus of both heavy chains of the full-length antibody,
d) the first antigen-binding domain comprises a scFv comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a full-length antibody with a Fc fragment comprising 1) the V_{H-2} comprising comprises a HC-CDR1 comprising the amino acid sequence set forth in SEQ ID NO: 38, a HC-CDR2 comprising the amino acid sequence set forth in SEQ ID NO: 39, and a HC-CDR3 comprising the amino acid sequence set forth in SEQ ID NO: 40, and 2) the V_{L-2} comprising a LC-CDR1 comprising the amino acid sequence set forth in SEQ ID NO: 41, a LC-CDR2 comprising the amino acid sequence set forth in SEQ ID NO: 42, and a LC-CDR3 comprising the amino acid sequence set forth in SEQ ID NO: 43, and the first antigen-binding domain is fused to C-terminus of both heavy chains of the full-length antibody, or
e) the first antigen-binding domain comprises a scFv comprising 1) the V_{H-1} comprising a HC-CDR1 comprising the amino acid sequence set forth in SEQ ID NO: 32, a HC-CDR2 comprising the amino acid sequence set forth in SEQ ID NO: 33, and a HC-CDR3 comprising the amino acid sequence set forth in SEQ ID NO: 34, and 2) the V_{L-1} comprising a LC-CDR1 comprising the amino acid sequence set forth in SEQ ID NO: 35, a LC-CDR2 comprising the amino acid sequence set forth in SEQ ID NO: 36, and a LC-CDR3 comprising the amino acid sequence set forth in SEQ ID NO: 37; the second antigen-binding domain comprises a full-length antibody with a Fc fragment comprising 1) the V_{H-2} comprising comprises a HC-CDR1 comprising the amino acid sequence set forth in SEQ ID NO: 38, a HC-CDR2 comprising the amino acid sequence set forth in SEQ ID NO: 39, and a HC-CDR3 comprising the amino acid sequence set forth in SEQ ID NO: 40, and 2) the V_{L-2} comprising a LC-CDR1 comprising the amino acid sequence set forth in SEQ ID NO: 41, a LC-CDR2 comprising the amino acid sequence set forth in SEQ ID NO: 42, and a LC-CDR3 comprising the amino acid sequence set forth in SEQ ID NO: 43, and the first antigen-binding domain is fused to N-terminus of both heavy chains of the full-length antibody,
optionally wherein:
a) the V_{H-1} comprises an amino acid sequence of SEQ ID NO: 7, or a variant comprising an amino acid sequence having at least 80% sequence identity; and the V_{L-1} comprises an amino acid sequence of SEQ ID NO: 8, or a variant comprising an amino acid sequence having at least 80% sequence identity, and
b) the V_{H-2} comprises an amino acid sequence of SEQ ID NO: 4, or a variant comprising an amino acid sequence having at least 80% sequence identity; and the V_{L-2} comprises an amino acid sequence of SEQ ID NO: 5, or a variant comprising an amino acid sequence having at least 80% sequence identity, or
optionally wherein:
a) the V_{H-1} comprises an amino acid sequence of SEQ ID NO: 7, or a variant comprising an amino acid sequence having at least 80% sequence identity; and the V_{L-1} comprises an amino acid sequence of SEQ ID NO: 8, or a variant comprising an amino acid sequence having at least 80% sequence identity, and
b) the V_{H-2} comprises an amino acid sequence of SEQ ID NO: 10, or a variant comprising an amino acid sequence having at least 80% sequence identity; and the V_{L-2} comprises an amino acid sequence of SEQ ID NO: 11, or a variant comprising an amino acid sequence having at least 80% sequence identity, or
optionally wherein:
a) the first antigen-binding domain comprises a scFv comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a full-length antibody with a Fc fragment comprising 1) the V_{H-2} comprising the amino acid sequence set forth in SEQ ID NO: 4, and 2) the V_{L-2} comprising the amino acid sequence set forth in SEQ ID NO: 5, and the first antigen-binding domain is fused to N-terminus of both heavy chains of the full-length antibody,
b) the first antigen-binding domain comprises a full-length antibody with a Fc fragment comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a scFv comprising 1) the V_{H-2} comprising the amino acid sequence set forth in SEQ ID NO: 4, and 2) the V_{L-2} comprising the amino acid sequence set forth in SEQ ID NO: 5, and the second antigen-binding domain is fused to N-terminus of both heavy chains of the full-length antibody,
c) the first antigen-binding domain comprises a full-length antibody with a Fc fragment comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a scFv comprising 1) the V_{H-2} comprising the amino acid sequence set forth in SEQ ID NO: 10 or 58, and 2) the V_{L-2} comprising the amino acid sequence set forth in SEQ ID NO: 11, and the second antigen-binding domain is fused to C-terminus of both heavy chains of the full-length antibody,
d) the first antigen-binding domain comprises a scFv comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a full-length antibody with a Fc fragment comprising 1) the V_{H-2} comprising the amino acid sequence set forth in SEQ ID NO: 10 or 58, and 2) the V_{L-2} comprising the amino acid sequence set forth in SEQ ID NO: 11, and the first antigen-binding domain is fused to C-terminus of both heavy chains of the full-length antibody, or
e) the first antigen-binding domain comprises a scFv comprising 1) the V_{H-1} comprising the amino acid sequence set forth in SEQ ID NO: 7, 55, or 56, and 2) the V_{L-1} comprising the amino acid sequence set forth in SEQ ID NO: 8 or 57; the second antigen-binding domain comprises a full-length antibody with a Fc fragment comprising 1) the V_{H-2} comprising the amino acid sequence set forth in SEQ ID NO: 10 or 58, and 2) the V_{L-2} comprising the amino acid sequence set forth in SEQ ID NO: 11, and the first antigen-binding domain is fused to N-terminus of both heavy chains of the full-length antibody.

7. The construct of any one of claims 3 and 4-6 wherein the second antigen-binding domain comprises a full-length antibody comprising a Fc fragment, wherein the Fc fragment comprises a heavy chain constant domain which optionally comprises an IgG constant domain and a light chain constant domain which optionally comprises a kappa constant region or a lambda constant region.

8. The construct of any one of claims 1-7, comprising:
a) two heavy chains comprising the amino acid sequence set forth in SEQ ID NO: 14, or a variant comprising an amino acid sequence having at least 80% sequence identity; two light chains comprising the amino acid sequence set forth in SEQ ID NO: 15, or a variant comprising an amino acid sequence having at least 80% sequence identity;
b) two heavy chains comprising the amino acid sequence set forth in SEQ ID NO: 16, or a variant comprising an amino acid sequence having at least 80% sequence identity; two light chains comprising the amino acid sequence set forth in SEQ ID NO: 17, or a variant comprising an amino acid sequence having at least 80% sequence identity;
c) two heavy chains comprising the amino acid sequence set forth in SEQ ID NO: 18, or a variant comprising an amino acid sequence having at least 80% sequence identity; two light chains comprising the amino acid sequence set forth in SEQ ID NO: 19, or a variant comprising an amino acid sequence having at least 80% sequence identity;
d) two heavy chains comprising the amino acid sequence set forth in SEQ ID NO: 20, or a variant comprising an amino acid sequence having at least 80% sequence identity; two light chains comprising the amino acid sequence set forth in SEQ ID NO: 21, or a variant comprising an amino acid sequence having at least 80% sequence identity;
e) two heavy chains comprising the amino acid sequence set forth in SEQ ID NO: 22, or a variant comprising an amino acid sequence having at least 80% sequence identity; two light chains comprising the amino acid sequence set forth in SEQ ID NO: 23, or a variant comprising an amino acid sequence having at least 80% sequence identity;
f) two heavy chains comprising the amino acid sequence set forth in SEQ ID NO: 60, or a variant comprising an amino acid sequence having at least 80% sequence identity; two light chains comprising the amino acid sequence set forth in SEQ ID NO: 15, or a variant comprising an amino acid sequence having at least 80% sequence identity;
g) two heavy chains comprising the amino acid sequence set forth in SEQ ID NO: 62, or a variant comprising an amino acid sequence having at least 80% sequence identity; two light chains comprising the amino acid sequence set forth in SEQ ID NO: 15, or a variant comprising an amino acid sequence having at least 80% sequence identity;
h) two heavy chains comprising the amino acid sequence set forth in SEQ ID NO: 59, or a variant comprising an amino acid sequence having at least about 80% sequence identity; two light chains comprising the amino acid sequence set forth in SEQ ID NO: 17, or a variant comprising an amino acid sequence having at least 80% sequence identity; or
i) two heavy chains comprising the amino acid sequence set forth in SEQ ID NO: 61, or a variant comprising an amino acid sequence having at least 80% sequence identity; two light chains comprising the amino acid sequence set forth in SEQ ID NO: 17, or a variant comprising an amino acid sequence having at least 80% sequence identity,
optionally wherein the construct binds specifically to HER2- and/or Trop-2-expressing cells and induces internalization upon binding to the target(s),
optionally wherein the construct is or comprises a bispecific antibody or binding fragment thereof, optionally wherein the bispecific antibody is a humanized antibody.

9. The construct of any one of claims 1-8, wherein the construct is or comprises a bispecific antibody or binding fragment thereof, optionally wherein the bispecific antibody is a humanized antibody, and wherein the construct comprises an antibody-drug conjugate (ADC) having the formula: Ab-(L-D)n, wherein Ab is the bispecific antibody or the binding fragment thereof, L is a linker or a direct bond, D is a therapeutic agent, and n is an integer from 1 to 10,
optionally wherein the therapeutic agent is a cytotoxic agent,
optionally wherein the cytotoxic agent is a monomethyl auristatin E (MMAE), or
optionally wherein the cytotoxic agent is a maytansynoid (DM1) or its derivatives.

10. A pharmaceutical composition comprising the construct of any one of claims 1-8, and a pharmaceutically acceptable carrier.

11. An isolated nucleic acid encoding the construct of any one of claims 1-8.

12. A vector comprising the isolated nucleic acid of claim 11.

13. An isolated host cell comprising the isolated nucleic acid of claim 11, or the vector of claim 12.

14. The construct of any one of claims 1-9, or the pharmaceutical composition of claim 10, for use in a method of treating a disease or condition in a subject, comprising administering the construct or pharmaceutical composition to said subject,
optionally wherein the disease or condition is a cancer,
optionally wherein the cancer is Trop2 positive and/or HER2 positive, and
optionally wherein the cancer is Trop2 medium or high and/or HER2 medium or high, or
optionally wherein the cancer is Trop2 low and/or HER2 low, and
optionally wherein the cancer is selected from the group consisting of prostate cancer, breast cancer, colon cancer, pancreatic cancer, and gastric cancer,
optionally wherein the method of treating a disease or condition in a subject further comprises a second therapy, wherein optionally the second therapy is selected from the group consisting of immunotherapy, chemotherapy, small molecule kinase inhibitor targeted therapy, surgery, radiation therapy, vaccination protocols, and stem cell transplantation.

## Patentansprüche

1. Konstrukt, umfassend eine erste Antigen-bindende Domäne, die spezifisch an humanen HER2 bindet, und eine zweite Antigen-bindende Domäne, die spezifisch an humanes Trop-2 bindet, wobei die erste Antigen-bindende Domäne, die spezifisch an humanen HER2 bindet, eine erste variable Region einer schweren Kette (V_{H-1}) und eine erste variable Region einer leichten Kette (V_{L-1}) umfasst und wobei die zweite Antigen-bindende Domäne, die spezifisch an humanes Trop-2 bindet, eine zweite variable Region einer schweren Kette (V_{H-2}) und eine zweite variable Region einer leichten Kette (V_{L-2}) umfasst, wobei:
a) die V_{H-2} eine HC-CDR1, umfassend die in SEQ ID NO: 26 dargelegte Aminosäuresequenz, eine HC-CDR2, umfassend die in SEQ ID NO: 27 dargelegte Aminosäuresequenz, und eine HC-CDR3, umfassend die in SEQ ID NO: 28 dargelegte Aminosäuresequenz, umfasst und
die V_{L-2} eine LC-CDR1, umfassend die in SEQ ID NO: 29 dargelegte Aminosäuresequenz, eine LC-CDR2, umfassend die in SEQ ID NO: 30 dargelegte Aminosäuresequenz, und eine LC-CDR3, umfassend die in SEQ ID NO: 31 dargelegte Aminosäuresequenz, umfasst; und
die V_{H-1} eine HC-CDR1, umfassend die in SEQ ID NO: 32 dargelegte Aminosäuresequenz, eine HC-CDR2, umfassend die in SEQ ID NO: 33 dargelegte Aminosäuresequenz, und eine HC-CDR3, umfassend die in SEQ ID NO: 34 dargelegte Aminosäuresequenz, umfasst und
die V_{L-1} eine LC-CDR1, umfassend die in SEQ ID NO: 35 dargelegte Aminosäuresequenz, eine LC-CDR2, umfassend die in SEQ ID NO: 36 dargelegte Aminosäuresequenz, und eine LC-CDR3, umfassend die in SEQ ID NO: 37 dargelegte Aminosäuresequenz, umfasst oder;
b) die V_{H-2} eine HC-CDR1, umfassend die in SEQ ID NO: 38 dargelegte Aminosäuresequenz, eine HC-CDR2, umfassend die in SEQ ID NO: 39 dargelegte Aminosäuresequenz, und eine HC-CDR3, umfassend die in SEQ ID NO: 40 dargelegte Aminosäuresequenz, umfasst und
die V_{L-2} eine LC-CDR1, umfassend die in SEQ ID NO: 41 dargelegte Aminosäuresequenz, eine LC-CDR2, umfassend die in SEQ ID NO: 42 dargelegte Aminosäuresequenz, und eine LC-CDR3, umfassend die in SEQ ID NO: 43 dargelegte Aminosäuresequenz, umfasst und: die V_{H-1} eine HC-CDR1, umfassend die in SEQ ID NO: 32 dargelegte Aminosäuresequenz, eine HC-CDR2, umfassend die in SEQ ID NO: 33 dargelegte Aminosäuresequenz, und eine HC-CDR3, umfassend die in SEQ ID NO: 34 dargelegte Aminosäuresequenz, umfasst und
die V_{L-1} eine LC-CDR1, umfassend die in SEQ ID NO: 35 dargelegte Aminosäuresequenz, eine LC-CDR2, umfassend die in SEQ ID NO: 36 dargelegte Aminosäuresequenz, und eine LC-CDR3, umfassend die in SEQ ID NO: 37 dargelegte Aminosäuresequenz, umfasst.

2. Konstrukt nach Anspruch 1, wobei die erste Antigen-bindende Domäne und/oder die zweite Antigen-bindende Domäne ausgewählt ist aus der Gruppe bestehend aus einem Volllängen-Antikörper, einem Fab, einem Fab', einem (Fab')2, einem Fv, einem einkettigen Fv-Fragment (scFv-Fragment), einem scFv-scFv, einem Minikörper oder einem Diakörper.

3. Konstrukt nach Anspruch 2, wobei die erste Antigen-bindende Domäne einen Volllängen-Antikörper umfasst, umfassend ein Fc-Fragment,
wobei optional die zweite Antigen-bindende Domäne ein scFv umfasst, umfassend die V_{H-2} und V_{L-2},
wobei optional die zweite Antigen-bindende Domäne an eine oder beide der schweren Ketten des Volllängen-Antikörpers anelliert ist oder optional
wobei die zweite Antigen-bindende Domäne an eine oder beide der leichten Ketten des Volllängen-Antikörpers anelliert ist,
wobei optional die zweite Antigen-bindende Domäne an den N-Terminus der einen oder der beiden der schweren Ketten oder leichten Ketten des Volllängen-Antikörpers anelliert ist,
wobei optional die zweite Antigen-bindende Domäne an den C-Terminus der einen oder der beiden der schweren Ketten oder leichten Ketten des Volllängen-Antikörpers anelliert ist,
wobei optional die zweite Antigen-bindende Domäne über einen ersten Linker an den Volllängen-Antikörper anelliert ist, wobei optional der erste Linker ein GS-Linker ist, der ausgewählt ist aus der Gruppe bestehend aus SEQ ID NOs: 24, 25 und 44-54.

4. Konstrukt nach Anspruch 2, wobei die erste Antigen-bindende Domäne ein scFv umfasst, umfassend die V_{H-1} und V_{L-1},
wobei optional die zweite Antigen-bindende Domäne einen Volllängen-Antikörper umfasst, umfassend ein Fc-Fragment,
wobei optional die erste Antigen-bindende Domäne an eine oder beide der schweren Ketten des Volllängen-Antikörpers anelliert ist oder
wobei optional die erste Antigen-bindende Domäne an eine oder beide der leichten Ketten des Volllängen-Antikörpers anelliert ist,
wobei optional die erste Antigen-bindende Domäne an den N-Terminus der einen oder der beiden der schweren Ketten oder leichten Ketten des Volllängen-Antikörpers anelliert ist,
wobei optional die erste Antigen-bindende Domäne an den C-Terminus der einen oder der beiden der schweren Ketten oder leichten Ketten des Volllängen-Antikörpers anelliert ist,
wobei optional die zweite Antigen-bindende Domäne über einen ersten Linker an den Volllängen-Antikörper anelliert ist,
wobei optional der erste Linker ein GS-Linker ist, der ausgewählt ist aus der Gruppe bestehend aus SEQ ID NOs: 24, 25 und 44-54.

5. Konstrukt nach Anspruch 3 oder 4, wobei die zweite Antigen-bindende Domäne ein scFv umfasst, umfassend die V_{H-2} und V_{L-2}, und wobei die V_{H-1} und V_{L-1} in dem scFv in der ersten Antigen-bindenden Domäne oder die V_{H-2} und V_{L-2} in dem scFv in der zweiten Antigen-bindenden Domäne über einen zweiten Linker miteinander verknüpft sind,
wobei optional der zweite Linker ein GS-Linker ist, der ausgewählt ist aus der Gruppe bestehend aus SEQ ID NOs: 24, 25 und 44-54.

6. Konstrukt nach einem der Ansprüche 2-5, wobei:
a) die erste Antigen-bindende Domäne ein scFv umfasst, umfassend 1) die V_{H-1}, umfassend eine HC-CDR1, umfassend die in SEQ ID NO: 32 dargelegte Aminosäuresequenz, eine HC-CDR2, umfassend die in SEQ ID NO: 33 dargelegte Aminosäuresequenz, und eine HC-CDR3, umfassend die in SEQ ID NO: 34 dargelegte Aminosäuresequenz, und 2) die V_{L-1}, umfassend eine LC-CDR1, umfassend die in SEQ ID NO: 35 dargelegte Aminosäuresequenz, eine LC-CDR2, umfassend die in SEQ ID NO: 36 dargelegte Aminosäuresequenz, und eine LC-CDR3, umfassend die in SEQ ID NO: 37 dargelegte Aminosäuresequenz; die zweite Antigen-bindende Domäne einen Volllängen-Antikörper mit einem Fc-Fragment umfasst, umfassend 1) die V_{H-2}, umfassend eine HC-CDR1, umfassend die in SEQ ID NO: 26 dargelegte Aminosäuresequenz, eine HC-CDR2, umfassend die in SEQ ID NO: 27 dargelegte Aminosäuresequenz, und eine HC-CDR3, umfassend die in SEQ ID NO: 28 dargelegte Aminosäuresequenz, und 2) die V_{L-2}, umfassend eine LC-CDR1, umfassend die in SEQ ID NO: 29 dargelegte Aminosäuresequenz, eine LC-CDR2, umfassend die in SEQ ID NO: 30 dargelegte Aminosäuresequenz, und eine LC-CDR3, umfassend die in SEQ ID NO: 31 dargelegte Aminosäuresequenz, und die erste Antigen-bindende Domäne an den N-Terminus beider schweren Ketten des Volllängen-Antikörpers anelliert ist,
b) die erste Antigen-bindende Domäne einen Volllängen-Antikörper mit einem Fc-Fragment umfasst, umfassend 1) die V_{H-1}, umfassend eine HC-CDR1, umfassend die in SEQ ID NO: 32 dargelegte Aminosäuresequenz, eine HC-CDR2, umfassend die in SEQ ID NO: 33 dargelegte Aminosäuresequenz, und eine HC-CDR3, umfassend die in SEQ ID NO: 34 dargelegte Aminosäuresequenz, und 2) die V_{L-1}, umfassend eine LC-CDR1, umfassend die in SEQ ID NO: 35 dargelegte Aminosäuresequenz, eine LC-CDR2, umfassend die in SEQ ID NO: 36 dargelegte Aminosäuresequenz, und eine LC-CDR3, umfassend die in SEQ ID NO: 37 dargelegte Aminosäuresequenz; die zweite Antigen-bindende Domäne ein scFv umfasst, umfassend 1) die V_{H-2}, umfassend eine HC-CDR1, umfassend die in SEQ ID NO: 26 dargelegte Aminosäuresequenz, eine HC-CDR2, umfassend die in SEQ ID NO: 27 dargelegte Aminosäuresequenz, und eine HC-CDR3, umfassend die in SEQ ID NO: 28 dargelegte Aminosäuresequenz, und 2) die V_{L-2}, umfassend eine LC-CDR1, umfassend die in SEQ ID NO: 29 dargelegte Aminosäuresequenz, eine LC-CDR2, umfassend die in SEQ ID NO: 30 dargelegte Aminosäuresequenz, und eine LC-CDR3, umfassend die in SEQ ID NO: 31 dargelegte Aminosäuresequenz, und die zweite Antigen-bindende Domäne an den N-Terminus beider schweren Ketten des Volllängen-Antikörpers anelliert ist,
c) die erste Antigen-bindende Domäne einen Volllängen-Antikörper mit einem Fc-Fragment umfasst, umfassend 1) die V_{H-1}, umfassend eine HC-CDR1, umfassend die in SEQ ID NO: 32 dargelegte Aminosäuresequenz, eine HC-CDR2, umfassend die in SEQ ID NO: 33 dargelegte Aminosäuresequenz, und eine HC-CDR3, umfassend die in SEQ ID NO: 34 dargelegte Aminosäuresequenz, und 2) die V_{L-1}, umfassend eine LC-CDR1, umfassend die in SEQ ID NO: 35 dargelegte Aminosäuresequenz, eine LC-CDR2, umfassend die in SEQ ID NO: 36 dargelegte Aminosäuresequenz, und eine LC-CDR3, umfassend die in SEQ ID NO: 37 dargelegte Aminosäuresequenz; die zweite Antigen-bindende Domäne ein scFv umfasst, umfassend 1) die V_{H-2}, umfassend umfasst eine HC-CDR1, umfassend die in SEQ ID NO: 38 dargelegte Aminosäuresequenz, eine HC-CDR2, umfassend die in SEQ ID NO: 39 dargelegte Aminosäuresequenz, und eine HC-CDR3, umfassend die in SEQ ID NO: 40 dargelegte Aminosäuresequenz, und 2) die V_{L-2}, umfassend eine LC-CDR1, umfassend die in SEQ ID NO: 41 dargelegte Aminosäuresequenz, eine LC-CDR2, umfassend die in SEQ ID NO: 42 dargelegte Aminosäuresequenz, und eine LC-CDR3, umfassend die in SEQ ID NO: 43 dargelegte Aminosäuresequenz, und die zweite Antigen-bindende Domäne an den C-Terminus beider schweren Ketten des Volllängen-Antikörpers anelliert ist,
d) die erste Antigen-bindende Domäne ein scFv umfasst, umfassend 1) die V_{H-1}, umfassend eine HC-CDR1, umfassend die in SEQ ID NO: 32 dargelegte Aminosäuresequenz, eine HC-CDR2, umfassend die in SEQ ID NO: 33 dargelegte Aminosäuresequenz, und eine HC-CDR3, umfassend die in SEQ ID NO: 34 dargelegte Aminosäuresequenz, und 2) die V_{L-1}, umfassend eine LC-CDR1, umfassend die in SEQ ID NO: 35 dargelegte Aminosäuresequenz, eine LC-CDR2, umfassend die in SEQ ID NO: 36 dargelegte Aminosäuresequenz, und eine LC-CDR3, umfassend die in SEQ ID NO: 37 dargelegte Aminosäuresequenz; die zweite Antigen-bindende Domäne einen Volllängen-Antikörper mit einem Fc-Fragment umfasst, umfassend 1) die V_{H-2}, umfassend umfasst eine HC-CDR1, umfassend die in SEQ ID NO: 38 dargelegte Aminosäuresequenz, eine HC-CDR2, umfassend die in SEQ ID NO: 39 dargelegte Aminosäuresequenz, und eine HC-CDR3, umfassend die in SEQ ID NO: 40 dargelegte Aminosäuresequenz, und 2) die V_{L-2}, umfassend eine LC-CDR1, umfassend die in SEQ ID NO: 41 dargelegte Aminosäuresequenz, eine LC-CDR2, umfassend die in SEQ ID NO: 42 dargelegte Aminosäuresequenz, und eine LC-CDR3, umfassend die in SEQ ID NO: 43 dargelegte Aminosäuresequenz, und die erste Antigen-bindende Domäne an den C-Terminus beider schweren Ketten des Volllängen-Antikörpers anelliert ist oder
e) die erste Antigen-bindende Domäne ein scFv umfasst, umfassend 1) die V_{H-1}, umfassend eine HC-CDR1, umfassend die in SEQ ID NO: 32 dargelegte Aminosäuresequenz, eine HC-CDR2, umfassend die in SEQ ID NO: 33 dargelegte Aminosäuresequenz, und eine HC-CDR3, umfassend die in SEQ ID NO: 34 dargelegte Aminosäuresequenz, und 2) die V_{L-1}, umfassend eine LC-CDR1, umfassend die in SEQ ID NO: 35 dargelegte Aminosäuresequenz, eine LC-CDR2, umfassend die in SEQ ID NO: 36 dargelegte Aminosäuresequenz, und eine LC-CDR3, umfassend die in SEQ ID NO: 37 dargelegte Aminosäuresequenz; die zweite Antigen-bindende Domäne einen Volllängen-Antikörper mit einem Fc-Fragment umfasst, umfassend 1) die V_{H-2}, umfassend umfasst eine HC-CDR1, umfassend die in SEQ ID NO: 38 dargelegte Aminosäuresequenz, eine HC-CDR2, umfassend die in SEQ ID NO: 39 dargelegte Aminosäuresequenz, und eine HC-CDR3, umfassend die in SEQ ID NO: 40 dargelegte Aminosäuresequenz, und 2) die V_{L-2}, umfassend eine LC-CDR1, umfassend die in SEQ ID NO: 41 dargelegte Aminosäuresequenz, eine LC-CDR2, umfassend die in SEQ ID NO: 42 dargelegte Aminosäuresequenz, und eine LC-CDR3, umfassend die in SEQ ID NO: 43 dargelegte Aminosäuresequenz, und die erste Antigen-bindende Domäne an den N-Terminus beider schweren Ketten des Volllängen-Antikörpers anelliert ist,
wobei optional:
a) die V_{H-1} eine Aminosäuresequenz der SEQ ID NO: 7 oder eine Variante, umfassend eine Aminosäuresequenz mit mindestens 80 % Sequenzidentität, umfasst; und die V_{L-1} eine Aminosäuresequenz der SEQ ID NO: 8 oder eine Variante, umfassend eine Aminosäuresequenz mit mindestens 80 % Sequenzidentität, umfasst und
b) die V_{H-2} eine Aminosäuresequenz der SEQ ID NO: 4 oder eine Variante, umfassend eine Aminosäuresequenz mit mindestens 80 % Sequenzidentität, umfasst; und die V_{L-2} eine Aminosäuresequenz der SEQ ID NO: 5 oder eine Variante, umfassend eine Aminosäuresequenz mit mindestens 80 % Sequenzidentität, umfasst oder
wobei optional:
a) die V_{H-1} eine Aminosäuresequenz der SEQ ID NO: 7 oder eine Variante, umfassend eine Aminosäuresequenz mit mindestens 80 % Sequenzidentität, umfasst; und die V_{L-1} eine Aminosäuresequenz der SEQ ID NO: 8 oder eine Variante, umfassend eine Aminosäuresequenz mit mindestens 80 % Sequenzidentität, umfasst und
b) die V_{H-2} eine Aminosäuresequenz der SEQ ID NO: 10 oder eine Variante, umfassend eine Aminosäuresequenz mit mindestens 80 % Sequenzidentität, umfasst; und die V_{L-2} eine Aminosäuresequenz der SEQ ID NO: 11 oder eine Variante, umfassend eine Aminosäuresequenz mit mindestens 80 % Sequenzidentität, umfasst oder
wobei optional:
a) die erste Antigen-bindende Domäne ein scFv umfasst, umfassend 1) die V_{H-1}, umfassend die in SEQ ID NO: 7, 55 oder 56 dargelegte Aminosäuresequenz, und 2) die V_{L-1}, umfassend die in SEQ ID NO: 8 oder 57 dargelegte Aminosäuresequenz; die zweite Antigen-bindende Domäne einen Volllängen-Antikörper mit einem Fc-Fragment umfasst, umfassend 1) die V_{H-2}, umfassend die in SEQ ID NO: 4 dargelegte Aminosäuresequenz, und 2) die V_{L-2}, umfassend die in SEQ ID NO: 5 dargelegte Aminosäuresequenz, und die erste Antigen-bindende Domäne an den N-Terminus beider schweren Ketten des Volllängen-Antikörpers anelliert ist,
b) die erste Antigen-bindende Domäne einen Volllängen-Antikörper mit einem Fc-Fragment umfasst, umfassend 1) die V_{H-1}, umfassend die in SEQ ID NO: 7, 55 oder 56 dargelegte Aminosäuresequenz, und 2) die V_{L-1}, umfassend die in SEQ ID NO: 8 oder 57 dargelegte Aminosäuresequenz; die zweite Antigen-bindende Domäne ein scFv umfasst, umfassend 1) die V_{H-2}, umfassend die in SEQ ID NO: 4 dargelegte Aminosäuresequenz, und 2) die V_{L-2}, umfassend die in SEQ ID NO: 5 dargelegte Aminosäuresequenz, und die zweite Antigen-bindende Domäne an den N-Terminus beider schweren Ketten des Volllängen-Antikörpers anelliert ist,
c) die erste Antigen-bindende Domäne einen Volllängen-Antikörper mit einem Fc-Fragment umfasst, umfassend 1) die V_{H-1}, umfassend die in SEQ ID NO: 7, 55 oder 56 dargelegte Aminosäuresequenz, und 2) die V_{L-1}, umfassend die in SEQ ID NO: 8 oder 57 dargelegte Aminosäuresequenz; die zweite Antigen-bindende Domäne ein scFv umfasst, umfassend 1) die V_{H-2}, umfassend die in SEQ ID NO: 10 oder 58 dargelegte Aminosäuresequenz, und 2) die V_{L-2}, umfassend die in SEQ ID NO: 11 dargelegte Aminosäuresequenz, und die zweite Antigen-bindende Domäne an den C-Terminus beider schweren Ketten des Volllängen-Antikörpers anelliert ist,
d) die erste Antigen-bindende Domäne ein scFv umfasst, umfassend 1) die V_{H-1}, umfassend die in SEQ ID NO: 7, 55 oder 56 dargelegte Aminosäuresequenz, und 2) die V_{L-1}, umfassend die in SEQ ID NO: 8 oder 57 dargelegte Aminosäuresequenz; die zweite Antigen-bindende Domäne einen Volllängen-Antikörper mit einem Fc-Fragment umfasst, umfassend 1) die V_{H-2}, umfassend die in SEQ ID NO: 10 oder 58 dargelegte Aminosäuresequenz, und 2) die V_{L-2}, umfassend die in SEQ ID NO: 11 dargelegte Aminosäuresequenz, und die erste Antigen-bindende Domäne an den C-Terminus beider schweren Ketten des Volllängen-Antikörpers anelliert ist oder
e) die erste Antigen-bindende Domäne ein scFv umfasst, umfassend 1) die V_{H-1}, umfassend die in SEQ ID NO: 7, 55 oder 56 dargelegte Aminosäuresequenz, und 2) die V_{L-1}, umfassend die in SEQ ID NO: 8 oder 57 dargelegte Aminosäuresequenz; die zweite Antigen-bindende Domäne einen Volllängen-Antikörper mit einem Fc-Fragment umfasst, umfassend 1) die V_{H-2}, umfassend die in SEQ ID NO: 10 oder 58 dargelegte Aminosäuresequenz, und 2) die V_{L-2}, umfassend die in SEQ ID NO: 11 dargelegte Aminosäuresequenz, und die erste Antigen-bindende Domäne an den N-Terminus beider schweren Ketten des Volllängen-Antikörpers anelliert ist.

7. Konstrukt nach einem der Ansprüche 3 und 4-6, wobei die zweite Antigen-bindende Domäne einen Volllängen-Antikörper umfasst, umfassend ein Fc-Fragment, wobei das Fc-Fragment eine konstante Domäne einer schweren Kette, die optional eine konstante IgG-Domäne umfasst, und eine konstante Domäne einer leichten Kette, die optional eine konstante kappa-Region oder eine konstante lambda-Region umfasst, umfasst.

8. Konstrukt nach einem der Ansprüche 1-7, umfassend:
a) zwei schwere Ketten, umfassend die in SEQ ID NO: 14 dargelegte Aminosäuresequenz oder eine Variante, umfassend eine Aminosäuresequenz mit mindestens 80 % Sequenzidentität; zwei leichte Ketten, umfassend die in SEQ ID NO: 15 dargelegte Aminosäuresequenz oder eine Variante, umfassend eine Aminosäuresequenz mit mindestens 80 % Sequenzidentität;
b) zwei schwere Ketten, umfassend die in SEQ ID NO: 16 dargelegte Aminosäuresequenz oder eine Variante, umfassend eine Aminosäuresequenz mit mindestens 80 % Sequenzidentität; zwei leichte Ketten, umfassend die in SEQ ID NO: 17 dargelegte Aminosäuresequenz oder eine Variante, umfassend eine Aminosäuresequenz mit mindestens 80 % Sequenzidentität;
c) zwei schwere Ketten, umfassend die in SEQ ID NO: 18 dargelegte Aminosäuresequenz oder eine Variante, umfassend eine Aminosäuresequenz mit mindestens 80 % Sequenzidentität; zwei leichte Ketten, umfassend die in SEQ ID NO: 19 dargelegte Aminosäuresequenz oder eine Variante, umfassend eine Aminosäuresequenz mit mindestens 80 % Sequenzidentität;
d) zwei schwere Ketten, umfassend die in SEQ ID NO: 20 dargelegte Aminosäuresequenz oder eine Variante, umfassend eine Aminosäuresequenz mit mindestens 80 % Sequenzidentität; zwei leichte Ketten, umfassend die in SEQ ID NO: 21 dargelegte Aminosäuresequenz oder eine Variante, umfassend eine Aminosäuresequenz mit mindestens 80 % Sequenzidentität;
e) zwei schwere Ketten, umfassend die in SEQ ID NO: 22 dargelegte Aminosäuresequenz oder eine Variante, umfassend eine Aminosäuresequenz mit mindestens 80 % Sequenzidentität; zwei leichte Ketten, umfassend die in SEQ ID NO: 23 dargelegte Aminosäuresequenz oder eine Variante, umfassend eine Aminosäuresequenz mit mindestens 80 % Sequenzidentität;
f) zwei schwere Ketten, umfassend die in SEQ ID NO: 60 dargelegte Aminosäuresequenz oder eine Variante, umfassend eine Aminosäuresequenz mit mindestens 80 % Sequenzidentität; zwei leichte Ketten, umfassend die in SEQ ID NO: 15 dargelegte Aminosäuresequenz oder eine Variante, umfassend eine Aminosäuresequenz mit mindestens 80 % Sequenzidentität;
g) zwei schwere Ketten, umfassend die in SEQ ID NO: 62 dargelegte Aminosäuresequenz oder eine Variante, umfassend eine Aminosäuresequenz mit mindestens 80 % Sequenzidentität; zwei leichte Ketten, umfassend die in SEQ ID NO: 15 dargelegte Aminosäuresequenz oder eine Variante, umfassend eine Aminosäuresequenz mit mindestens 80 % Sequenzidentität;
h) zwei schwere Ketten, umfassend die in SEQ ID NO: 59 dargelegte Aminosäuresequenz oder eine Variante, umfassend eine Aminosäuresequenz mit mindestens etwa 80 % Sequenzidentität; zwei leichte Ketten, umfassend die in SEQ ID NO: 17 dargelegte Aminosäuresequenz oder eine Variante, umfassend eine Aminosäuresequenz mit mindestens 80 % Sequenzidentität; oder
i) zwei schwere Ketten, umfassend die in SEQ ID NO: 61 dargelegte Aminosäuresequenz oder eine Variante, umfassend eine Aminosäuresequenz mit mindestens 80 % Sequenzidentität; zwei leichte Ketten, umfassend die in SEQ ID NO: 17 dargelegte Aminosäuresequenz oder eine Variante, umfassend eine Aminosäuresequenz mit mindestens 80 % Sequenzidentität,
wobei optional das Konstrukt spezifisch an HER2- und/oder Trop-2-exprimierende Zellen bindet und bei Bindung an das/die Ziel(e) Internalisierung induziert,
wobei optional das Konstrukt ein bispezifischer Antikörper oder Bindungsfragment davon ist oder diesen/dieses umfasst, wobei optional der bispezifische Antikörper ein humanisierter Antikörper ist.

9. Konstrukt nach einem der Ansprüche 1-8, wobei das Konstrukt ein bispezifischer Antikörper oder Bindungsfragment davon ist oder diesen/dieses umfasst, wobei optional der bispezifische Antikörper ein humanisierter Antikörper ist und wobei das Konstrukt ein Antikörper-Arzneistoff-Konjugat (ADC) mit der folgenden Formel umfasst: Ab-(L-D)n, wobei Ab der bispezifische Antikörper oder das Bindungsfragment davon ist, L ein Linker oder eine Direktbindung ist, D ein therapeutisches Mittel ist und n eine ganze Zahl von 1 bis 10 ist,
wobei optional das therapeutische Mittel ein zytotoxisches Mittel ist,
wobei optional das zytotoxische Mittel ein Monomethyl-Auristatin E (MMAE) ist oder
wobei es sich optional bei dem zytotoxischen Mittel um ein Maytansinoid (DM1) oder seine Derivate handelt.

10. Pharmazeutische Zusammensetzung, umfassend das Konstrukt nach einem der Ansprüche 1-8 und einen pharmazeutisch unbedenklichen Träger.

11. Isolierte Nucleinsäure, die für das Konstrukt nach einem der Ansprüche 1-8 codiert.

12. Vektor, umfassend die isolierte Nucleinsäure nach Anspruch 11.

13. Isolierte Wirtszelle, umfassend die isolierte Nucleinsäure nach Anspruch 11 oder den Vektor nach Anspruch 12.

14. Konstrukt nach einem der Ansprüche 1-9 oder pharmazeutische Zusammensetzung nach Anspruch 10 zur Verwendung in einem Verfahren zum Behandeln einer Krankheit oder Erkrankung bei einem Subjekt, umfassend Verabreichen des Konstrukts oder der pharmazeutischen Zusammensetzung an das Subjekt,
wobei optional die Krankheit oder Erkrankung ein Krebs ist,
wobei optional der Krebs Trop2-positiv und/oder HER2-positiv ist und
wobei optional der Krebs Trop2-mittel oder -hoch und/oder HER2-mittel oder -hoch ist oder
wobei optional der Krebs Trop2-niedrig und/oder HER2-niedrig ist und
wobei optional der Krebs ausgewählt ist aus der Gruppe bestehend aus Prostatakrebs, Brustkrebs, Kolonkrebs, Bauchspeicheldrüsenkrebs und gastrischem Krebs,
wobei optional das Verfahren zum Behandeln einer Krankheit oder Erkrankung bei einem Subjekt ferner ein zweite Therapie umfasst, wobei die zweite Therapie optional ausgewählt ist aus der Gruppe bestehend aus Immuntherapie, Chemotherapie, auf niedermolekulare Kinaseinhibitoren gerichteter Therapie, Chirurgie, Strahlentherapie, Impfprotokollen und Stammzelltransplantation.

## Revendications

1. Construction comprenant un premier domaine de liaison à l'antigène qui se lie spécifiquement à l'HER2 humain et un second domaine de liaison à l'antigène qui se lie spécifiquement au Trop-2 humain, dans laquelle le premier domaine de liaison à l'antigène qui se lie spécifiquement à l'HER2 humain comprend une première région variable de chaîne lourde (V_{H-1}) et une première région variable de chaîne légère (V_{L-1}), et dans laquelle le second domaine de liaison à l'antigène qui se lie spécifiquement au Trop-2 humain comprend une seconde région variable de chaîne lourde (V_{H-2}) et une seconde région variable de chaîne légère (V_{L-2}), dans laquelle :
a) la V_{H-2} comprend une HC-CDR1 comprenant la séquence d'acides aminés définie dans SEQ ID NO: 26, une HC-CDR2 comprenant la séquence d'acides aminés définie dans SEQ ID NO: 27, et une HC-CDR3 comprenant la séquence d'acides aminés définie dans SEQ ID NO: 28, et
la V_{L-2} comprend une LC-CDR1 comprenant la séquence d'acides aminés définie dans SEQ ID NO: 29, une LC-CDR2 comprenant la séquence d'acides aminés définie dans SEQ ID NO: 30, et une LC-CDR3 comprenant la séquence d'acides aminés définie dans SEQ ID NO: 31 ; et
la V_{H-1} comprend une HC-CDR1 comprenant la séquence d'acides aminés définie dans SEQ ID NO: 32, une HC-CDR2 comprenant la séquence d'acides aminés définie dans SEQ ID NO: 33, et une HC-CDR3 comprenant la séquence d'acides aminés définie dans SEQ ID NO: 34, et
la V_{L-1} comprend une LC-CDR1 comprenant la séquence d'acides aminés définie dans SEQ ID NO: 35, une LC-CDR2 comprenant la séquence d'acides aminés définie dans SEQ ID NO: 36, et une LC-CDR3 comprenant la séquence d'acides aminés définie dans SEQ ID NO: 37 ou ;
b) la V_{H-2} comprend une HC-CDR1 comprenant la séquence d'acides aminés définie dans SEQ ID NO: 38, une HC-CDR2 comprenant la séquence d'acides aminés définie dans SEQ ID NO: 39, et une HC-CDR3 comprenant la séquence d'acides aminés définie dans SEQ ID NO: 40, et
la V_{L-2} comprend une LC-CDR1 comprenant la séquence d'acides aminés définie dans SEQ ID NO: 41, une LC-CDR2 comprenant la séquence d'acides aminés définie dans SEQ ID NO: 42, et une LC-CDR3 comprenant la séquence d'acides aminés définie dans SEQ ID NO: 43, et :la V_{H-1} comprend une HC-CDR1 comprenant la séquence d'acides aminés définie dans SEQ ID NO: 32, une HC-CDR2 comprenant la séquence d'acides aminés définie dans SEQ ID NO: 33, et une HC-CDR3 comprenant la séquence d'acides aminés définie dans SEQ ID NO: 34, et
la V_{L-1} comprend une LC-CDR1 comprenant la séquence d'acides aminés définie dans SEQ ID NO: 35, une LC-CDR2 comprenant la séquence d'acides aminés définie dans SEQ ID NO: 36, et une LC-CDR3 comprenant la séquence d'acides aminés définie dans SEQ ID NO: 37.

2. Construction selon la revendication 1, dans laquelle le premier domaine de liaison à l'antigène et/ou le second domaine de liaison à l'antigène sont choisis parmi le groupe constitué d'un anticorps de pleine longueur, d'un Fab, d'un Fab', d'un (Fab')2, d'un Fv, d'un fragment Fv à chaîne unique (scFv), d'un scFv-scFv, d'un minianticorps ou d'un dianticorps.

3. Construction selon la revendication 2, dans laquelle le premier domaine de liaison à l'antigène comprend un anticorps de pleine longueur comprenant un fragment Fc,
éventuellement dans laquelle le second domaine de liaison à l'antigène comprend un scFv comprenant les V_{H-2} et V_{L-2},
éventuellement dans laquelle le second domaine de liaison à l'antigène est fusionné à l'une ou aux deux chaînes lourdes de l'anticorps de pleine longueur, ou éventuellement
dans laquelle le second domaine de liaison à l'antigène est fusionné à l'une ou aux deux chaînes légères de l'anticorps de pleine longueur,
éventuellement dans laquelle le second domaine de liaison à l'antigène est fusionné à l'extrémité N-terminale de l'une ou des deux parmi les chaînes lourdes ou chaînes légères de l'anticorps de pleine longueur,
éventuellement dans laquelle le second domaine de liaison à l'antigène est fusionné à l'extrémité C-terminale de l'une ou des deux parmi les chaînes lourdes ou chaînes légères de l'anticorps de pleine longueur,
éventuellement dans laquelle le second domaine de liaison à l'antigène est fusionné à l'anticorps de pleine longueur via un premier lieur, éventuellement dans laquelle le premier lieur est un lieur GS sélectionné parmi le groupe constitué des SEQ ID NO: 24, 25 et 44 à 54.

4. Construction selon la revendication 2, dans laquelle le premier domaine de liaison à l'antigène comprend un scFv comprenant les V_{H-1} et V_{L-1},
éventuellement dans laquelle le second domaine de liaison à l'antigène comprend un anticorps de pleine longueur comprenant un fragment Fc,
éventuellement dans laquelle le premier domaine de liaison à l'antigène est fusionné à l'une ou aux deux parmi les chaînes lourdes de l'anticorps de pleine longueur, ou
éventuellement dans laquelle le premier domaine de liaison à l'antigène est fusionné à l'une ou aux deux parmi les chaînes légères de l'anticorps de pleine longueur,
éventuellement dans laquelle le premier domaine de liaison à l'antigène est fusionné à l'extrémité N-terminale de l'une ou des deux parmi les chaînes lourdes ou chaînes légères de l'anticorps de pleine longueur,
éventuellement dans laquelle le premier domaine de liaison à l'antigène est fusionné à l'extrémité C-terminale de l'une ou des deux parmi les chaînes lourdes ou chaînes légères de l'anticorps de pleine longueur,
éventuellement dans laquelle le second domaine de liaison à l'antigène est fusionné à l'anticorps de pleine longueur via un premier lieur,
éventuellement dans laquelle le premier lieur est un lieur GS sélectionné parmi le groupe constitué des SEQ ID NO: 24, 25 et 44 à 54.

5. Construction selon les revendications 3 ou 4, dans laquelle le second domaine de liaison à l'antigène comprend un scFv comprenant les V_{H-2} et V_{L-2}, et dans laquelle les V_{H-1} et V_{L-1} dans le scFv dans le premier domaine de liaison à l'antigène, ou les V_{H-2} et V_{L-2} dans le scFv dans le second domaine de liaison à l'antigène sont liés entre eux par un second lieur,
éventuellement dans laquelle le second lieur est un lieur GS sélectionné parmi le groupe constitué des SEQ ID NO: 24, 25 et 44 à 54.

6. Construction selon l'une quelconque des revendications 2 à 5, dans laquelle :
a) le premier domaine de liaison à l'antigène comprend un scFv comprenant 1) la V_{H-1} comprenant une HC-CDR1 comprenant la séquence d'acides aminés définie dans SEQ ID NO: 32, une HC-CDR2 comprenant la séquence d'acides aminés définie dans SEQ ID NO: 33, et une HC-CDR3 comprenant la séquence d'acides aminés définie dans SEQ ID NO: 34, et 2) la V_{L-1} comprenant une LC-CDR1 comprenant la séquence d'acides aminés définie dans SEQ ID NO: 35, une LC-CDR2 comprenant la séquence d'acides aminés définie dans SEQ ID NO: 36, et une LC-CDR3 comprenant la séquence d'acides aminés définie dans SEQ ID NO: 37 ; le second domaine de liaison à l'antigène comprend un anticorps de pleine longueur avec un fragment Fc comprenant 1) la V_{H-2} comprenant une HC-CDR1 comprenant la séquence d'acides aminés définie dans SEQ ID NO: 26, une HC-CDR2 comprenant la séquence d'acides aminés définie dans SEQ ID NO: 27, et une HC-CDR3 comprenant la séquence d'acides aminés définie dans SEQ ID NO: 28, et 2) la V_{L-2} comprenant une LC-CDR1 comprenant la séquence d'acides aminés définie dans SEQ ID NO: 29, une LC-CDR2 comprenant la séquence d'acides aminés définie dans SEQ ID NO: 30, et une LC-CDR3 comprenant la séquence d'acides aminés définie dans SEQ ID NO: 31, et le premier domaine de liaison à l'antigène est fusionné à l'extrémité N-terminale des deux chaînes lourdes de l'anticorps de pleine longueur,
b) le premier domaine de liaison à l'antigène comprend un anticorps de pleine longueur avec un fragment Fc comprenant 1) la V_{H-1} comprenant une HC-CDR1 comprenant la séquence d'acides aminés définie dans SEQ ID NO: 32, une HC-CDR2 comprenant la séquence d'acides aminés définie dans SEQ ID NO: 33, et une HC-CDR3 comprenant la séquence d'acides aminés définie dans SEQ ID NO: 34, et 2) la V_{L-1} comprenant une LC-CDR1 comprenant la séquence d'acides aminés définie dans SEQ ID NO: 35, une LC-CDR2 comprenant la séquence d'acides aminés définie dans SEQ ID NO: 36, et une LC-CDR3 comprenant la séquence d'acides aminés définie dans SEQ ID NO: 37 ; le second domaine de liaison à l'antigène comprend un scFv comprenant 1) la V_{H-2} comprenant une HC-CDR1 comprenant la séquence d'acides aminés définie dans SEQ ID NO: 26, une HC-CDR2 comprenant la séquence d'acides aminés définie dans SEQ ID NO: 27, et une HC-CDR3 comprenant la séquence d'acides aminés définie dans SEQ ID NO: 28, et 2) la V_{L-2} comprenant une LC-CDR1 comprenant la séquence d'acides aminés définie dans SEQ ID NO: 29, une LC-CDR2 comprenant la séquence d'acides aminés définie dans SEQ ID NO: 30, et une LC-CDR3 comprenant la séquence d'acides aminés définie dans SEQ ID NO: 31, et le second domaine de liaison à l'antigène est fusionné à l'extrémité N-terminale des deux chaînes lourdes de l'anticorps de pleine longueur,
c) le premier domaine de liaison à l'antigène comprend un anticorps de pleine longueur avec un fragment Fc comprenant 1) la V_{H-1} comprenant une HC-CDR1 comprenant la séquence d'acides aminés définie dans SEQ ID NO: 32, une HC-CDR2 comprenant la séquence d'acides aminés définie dans SEQ ID NO: 33, et une HC-CDR3 comprenant la séquence d'acides aminés définie dans SEQ ID NO: 34, et 2) la V_{L-1} comprenant une LC-CDR1 comprenant la séquence d'acides aminés définie dans SEQ ID NO: 35, une LC-CDR2 comprenant la séquence d'acides aminés définie dans SEQ ID NO: 36, et une LC-CDR3 comprenant la séquence d'acides aminés définie dans SEQ ID NO: 37 ; le second domaine de liaison à l'antigène comprend un scFv comprenant 1) la V_{H-2} comprenant comprend une HC-CDR1 comprenant la séquence d'acides aminés définie dans SEQ ID NO: 38, une HC-CDR2 comprenant la séquence d'acides aminés définie dans SEQ ID NO: 39, et une HC-CDR3 comprenant la séquence d'acides aminés définie dans SEQ ID NO: 40, et 2) la V_{L-2} comprenant une LC-CDR1 comprenant la séquence d'acides aminés définie dans SEQ ID NO: 41, une LC-CDR2 comprenant la séquence d'acides aminés définie dans SEQ ID NO: 42, et une LC-CDR3 comprenant la séquence d'acides aminés définie dans SEQ ID NO: 43, et le second domaine de liaison à l'antigène est fusionné à l'extrémité C-terminale des deux chaînes lourdes de l'anticorps de pleine longueur,
d) le premier domaine de liaison à l'antigène comprend un scFv comprenant 1) la V_{H-1} comprenant une HC-CDR1 comprenant la séquence d'acides aminés définie dans SEQ ID NO: 32, une HC-CDR2 comprenant la séquence d'acides aminés définie dans SEQ ID NO: 33, et une HC-CDR3 comprenant la séquence d'acides aminés définie dans SEQ ID NO: 34, et 2) la V_{L-1} comprenant une LC-CDR1 comprenant la séquence d'acides aminés définie dans SEQ ID NO: 35, une LC-CDR2 comprenant la séquence d'acides aminés définie dans SEQ ID NO: 36, et une LC-CDR3 comprenant la séquence d'acides aminés définie dans SEQ ID NO: 37 ; le second domaine de liaison à l'antigène comprend un anticorps de pleine longueur avec un fragment Fc comprenant 1) la V_{H-2} comprenant une HC-CDR1 comprenant la séquence d'acides aminés définie dans SEQ ID NO: 38, une HC-CDR2 comprenant la séquence d'acides aminés définie dans SEQ ID NO: 39, et une HC-CDR3 comprenant la séquence d'acides aminés définie dans SEQ ID NO: 40, et 2) la V_{L-2} comprenant une LC-CDR1 comprenant la séquence d'acides aminés définie dans SEQ ID NO: 41, une LC-CDR2 comprenant la séquence d'acides aminés définie dans SEQ ID NO: 42, et une LC-CDR3 comprenant la séquence d'acides aminés définie dans SEQ ID NO: 43, et le premier domaine de liaison à l'antigène est fusionné à l'extrémité C-terminale des deux chaînes lourdes de l'anticorps de pleine longueur, ou
e) le premier domaine de liaison à l'antigène comprend un scFv comprenant 1) la V_{H-1} comprenant une HC-CDR1 comprenant la séquence d'acides aminés définie dans SEQ ID NO: 32, une HC-CDR2 comprenant la séquence d'acides aminés définie dans SEQ ID NO: 33, et une HC-CDR3 comprenant la séquence d'acides aminés définie dans SEQ ID NO: 34, et 2) la V_{L-1} comprenant une LC-CDR1 comprenant la séquence d'acides aminés définie dans SEQ ID NO: 35, une LC-CDR2 comprenant la séquence d'acides aminés définie dans SEQ ID NO: 36, et une LC-CDR3 comprenant la séquence d'acides aminés définie dans SEQ ID NO: 37 ; le second domaine de liaison à l'antigène comprend un anticorps de pleine longueur avec un fragment Fc comprenant 1) la V_{H-2} comprenant une HC-CDR1 comprenant la séquence d'acides aminés définie dans SEQ ID NO: 38, une HC-CDR2 comprenant la séquence d'acides aminés définie dans SEQ ID NO: 39, et une HC-CDR3 comprenant la séquence d'acides aminés définie dans SEQ ID NO: 40, et 2) la V_{L-2} comprenant une LC-CDR1 comprenant la séquence d'acides aminés définie dans SEQ ID NO: 41, une LC-CDR2 comprenant la séquence d'acides aminés définie dans SEQ ID NO: 42, et une LC-CDR3 comprenant la séquence d'acides aminés définie dans SEQ ID NO: 43, et le premier domaine de liaison à l'antigène est fusionné à l'extrémité N-terminale des deux chaînes lourdes de l'anticorps de pleine longueur,
éventuellement dans laquelle :
a) la V_{H-1} comprend une séquence d'acides aminés de SEQ ID NO: 7, ou une variante comprenant une séquence d'acides aminés ayant au moins 80 % d'identité de séquence ; et la V_{L-1} comprend une séquence d'acides aminés de SEQ ID NO: 8, ou une variante comprenant une séquence d'acides aminés ayant au moins 80 % d'identité de séquence, et
b) la V_{H-2} comprend une séquence d'acides aminés de SEQ ID NO: 4, ou une variante comprenant une séquence d'acides aminés ayant au moins 80 % d'identité de séquence ; et la V_{L-2} comprend une séquence d'acides aminés de SEQ ID NO: 5, ou une variante comprenant une séquence d'acides aminés ayant au moins 80 % d'identité de séquence, ou
éventuellement dans laquelle :
a) la V_{H-1} comprend une séquence d'acides aminés de SEQ ID NO: 7, ou une variante comprenant une séquence d'acides aminés ayant au moins 80 % d'identité de séquence ; et la V_{L-1} comprend une séquence d'acides aminés de SEQ ID NO: 8, ou une variante comprenant une séquence d'acides aminés ayant au moins 80 % d'identité de séquence, et
b) la V_{H-2} comprend une séquence d'acides aminés de SEQ ID NO: 10, ou une variante comprenant une séquence d'acides aminés ayant au moins 80 % d'identité de séquence ; et la V_{L-2} comprend une séquence d'acides aminés de SEQ ID NO: 11, ou une variante comprenant une séquence d'acides aminés ayant au moins 80 % d'identité de séquence, ou
éventuellement dans laquelle :
a) le premier domaine de liaison à l'antigène comprend un scFv comprenant 1) la V_{H-1} comprenant la séquence d'acides aminés définie dans SEQ ID NO: 7, 55 ou 56, et 2) la V_{L-1} comprenant la séquence d'acides aminés définie dans SEQ ID NO: 8 ou 57 ; le second domaine de liaison à l'antigène comprend un anticorps de pleine longueur avec un fragment Fc comprenant 1) la V_{H-2} comprenant la séquence d'acides aminés définie dans SEQ ID NO: 4, et 2) la V_{L-2} comprenant la séquence d'acides aminés définie dans SEQ ID NO: 5, et le premier domaine de liaison à l'antigène est fusionné à l'extrémité N-terminale des deux chaînes lourdes de l'anticorps de pleine longueur,
b) le premier domaine de liaison à l'antigène comprend un anticorps de pleine longueur avec un fragment Fc comprenant 1) la V_{H-1} comprenant la séquence d'acides aminés définie dans SEQ ID NO: 7, 55 ou 56, et 2) la V_{L-1} comprenant la séquence d'acides aminés définie dans SEQ ID NO: 8 ou 57 ; le second domaine de liaison à l'antigène comprend un scFv comprenant 1) la V_{H-2} comprenant la séquence d'acides aminés définie dans SEQ ID NO: 4, et 2) la V_{L-2} comprenant la séquence d'acides aminés définie dans SEQ ID NO: 5, et le second domaine de liaison à l'antigène est fusionné à l'extrémité N-terminale des deux chaînes lourdes de l'anticorps de pleine longueur,
c) le premier domaine de liaison à l'antigène comprend un anticorps de pleine longueur avec un fragment Fc comprenant 1) la V_{H-1} comprenant la séquence d'acides aminés définie dans SEQ ID NO: 7, 55 ou 56, et 2) la V_{L-1} comprenant la séquence d'acides aminés définie dans SEQ ID NO: 8 ou 57 ; le second domaine de liaison à l'antigène comprend un scFv comprenant 1) la V_{H-2} comprenant la séquence d'acides aminés définie dans SEQ ID NO: 10 ou 58, et 2) la V_{L-2} comprenant la séquence d'acides aminés définie dans SEQ ID NO: 11, et le second domaine de liaison à l'antigène est fusionné à l'extrémité C-terminale des deux chaînes lourdes de l'anticorps de pleine longueur,
d) le premier domaine de liaison à l'antigène comprend un scFv comprenant 1) la V_{H-1} comprenant la séquence d'acides aminés définie dans SEQ ID NO: 7, 55 ou 56, et 2) la V_{L-1} comprenant la séquence d'acides aminés définie dans SEQ ID NO: 8 ou 57 ; le second domaine de liaison à l'antigène comprend un anticorps de pleine longueur avec un fragment Fc comprenant 1) la V_{H-2} comprenant la séquence d'acides aminés définie dans SEQ ID NO: 10 ou 58, et 2) la V_{L-2} comprenant la séquence d'acides aminés définie dans SEQ ID NO: 11, et le premier domaine de liaison à l'antigène est fusionné à l'extrémité C-terminale des deux chaînes lourdes de l'anticorps de pleine longueur, ou
e) le premier domaine de liaison à l'antigène comprend un scFv comprenant 1) la V_{H-1} comprenant la séquence d'acides aminés définie dans SEQ ID NO: 7, 55 ou 56, et 2) la V_{L-1} comprenant la séquence d'acides aminés définie dans SEQ ID NO: 8 ou 57 ; le second domaine de liaison à l'antigène comprend un anticorps de pleine longueur avec un fragment Fc comprenant 1) la V_{H-2} comprenant la séquence d'acides aminés définie dans SEQ ID NO: 10 ou 58, et 2) la V_{L-2} comprenant la séquence d'acides aminés définie dans SEQ ID NO: 11, et le premier domaine de liaison à l'antigène est fusionné à l'extrémité N-terminale des deux chaînes lourdes de l'anticorps de pleine longueur.

7. Construction selon l'une quelconque des revendications 3 et 4 à 6, dans laquelle le second domaine de liaison à l'antigène comprend un anticorps de pleine longueur comprenant un fragment Fc, dans laquelle le fragment Fc comprend un domaine constant de chaîne lourde qui comprend éventuellement un domaine constant d'IgG et un domaine constant de chaîne légère qui comprend éventuellement une région constante kappa ou une région constante lambda.

8. Construction selon l'une quelconque des revendications 1 à 7, comprenant :
a) deux chaînes lourdes comprenant la séquence d'acides aminés définie dans SEQ ID NO: 14, ou une variante comprenant une séquence d'acides aminés ayant au moins 80 % d'identité de séquence ; deux chaînes légères comprenant la séquence d'acides aminés définie dans SEQ ID NO: 15, ou une variante comprenant une séquence d'acides aminés ayant au moins 80 % d'identité de séquence ;
b) deux chaînes lourdes comprenant la séquence d'acides aminés définie dans SEQ ID NO: 16, ou une variante comprenant une séquence d'acides aminés ayant au moins 80 % d'identité de séquence ; deux chaînes légères comprenant la séquence d'acides aminés définie dans SEQ ID NO: 17, ou une variante comprenant une séquence d'acides aminés ayant au moins 80 % d'identité de séquence ;
c) deux chaînes lourdes comprenant la séquence d'acides aminés définie dans SEQ ID NO: 18, ou une variante comprenant une séquence d'acides aminés ayant au moins 80 % d'identité de séquence ; deux chaînes légères comprenant la séquence d'acides aminés définie dans SEQ ID NO: 19, ou une variante comprenant une séquence d'acides aminés ayant au moins 80 % d'identité de séquence ;
d) deux chaînes lourdes comprenant la séquence d'acides aminés définie dans SEQ ID NO: 20, ou une variante comprenant une séquence d'acides aminés ayant au moins 80 % d'identité de séquence ; deux chaînes légères comprenant la séquence d'acides aminés définie dans SEQ ID NO: 21, ou une variante comprenant une séquence d'acides aminés ayant au moins 80 % d'identité de séquence ;
e) deux chaînes lourdes comprenant la séquence d'acides aminés définie dans SEQ ID NO: 22, ou une variante comprenant une séquence d'acides aminés ayant au moins 80 % d'identité de séquence ; deux chaînes légères comprenant la séquence d'acides aminés définie dans SEQ ID NO: 23, ou une variante comprenant une séquence d'acides aminés ayant au moins 80 % d'identité de séquence ;
f) deux chaînes lourdes comprenant la séquence d'acides aminés définie dans SEQ ID NO: 60, ou une variante comprenant une séquence d'acides aminés ayant au moins 80 % d'identité de séquence ; deux chaînes légères comprenant la séquence d'acides aminés définie dans SEQ ID NO: 15, ou une variante comprenant une séquence d'acides aminés ayant au moins 80 % d'identité de séquence ;
g) deux chaînes lourdes comprenant la séquence d'acides aminés définie dans SEQ ID NO : 62, ou une variante comprenant une séquence d'acides aminés ayant au moins 80 % d'identité de séquence ; deux chaînes légères comprenant la séquence d'acides aminés définie dans SEQ ID NO: 15, ou une variante comprenant une séquence d'acides aminés ayant au moins 80 % d'identité de séquence ;
h) deux chaînes lourdes comprenant la séquence d'acides aminés définie dans SEQ ID NO: 59, ou une variante comprenant une séquence d'acides aminés ayant au moins environ 80 % d'identité de séquence ; deux chaînes légères comprenant la séquence d'acides aminés définie dans SEQ ID NO: 17, ou une variante comprenant une séquence d'acides aminés ayant au moins 80 % d'identité de séquence, ou
i) deux chaînes lourdes comprenant la séquence d'acides aminés définie dans SEQ ID NO: 61, ou une variante comprenant une séquence d'acides aminés ayant au moins 80 % d'identité de séquence ; deux chaînes légères comprenant la séquence d'acides aminés définie dans SEQ ID NO: 17, ou une variante comprenant une séquence d'acides aminés ayant au moins 80 % d'identité de séquence ;
éventuellement dans laquelle la construction se lie spécifiquement aux cellules exprimant HER2 et/ou Trop-2 et induit une internalisation lors de sa liaison à la ou aux cibles,
éventuellement dans laquelle la construction est ou comprend un anticorps bispécifique ou un fragment de liaison de celui-ci, éventuellement dans laquelle l'anticorps bispécifique est un anticorps humanisé.

9. Construction selon l'une quelconque des revendications 1 à 8, dans laquelle la construction est ou comprend un anticorps bispécifique ou un fragment de liaison de celui-ci, éventuellement dans laquelle l'anticorps bispécifique est un anticorps humanisé, et dans laquelle la construction comprend un conjugué anticorps-médicament (ADC) ayant la formule : Ab-(L-D)n, dans laquelle Ab est l'anticorps bispécifique ou le fragment de liaison de celui-ci, L est un lieur ou une liaison directe, D est un agent thérapeutique et n est un entier de 1 à 10,
éventuellement dans laquelle l'agent thérapeutique est un agent cytotoxique,
éventuellement dans laquelle l'agent cytotoxique est une monométhylauristatine E (MMAE), ou éventuellement dans laquelle l'agent cytotoxique est un maytansynoïde (DM1) ou ses dérivés.

10. Composition pharmaceutique comprenant la construction selon l'une quelconque des revendications 1 à 8 et un support pharmaceutiquement acceptable.

11. Acide nucléique isolé codant pour la construction selon l'une quelconque des revendications 1 à 8.

12. Vecteur comprenant l'acide nucléique isolé selon la revendication 11.

13. Cellule hôte isolée comprenant l'acide nucléique isolé selon la revendication 11, ou le vecteur selon la revendication 12.

14. Construction selon l'une quelconque des revendications 1 à 9, ou composition pharmaceutique selon la revendication 10, destinée à être utilisée dans un procédé de traitement d'une maladie ou d'une affection chez un sujet, comprenant l'administration de la construction ou de la composition pharmaceutique audit sujet,
éventuellement dans laquelle la maladie ou l'affection est un cancer,
éventuellement dans laquelle le cancer est Trop2 positif et/ou HER2 positif, et
éventuellement dans laquelle le cancer est à expression moyenne ou élevée de Trop-2 et/ou à expression moyenne ou élevée de HER2, ou
éventuellement dans laquelle le cancer est à faible expression de Trop-2 et/ou à faible expression de HER2, et
éventuellement dans laquelle le cancer est choisi parmi le groupe constitué du cancer de la prostate, du cancer du sein, du cancer du côlon, du cancer du pancréas et du cancer gastrique,
éventuellement dans laquelle le procédé de traitement d'une maladie ou d'une affection chez un sujet comprend en outre une seconde thérapie, dans laquelle éventuellement la seconde thérapie est choisie parmi le groupe constitué de l'immunothérapie, de la chimiothérapie, de la thérapie ciblée par inhibiteur de kinase à petite molécule, de la chirurgie, de la radiothérapie, des protocoles de vaccination et de la transplantation de cellules souches.
